(19) Europäisches Patentamt
European Patent Office
Office européen des brevets

(11) EP 4 365 183 A1

(12) # EUROPEAN PATENT APPLICATION
published in accordance with Art. 153(4) EPC

(43) Date of publication:
**08.05.2024 Bulletin 2024/19**

(21) Application number: **22827668.9**

(22) Date of filing: **23.06.2022**

(51) International Patent Classification (IPC):
***C07J 71/00*** *(2006.01)* ***A61K 31/58*** *(2006.01)*
***A61P 5/46*** *(2006.01)*

(52) Cooperative Patent Classification (CPC):
**A61K 31/58; A61P 5/46; C07J 71/00**

(86) International application number:
**PCT/CN2022/100881**

(87) International publication number:
**WO 2022/268176 (29.12.2022 Gazette 2022/52)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **24.06.2021 CN 202110706921**
**27.01.2022 CN 202210101639**
**01.04.2022 CN 202210340109**

(71) Applicant: **Jiangsu Simcere Pharmaceutical Co., Ltd.**
**Nanjing, Jiangsu 210042 (CN)**

(72) Inventors:
- **LIU, Lifeng**
  **Shanghai 201318 (CN)**
- **LI, Zhen**
  **Shanghai 201318 (CN)**
- **LIU, Xiaoqing**
  **Shanghai 201318 (CN)**
- **ZHAO, Xiaofeng**
  **Shanghai 201318 (CN)**
- **CHAI, Xiaojuan**
  **Shanghai 201318 (CN)**
- **TANG, Feng**
  **Shanghai 201318 (CN)**
- **FU, Yayuan**
  **Shanghai 201318 (CN)**
- **CAO, Zhuoxiao**
  **Shanghai 201318 (CN)**
- **TANG, Renhong**
  **Shanghai 201318 (CN)**
- **REN, Jinsheng**
  **Nanjing, Jiangsu 210042 (CN)**

(74) Representative: **V.O.**
**P.O. Box 87930**
**2508 DH Den Haag (NL)**

(54) **STEROID COMPOUND, AND PHARMACEUTICAL COMPOSITION THEREOF AND USE THEREOF**

(57) A compound as represented by formula (I) or a pharmaceutically acceptable salt thereof or a pharmaceutical composition thereof, a preparation method therefor, and the use thereof in the treatment of tumors, inflammatory diseases or autoimmune diseases.

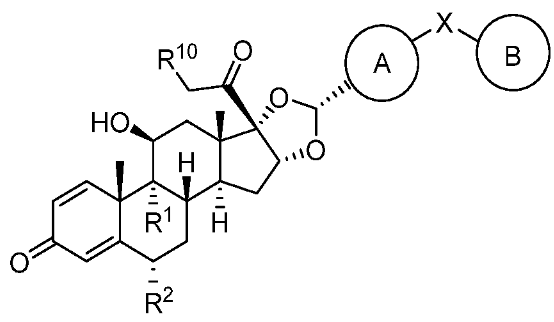

(I)

EP 4 365 183 A1

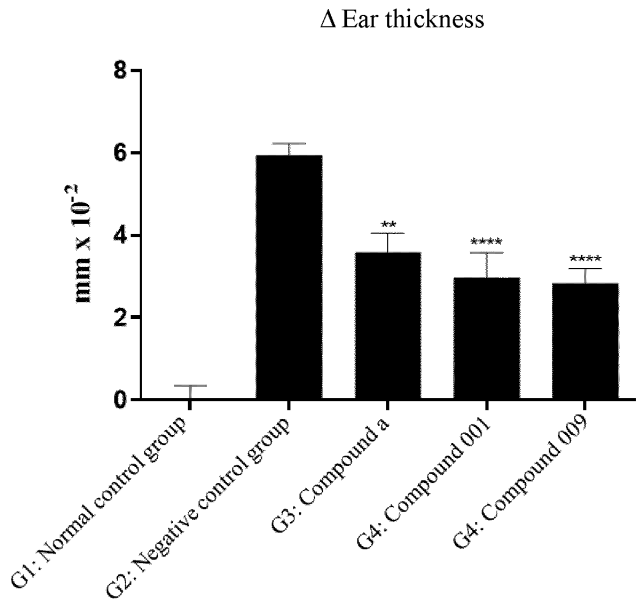
Δ Ear thickness
mm x 10^-2
8
6
4
2
0
**
****
****
G1: Normal control group
G2: Negative control group
G3: Compound a
G4: Compound 001
G4: Compound 009

FIG. 2

## Description

The present invention claims priority to the following applications: Chinese Patent Application No. 202110706921.X filed with China National Intellectual Property Administration on Jun. 24, 2021 and entitled "STEROID COMPOUND AND USE THEREOF", Chinese Patent Application No. 202210101639.3 filed with China National Intellectual Property Administration on Jan. 27, 2022 and entitled "STEROID COMPOUND AND USE THEREOF", and Chinese Patent Application No. 202210340109.4 filed with China National Intellectual Property Administration on Apr. 1, 2022 and entitled "STEROID COMPOUND AND USE THEREOF". The prior applications described above are incorporated herein by reference in their entirety.

## TECHNICAL FIELD

The present invention relates to a novel glucocorticoid receptor agonist, a preparation method therefor, a pharmaceutical composition containing the same, and use thereof in treating tumors, inflammatory diseases, or autoimmune diseases.

## BACKGROUND

The glucocorticoid receptor (GR) is a part of the nuclear receptor family. GR is expressed in almost every cell in the human body and regulates genes that control development, metabolism, and immune response. When the glucocorticoid receptor binds to a glucocorticoid, its main mechanism of action is to regulate gene transcription. The direct route: glucocorticoids enter the cytoplasm via the cell membrane and bind to GR within the cytoplasm, and the GR is activated, enters the nucleus, and binds to GR response elements (GRE) on DNA, which initiates or inhibits mRNA transcription of the corresponding gene. For example, mRNA transcription of TNF$\alpha$, IL-1$\beta$, IL-2, IL-6 and the like is inhibited, and mRNA transcription of IκB (capable of inhibiting the activation of NF-$\kappa$B) is started. The indirect route: interacting with other transcription factors, etc., and performing anti-inflammatory and immune effects.

Glucocorticoid receptor agonists can be classified into endogenous or artificially synthesized ones according to sources. The synthetic glucocorticoid receptor agonists are a class of potent small molecule drugs used in the treatment of inflammatory diseases and immune diseases. Glucocorticoids that have been marketed include prednisone, prednisolone, betamethasone, dexamethasone, fluticasone propionate, budesonide, and the like. Glucocorticoid receptor agonists can be used for controlling inflammation and allergic diseases such as asthma, rheumatoid arthritis, obstructive airways disease, rhinitis, inflammatory bowel disease, psoriasis, eczema, and the like. However, if the medicine is taken for a long time at a supraphysiological dose, side effects such as muscle atrophy, osteoporosis and the like may appear, so that the dose and the duration of medication of the medicine for patients are limited, and the therapeutic potential of the medicine has not yet been effectively exploited. Therefore, there is a need to develop new glucocorticoid receptor agonists, which can treat more diseases and reduce toxic and side effects.

## SUMMARY

The present invention provides a compound of formula (I) or a pharmaceutically acceptable salt thereof:

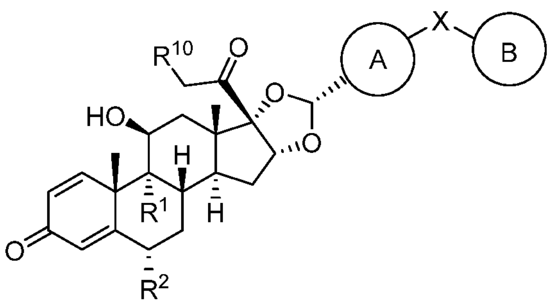

(I)

wherein

$R^1$ and $R^2$ are each independently selected from H, $CH_3$ or halogen;
ring A is selected from phenyl, 5-10 membered heteroaryl, or $C_3$-$C_{10}$ cycloalkyl, wherein the phenyl, 5-10 membered

heteroaryl, or $C_3$-$C_{10}$ cycloalkyl is optionally substituted with $R^{1a}$;
X is selected from O, S, $C_1$-$C_3$ alkylene-O, $C_1$-$C_3$ alkylene-S, $NR^6$, or $C(R^7)(R^8)$;
$R^6$ is selected from H, $C_1$-$C_6$ alkyl, $C_3$-$C_6$ cycloalkyl, or 4-7 membered heterocyclyl, wherein the $C_1$-$C_6$ alkyl, $C_3$-$C_6$ cycloalkyl, or 4-7 membered heterocyclyl is optionally substituted with $R^b$;
$R^7$ and $R^8$ are each independently selected from H, halogen, CN, OH, $NH_2$, $C_1$-$C_6$ alkyl, $C_3$-$C_6$ cycloalkyl, or 4-7 membered heterocyclyl, or $R^7$ and $R^8$, together with the atom linked thereto, form $C_3$-$C_6$ cycloalkyl or 4-7 membered heterocyclyl, wherein the OH, $NH_2$, $C_1$-$C_6$ alkyl, $C_3$-$C_6$ cycloalkyl, or 4-7 membered heterocyclyl is optionally substituted with $R^b$;
ring B is selected from $C_6$-$C_{10}$ aryl, 5-10 membered heteroaryl, $C_3$-$C_{10}$ cycloalkyl, or 4-14 membered heterocyclyl, wherein the $C_6$-$C_{10}$ aryl, 5-10 membered heteroaryl, $C_3$-$C_{10}$ cycloalkyl, or 4-14 membered heterocyclyl is optionally substituted with $R^{2a}$;
$R^{10}$ is selected from OH, SH, O($C_1$-$C_6$ alkyl), S($C_1$-$C_6$ alkyl), O-C(=O)-($C_1$-$C_6$ alkyl), or

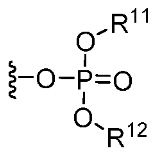
,

wherein the O($C_1$-$C_6$ alkyl), S($C_1$-$C_6$ alkyl), or O-C(=O)-($C_1$-$C_6$ alkyl) is optionally substituted with halogen or CN;
$R^{11}$ and $R^{12}$ are each independently selected from H or $C_1$-$C_6$ alkyl;
$R^{1a}$ and $R^{2a}$ are each independently selected from halogen, CN, =O, OH, $NH_2$, $C_1$-$C_6$ alkyl, $C_3$-$C_6$ cycloalkyl, or 4-7 membered heterocyclyl, wherein the OH, $NH_2$, $C_1$-$C_6$ alkyl, $C_3$-$C_6$ cycloalkyl, or 4-7 membered heterocyclyl is optionally substituted with $R^b$;
each $R^b$ is independently selected from halogen, CN, =O, OH, $NH_2$, $C_1$-$C_6$ alkyl, $C_3$-$C_6$ cycloalkyl, or 4-7 membered heterocyclyl, wherein the OH, $NH_2$, $C_1$-$C_6$ alkyl, $C_3$-$C_6$ cycloalkyl, or 4-7 membered heterocyclyl is optionally substituted with $R^c$;
each $R^c$ is independently selected from halogen, CN, =O, $C_1$-$C_3$ alkyl, OH, O($C_1$-$C_3$ alkyl), $NH_2$, NH($C_1$-$C_3$ alkyl), or N($C_1$-$C_3$ alkyl)$_2$.

In some embodiments, $R^1$ and $R^2$ are each independently selected from H or F.
In some embodiments, ring A is selected from phenyl or 5-6 membered heteroaryl, wherein the phenyl or 5-6 membered heteroaryl is optionally substituted with $R^{1a}$.
In some embodiments, ring A is selected from phenyl, wherein the phenyl is optionally substituted with $R^{1a}$.
In some embodiments, $R^{1a}$ is selected from halogen, CN, =O, OH, $NH_2$, or $C_1$-$C_6$ alkyl.
In some embodiments, $R^{1a}$ is selected from $NH_2$.
In some embodiments, ring A is selected from

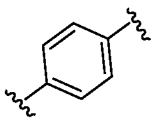
or
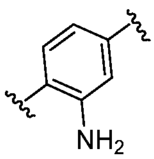
.

In some embodiments, ring A is selected from

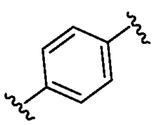
.

In some embodiments, X is selected from O, S, $C_1$-$C_3$ alkylene-O, $C_1$-$C_3$ alkylene-S, or $C(R^7)(R^8)$.
In some embodiments, $R^7$ and $R^8$ are each independently selected from H, halogen, CN, OH, $NH_2$, or $C_1$-$C_3$ alkyl, or $R^7$ and $R^8$, together with the atom linked thereto, form $C_3$-$C_6$ cycloalkyl, wherein the OH, $NH_2$, $C_1$-$C_3$ alkyl, or $C_3$-$C_6$ cycloalkyl is optionally substituted with $R^b$.

In some embodiments, $R^7$ and $R^8$ are each independently selected from H, halogen, or $C_1$-$C_3$ alkyl, or $R^7$ and $R^8$, together with the atom linked thereto, form $C_3$-$C_6$ cycloalkyl.
In some embodiments, $R^7$ and $R^8$ are each independently selected from H, F, or methyl, or $R^7$ and $R^8$, together with the atom linked thereto, form cyclopropyl.
In some embodiments, X is selected from O, S, $CH_2O$, $CH_2S$, $CH_2$, $CF_2$, $CHCH_3$, or

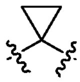

.

In some embodiments, X is selected from $CH_2$.
In some embodiments, $R^{10}$ is selected from OH, SH, O($C_1$-$C_3$ alkyl), O-C(=O)-($C_1$-$C_3$ alkyl), or

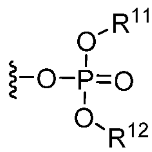

,

wherein the O($C_1$-$C_3$ alkyl) or O-C(=O)-($C_1$-$C_3$ alkyl) is optionally substituted with halogen. In some embodiments, the O($C_1$-$C_3$ alkyl) is optionally substituted with halogen.
In some embodiments, $R^{11}$ and $R^{12}$ are each independently selected from H, methyl, or ethyl.
In some embodiments, $R^{10}$ is selected from OH, SH, $OCH_2F$, $O(C=O)CH_3$,

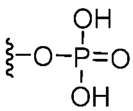

,

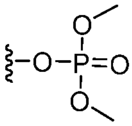

,

or

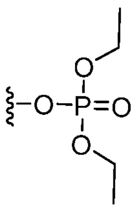

.

In some embodiments, $R^{10}$ is selected from OH or

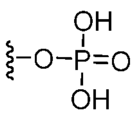

.

In some embodiments, ring B is selected from $C_6$-$C_{10}$ aryl, 5-10 membered heteroaryl, or 4-14 membered heterocyclyl, wherein the $C_6$-$C_{10}$ aryl, 5-10 membered heteroaryl, or 4-14 membered heterocyclyl is optionally substituted with $R^{2a}$.
In some embodiments, ring B is selected from 5-10 membered heteroaryl or 4-14 membered heterocyclyl, wherein the 5-10 membered heteroaryl or 4-14 membered heterocyclyl is optionally substituted with $R^{2a}$.
In some embodiments, ring A is substituted with $R^{1a}$ and/or ring B is substituted with $R^{2a}$.
In some embodiments, ring A is substituted with $R^{1a}$ or ring B is substituted with $R^{2a}$. In some embodiments, $R^{2a}$ is selected from halogen, CN, =O, OH, $NH_2$, $C_1$-$C_6$ alkyl, $C_3$-$C_6$ cycloalkyl, or 4-7 membered heterocyclyl.
In some embodiments, $R^{2a}$ is selected from $NH_2$ or =O.
In some embodiments, $R^{2a}$ is selected from $NH_2$. In some embodiments, the compound of formula (I) or the

pharmaceutically acceptable salt thereof is selected from a compound of formula (Ia) or a pharmaceutically acceptable salt thereof:

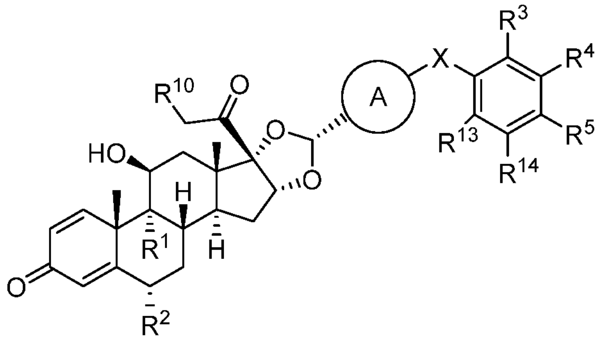

(Ia)

wherein $R^1$, $R^2$, ring A, X, and $R^{10}$ are as defined above;

$R^3$ is selected from H, OH, or $NHR^9$, $R^4$ and $R^5$, together with the atom linked thereto, form $C_5$-$C_6$ cycloalkenyl, 5-6 membered heterocyclyl, or 5-6 membered heteroaryl, wherein the $C_5$-$C_6$ cycloalkenyl, 5-6 membered heterocyclyl, or 5-6 membered heteroaryl is optionally substituted with $R^{4a}$; or $R^5$ is selected from H, OH, or $NHR^9$, and $R^3$ and $R^4$, together with the atom linked thereto, form $C_5$-$C_6$ cycloalkenyl, 5-6 membered heterocyclyl, or 5-6 membered heteroaryl, wherein the $C_5$-$C_6$ cycloalkenyl, 5-6 membered heterocyclyl, or 5-6 membered heteroaryl is optionally substituted with $R^{4a}$;

$R^9$ is selected from H, $C_1$-$C_6$ alkyl, $C_3$-$C_6$ cycloalkyl, or 4-7 membered heterocyclyl, wherein the $C_1$-$C_6$ alkyl, $C_3$-$C_6$ cycloalkyl, or 4-7 membered heterocyclyl is optionally substituted with $R^d$;

$R^{13}$ and $R^{14}$ are each independently selected from H, halogen, CN, OH, $NH_2$, O($C_1$-$C_3$ alkyl), or $C_1$-$C_6$ alkyl;

each $R^{4a}$ is independently selected from halogen, CN, =O, OH, $NH_2$, $C_1$-$C_6$ alkyl, $C_3$-$C_6$ cycloalkyl, or 4-7 membered heterocyclyl, wherein the OH, $NH_2$, $C_1$-$C_6$ alkyl, $C_3$-$C_6$ cycloalkyl, or 4-7 membered heterocyclyl is optionally substituted with $R^d$;

each $R^d$ is independently selected from halogen, CN, =O, $C_1$-$C_3$ alkyl, OH, O($C_1$-$C_3$ alkyl), $NH_2$, NH($C_1$-$C_3$ alkyl), or N($C_1$-$C_3$ alkyl)$_2$.

In some embodiments, $R^3$ is selected from H, OH, or $NHR^9$, and $R^4$ and $R^5$, together with the atom linked thereto, form 5-6 membered heterocyclyl or 5-6 membered heteroaryl, wherein the 5-6 membered heterocyclyl or 5-6 membered heteroaryl is optionally substituted with $R^{4a}$; or $R^5$ is selected from H, OH, or $NHR^9$, and $R^3$ and $R^4$, together with the atom linked thereto, form 5-6 membered heterocyclyl or 5-6 membered heteroaryl, wherein the 5-6 membered heterocyclyl or 5-6 membered heteroaryl is optionally substituted with $R^{4a}$.

In some embodiments, $R^9$ is selected from H or $C_1$-$C_6$ alkyl.

In some embodiments, $R^9$ is selected from H.

In some embodiments, $R^3$ is selected from H, OH, or $NH_2$, and $R^4$ and $R^5$, together with the atom linked thereto, form 5-6 membered heterocyclyl or 5-6 membered heteroaryl, wherein the 5-6 membered heterocyclyl or 5-6 membered heteroaryl is optionally substituted with $R^{4a}$.

In some embodiments, $R^5$ is selected from H, OH, or $NH_2$, and $R^3$ and $R^4$, together with the atom linked thereto, form $C_5$-$C_6$ cycloalkenyl, 5-6 membered heterocyclyl, or 5-6 membered heteroaryl, wherein the $C_5$-$C_6$ cycloalkenyl, 5-6 membered heterocyclyl, or 5-6 membered heteroaryl is optionally substituted with $R^{4a}$.

In some embodiments, $R^5$ is selected from H, OH or $NH_2$, and $R^3$ and $R^4$, together with the atom linked thereto, form 5-6 membered heterocyclyl or 5-6 membered heteroaryl, wherein the 5-6 membered heterocyclyl or 5-6 membered heteroaryl is optionally substituted with $R^{4a}$.

In some embodiments, $R^3$ is selected from H, and $R^4$ and $R^5$, together with the atom linked thereto, form 5-6 membered heterocyclyl or 5-6 membered heteroaryl, wherein the 5-6 membered heterocyclyl or 5-6 membered heteroaryl is optionally substituted with $R^{4a}$.

In some embodiments, $R^5$ is selected from H or $NH_2$, and $R^3$ and $R^4$, together with the atom linked thereto, form $C_5$-$C_6$ cycloalkenyl, 5-6 membered heterocyclyl, or 5-6 membered heteroaryl, wherein the $C_5$-$C_6$ cycloalkenyl, 5-6 membered heterocyclyl, or 5-6 membered heteroaryl is optionally substituted with $R^{4a}$.

In some embodiments, $R^5$ is selected from H or $NH_2$, and $R^3$ and $R^4$, together with the atom linked thereto, form 5-6 membered heterocyclyl or 5-6 membered heteroaryl, wherein the 5-6 membered heterocyclyl or 5-6 membered heteroaryl is optionally substituted with $R^{4a}$.

In some embodiments, $R^3$ is selected from H, OH, or $NH_2$, and $R^4$ and $R^5$, together with the atom linked thereto, form

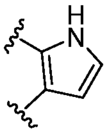, 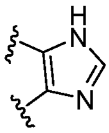, 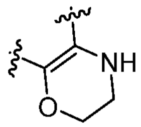, 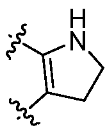, or 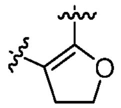,

wherein the

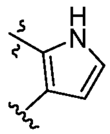,

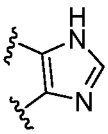, 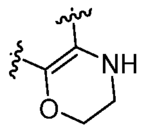, 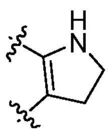, or 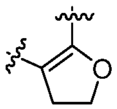

is optionally substituted with $R^{4a}$.

In some embodiments, $R^3$ is selected from H, and $R^4$ and $R^5$, together with the atom linked thereto, form

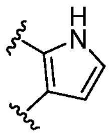, 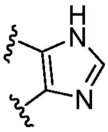, 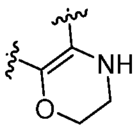, 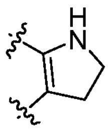, or 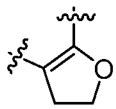,

wherein the

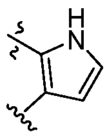, 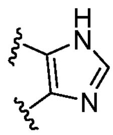,

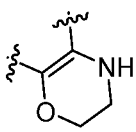, 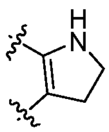, or 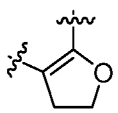

is optionally substituted with $R^{4a}$.

In some embodiments, $R^5$ is selected from H, OH, or $NH_2$, and $R^3$ and $R^4$, together with the atom linked thereto, form

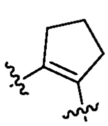, 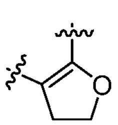, 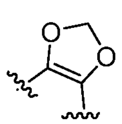, 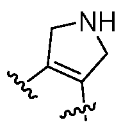, 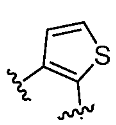, 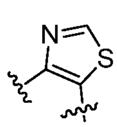, 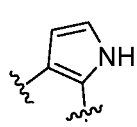,

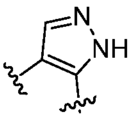, 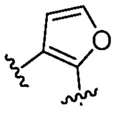, or 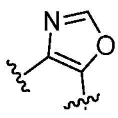,

wherein the

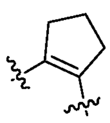, 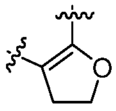, 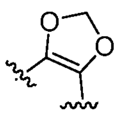, 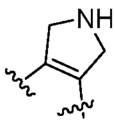,

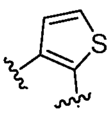, 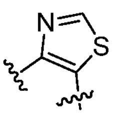, 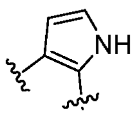, 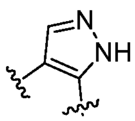, 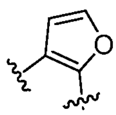, or 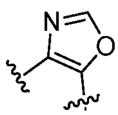

is optionally substituted with $R^{4a}$.

In some embodiments, $R^5$ is selected from H, OH, or $NH_2$, and $R^3$ and $R^4$, together with the atom linked thereto, form

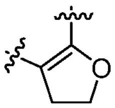, 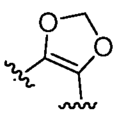, 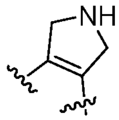, 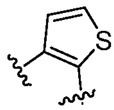, 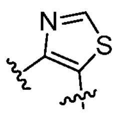, 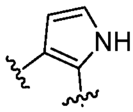, 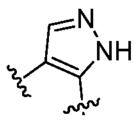,

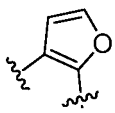, or 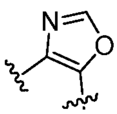,

wherein the

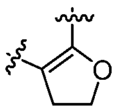, 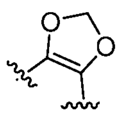, 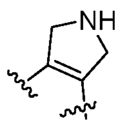, 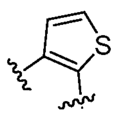, 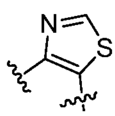,

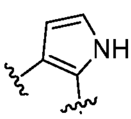, 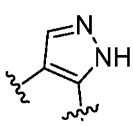, 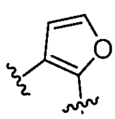, or 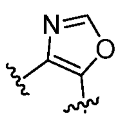

is optionally substituted with $R^{4a}$.

In some embodiments, $R^5$ is selected from H, OH, or $NH_2$, and $R^3$ and $R^4$, together with the atom linked thereto, form

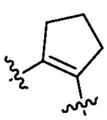, 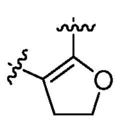, 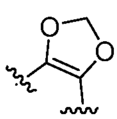, 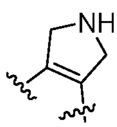, 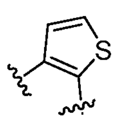, 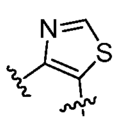, 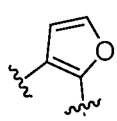,

or

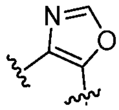,

wherein the

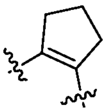, 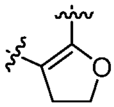, 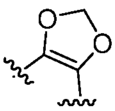, 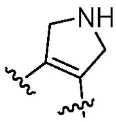, 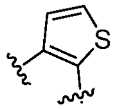, 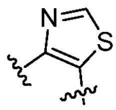, 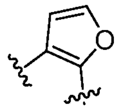,

or

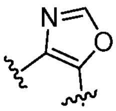

is optionally substituted with $R^{4a}$.

In some embodiments, $R^5$ is selected from H or $NH_2$, and $R^3$ and $R^4$, together with the atom linked thereto, form

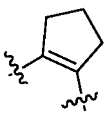, 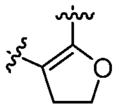, 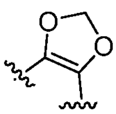, 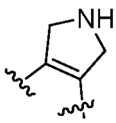, 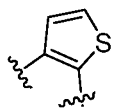, 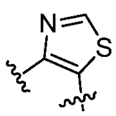, 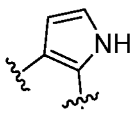,

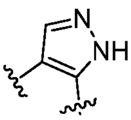, 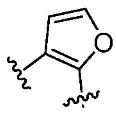, or 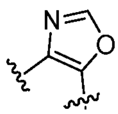,

wherein the

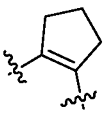, 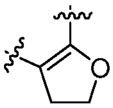, 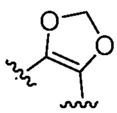, 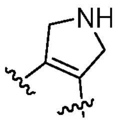,

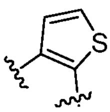, 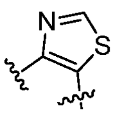, 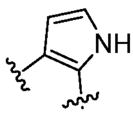, 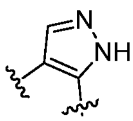, 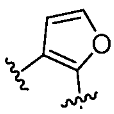, or 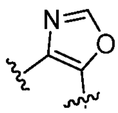

is optionally substituted with $R^{4a}$.

In some embodiments, $R^5$ is selected from H or $NH_2$, and $R^3$ and $R^4$, together with the atom linked thereto, form

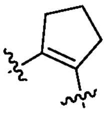, 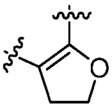, 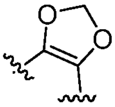, 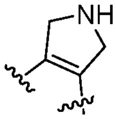, 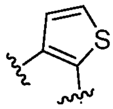, 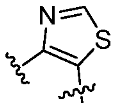, 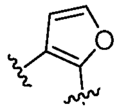,

or

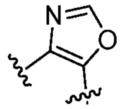,

wherein the

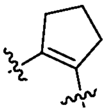, 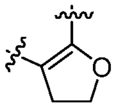, 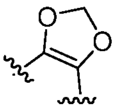, 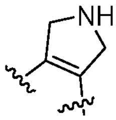, 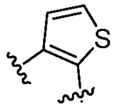, 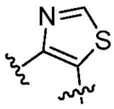, 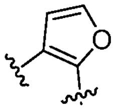,

or

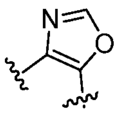

is optionally substituted with $R^{4a}$.

In some embodiments, $R^5$ is selected from H or $NH_2$, and $R^3$ and $R^4$, together with the atom linked thereto, form

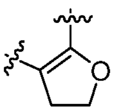, 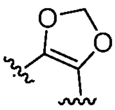, 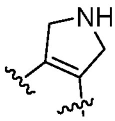, 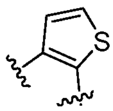, 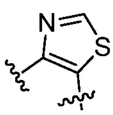, 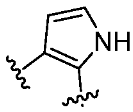, 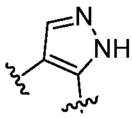,

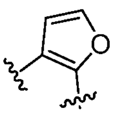, or 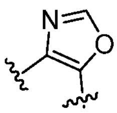,

wherein the

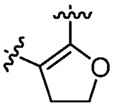, 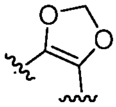, 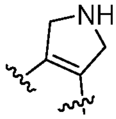, 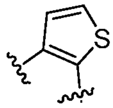, 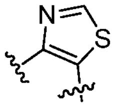,

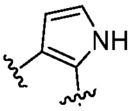, 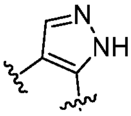, 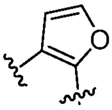, or 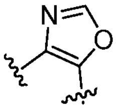

is optionally substituted with $R^{4a}$.

In some embodiments, $R^5$ is selected from H or $NH_2$, and $R^3$ and $R^4$, together with the atom linked thereto, form

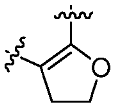, 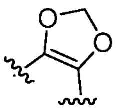, 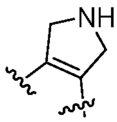, 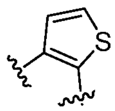, 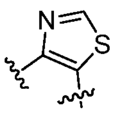, 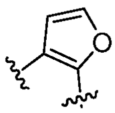, or 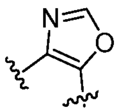,

wherein the

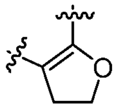, 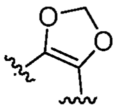, 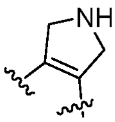, 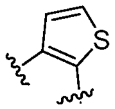, 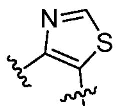, 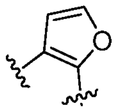, or 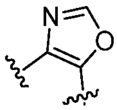

is optionally substituted with $R^{4a}$.

In some embodiments, $R^{4a}$ is selected from halogen, CN, =O, OH, $NH_2$, $C_1$-$C_6$ alkyl, or $C_3$-$C_6$ cycloalkyl.

In some embodiments, $R^{4a}$ is selected from halogen, CN, =O, OH, $NH_2$, or $C_1$-$C_3$ alkyl.

In some embodiments, $R^{4a}$ is selected from =O or $NH_2$.

In some embodiments, $R^3$ is selected from H, and $R^4$ and $R^5$, together with the atom linked thereto, form

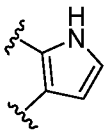, 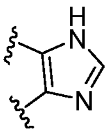, 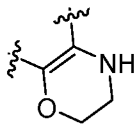, 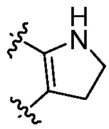, or 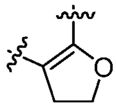.

In some embodiments, $R^3$ is selected from H, and $R^4$ and $R^5$, together with the atom linked thereto, form

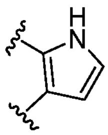, 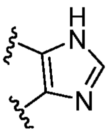, 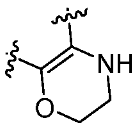, or 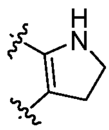.

In some embodiments, $R^5$ is selected from $NH_2$, and $R^3$ and $R^4$, together with the atom linked thereto, form

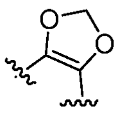, 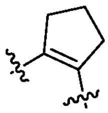, 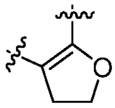, or 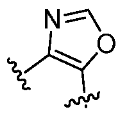.

In some embodiments, $R^5$ is selected from $NH_2$, and $R^3$ and $R^4$, together with the atom linked thereto, form

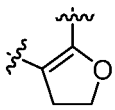 or 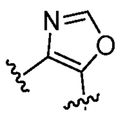.

In some embodiments, $R^{13}$ and $R^{14}$ are each independently selected from H or $NH_2$.

In some embodiments, $R^{13}$ is selected from H.

In some embodiments, $R^{14}$ is selected from H or $NH_2$.

In some embodiments, $R^{14}$ is selected from H.

In some embodiments,

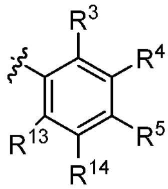

is selected from

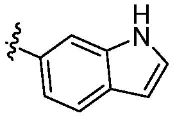 , 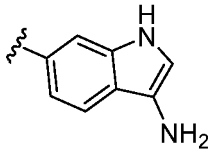 ,

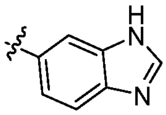 , 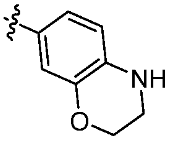 , 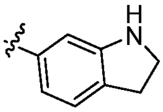 , 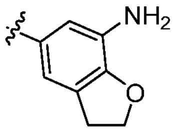 , 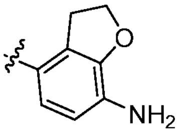 , 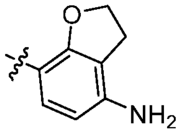 ,

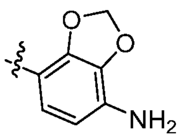 , 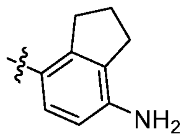 , 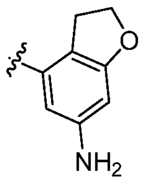 , 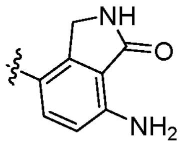 , 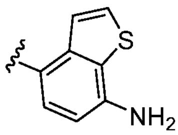 , 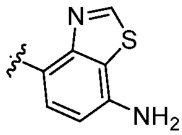 ,

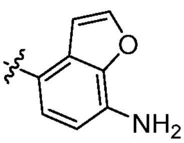 , 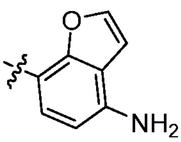 , 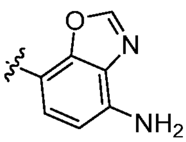 , 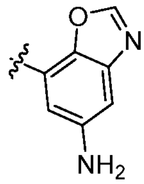 , or 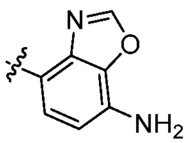 .

In some embodiments,

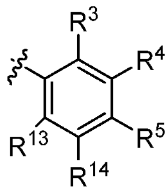

is selected from

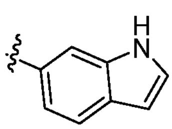 , 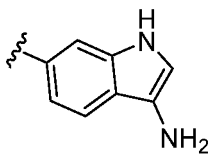 ,

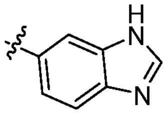, 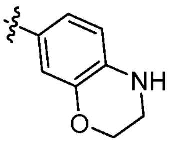, 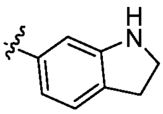, 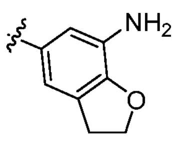, 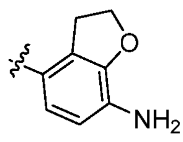, 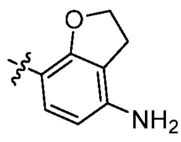,

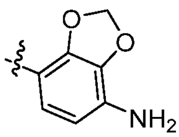, 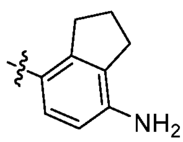, 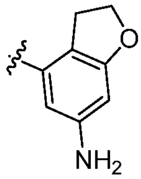, 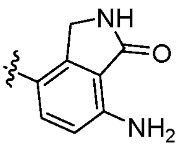, 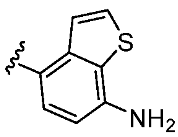, 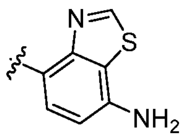,

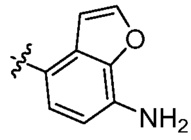, 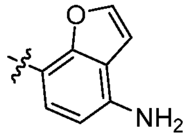, 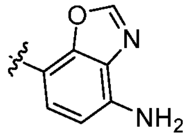, or 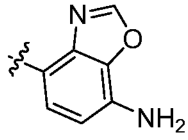.

In some embodiments,

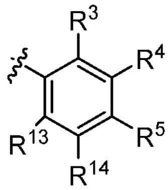

is selected from

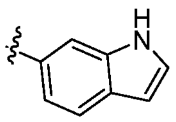, 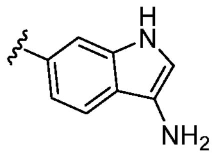,

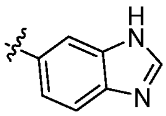, 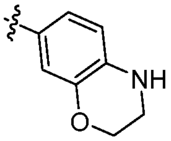, 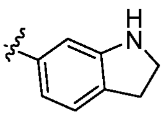, 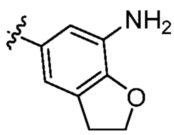, 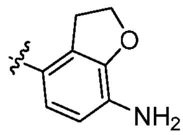, 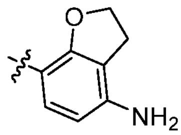,

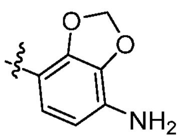, 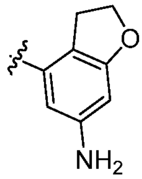, 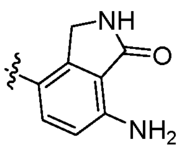, 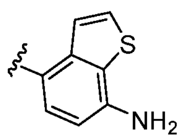, 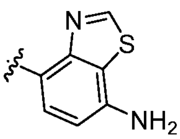, 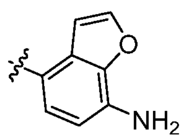,

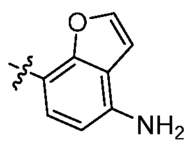, 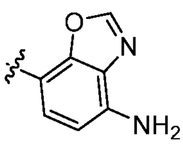, or 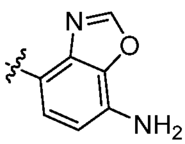.

In some embodiments,

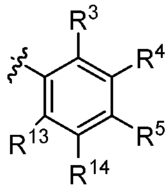

is selected from

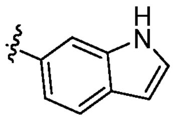, 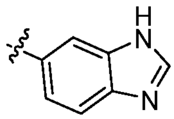, 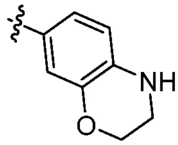,

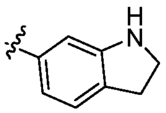, 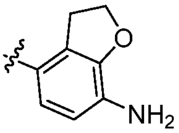, 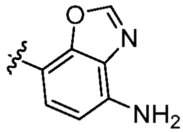, 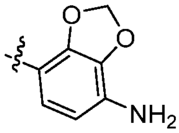, 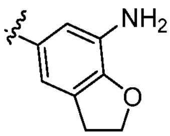, 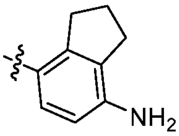,

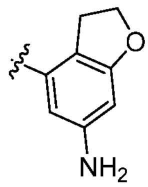, or 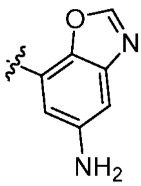.

In some embodiments,

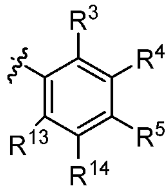

is selected from

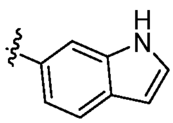, 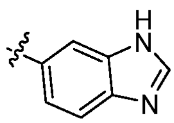, 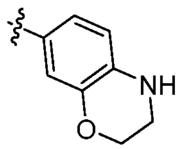,

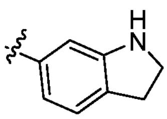, 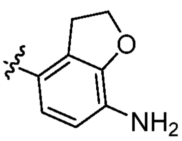, 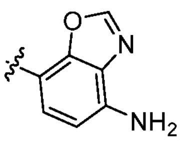, 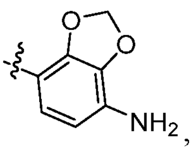, 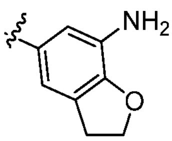, or 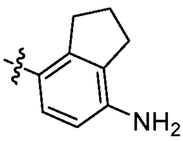.

In some embodiments,

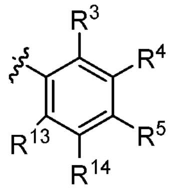

is selected from

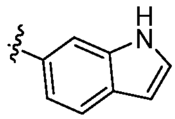, 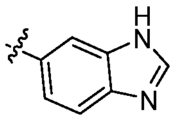, 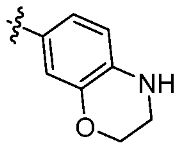,

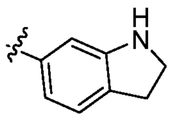, 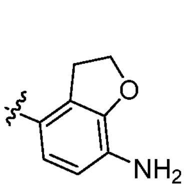, or 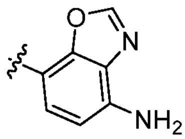.

Without conflict, it should be understood that the embodiments described above may be combined arbitrarily, forming a technical solution that includes the features of the combined embodiments. Such combined technical solutions are within the scope of the present invention.

In some embodiments, the compound of formula (I) or the pharmaceutically acceptable salt thereof is selected from the following compounds or pharmaceutically acceptable salts thereof:

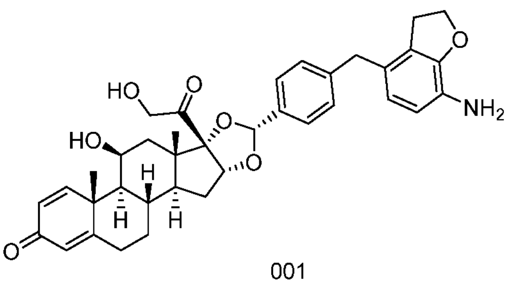

001

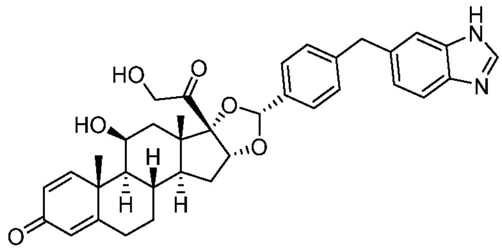

002

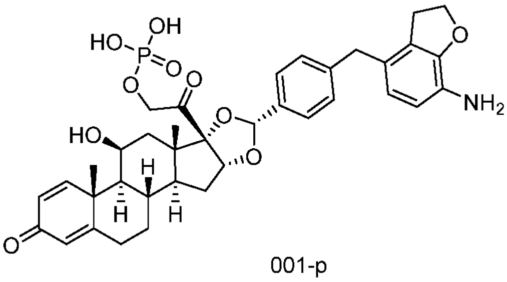

001-p

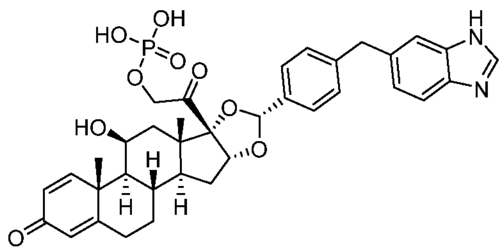

002-p

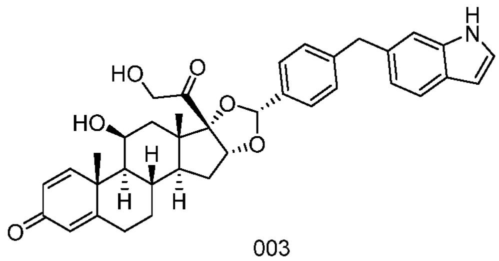

003

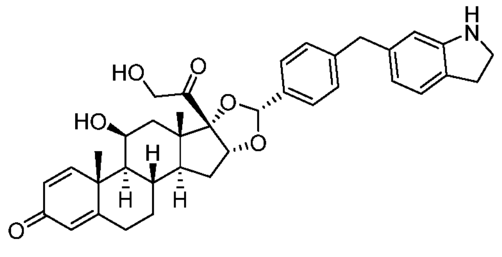

004

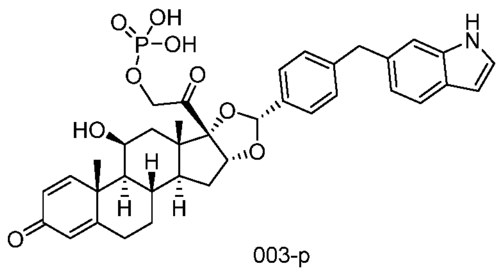

003-p

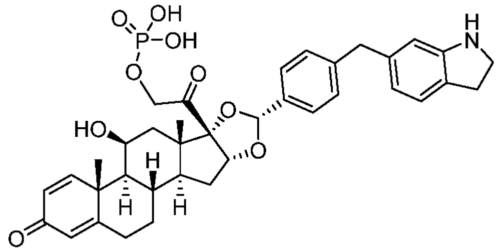

004-p

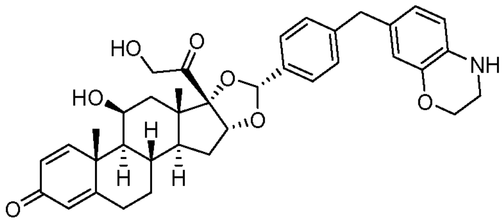

005

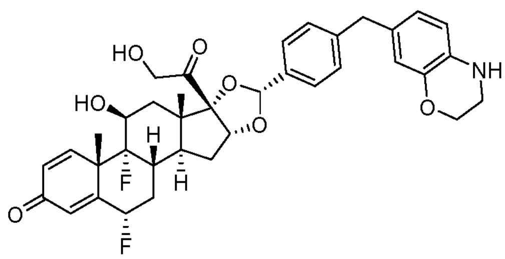

006

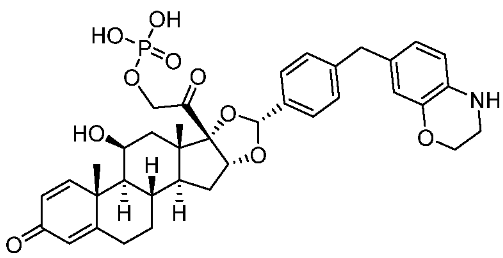

005-p

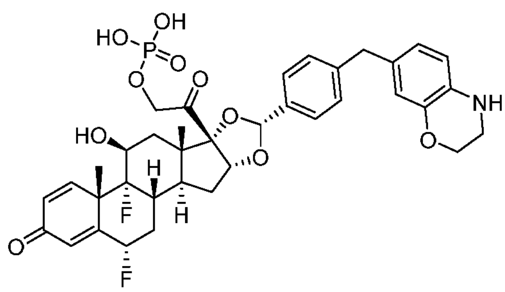

006-p

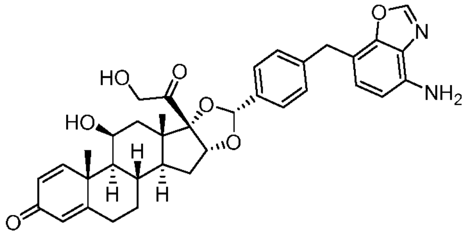

007

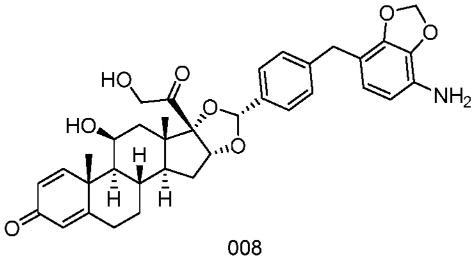

008

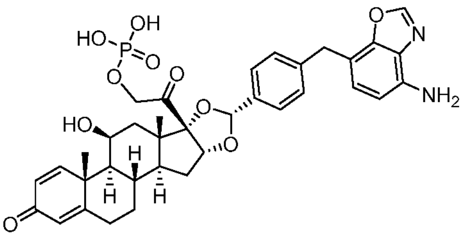

007-p

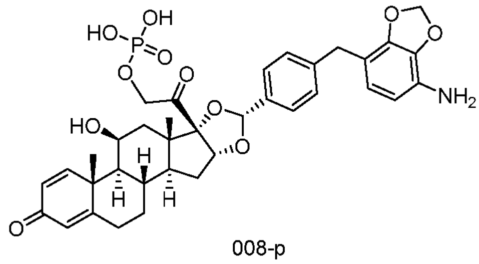

008-p

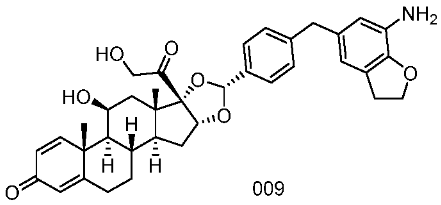

009

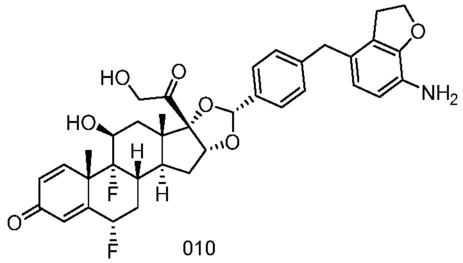

010

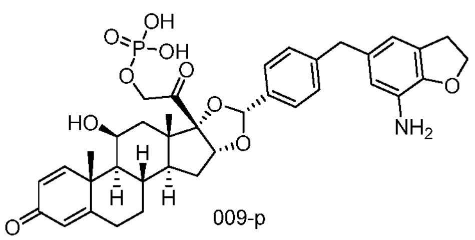

009-p

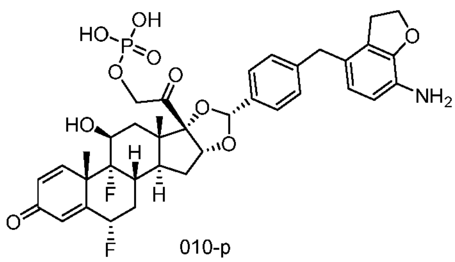

010-p

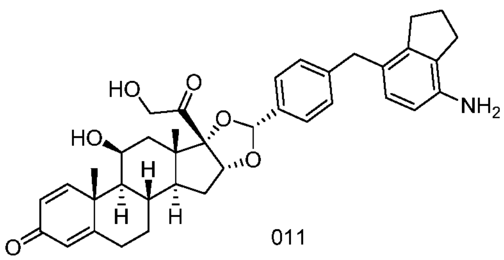

011

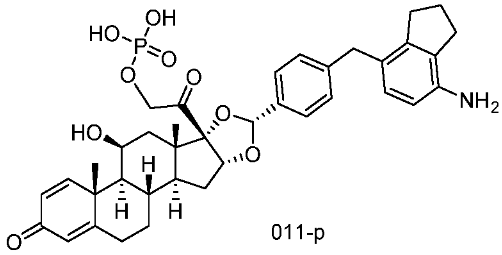

011-p

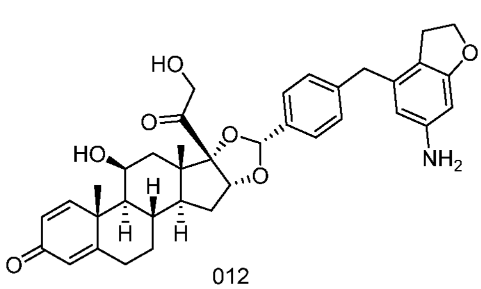

012

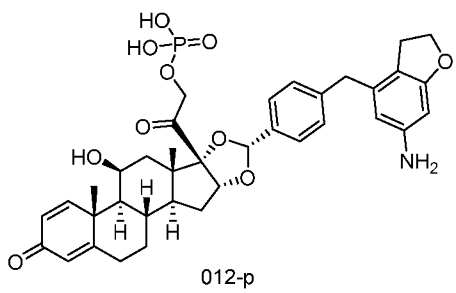

012-p

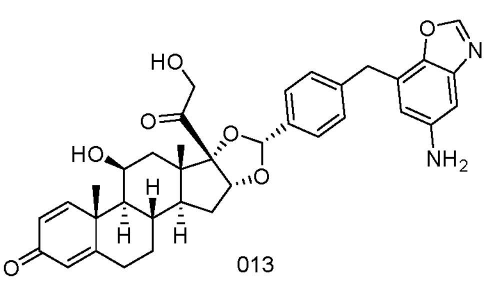

013

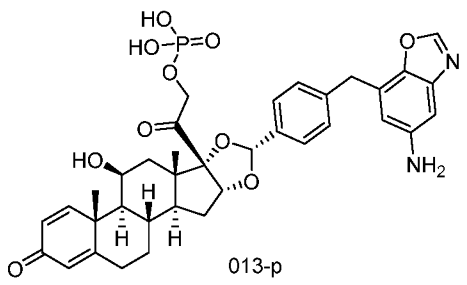

013-p

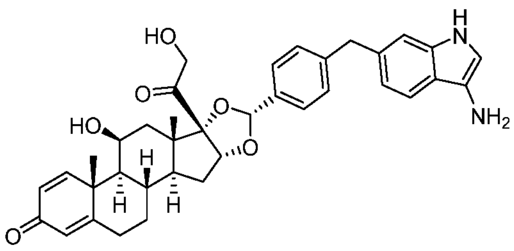

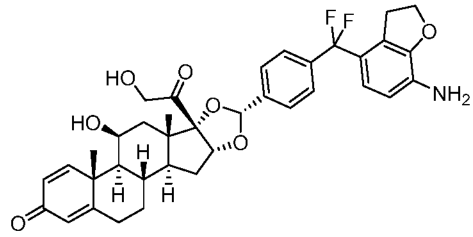

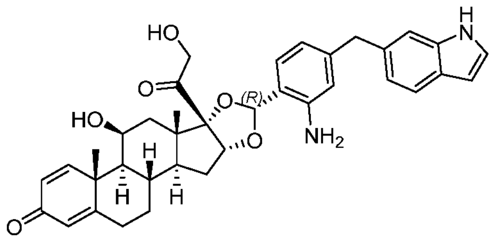

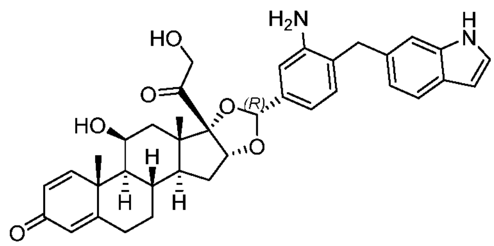

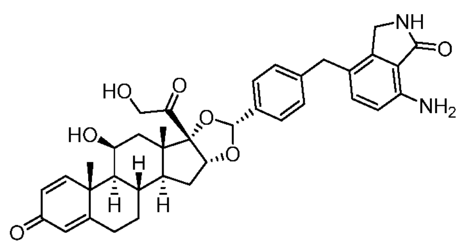

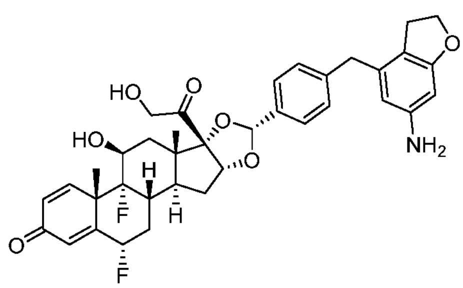

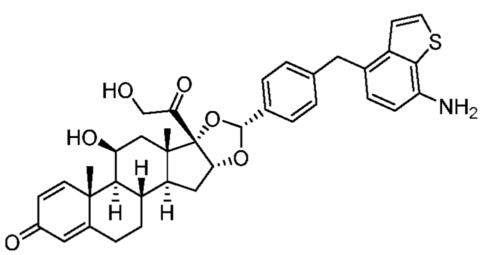

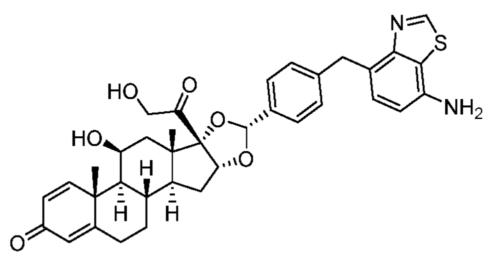

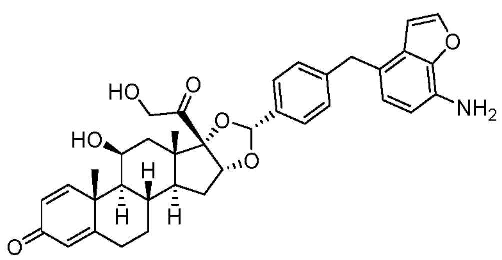

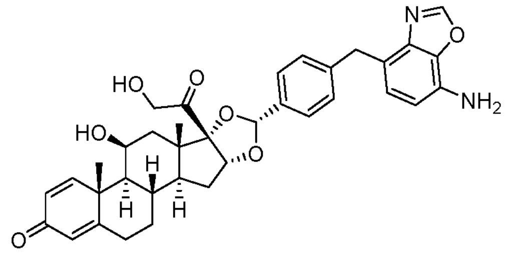

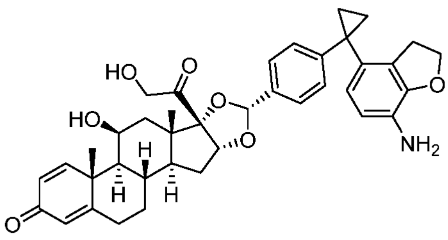

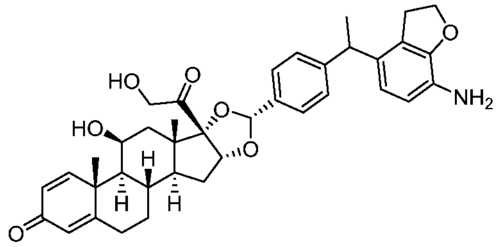

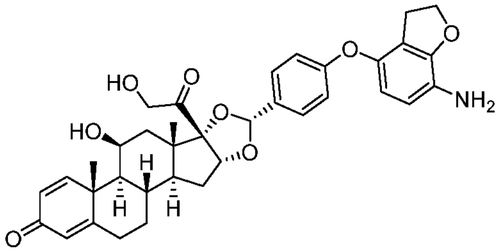

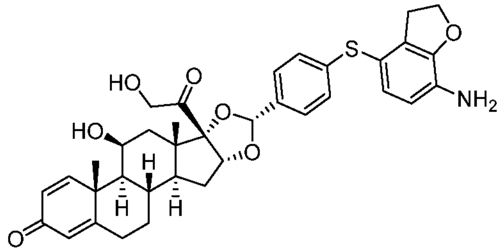

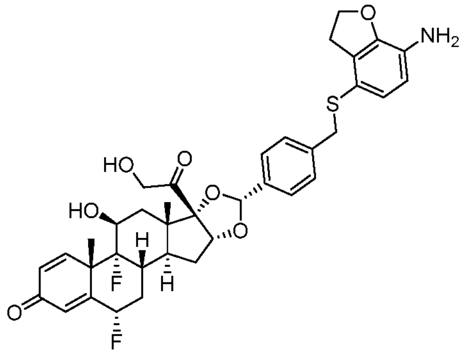

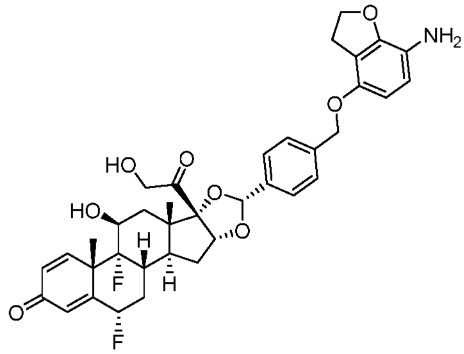

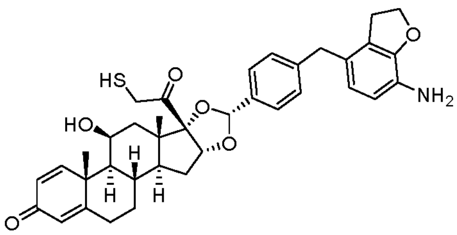

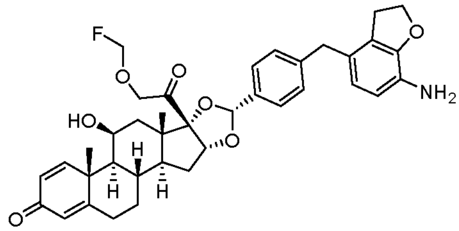

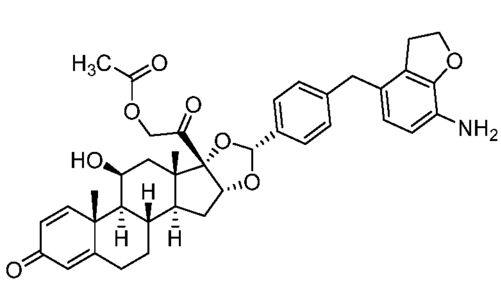

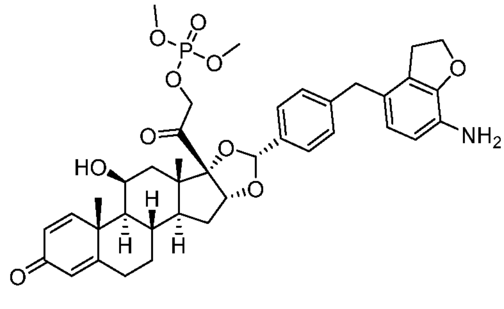

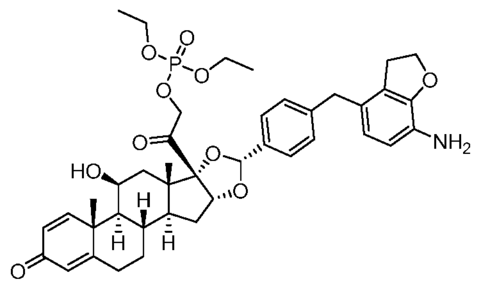
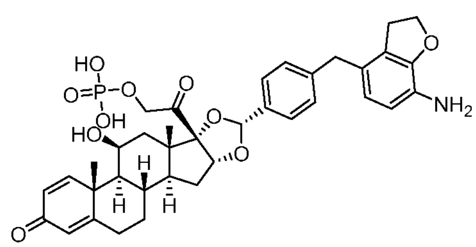
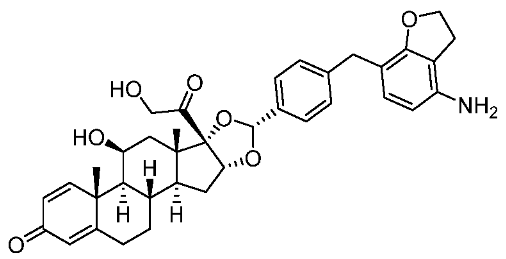
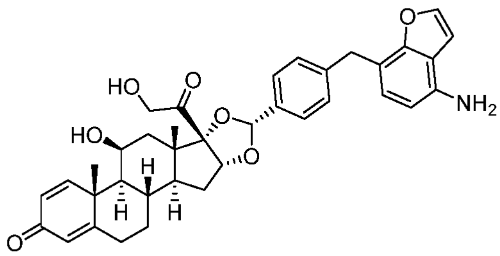

or

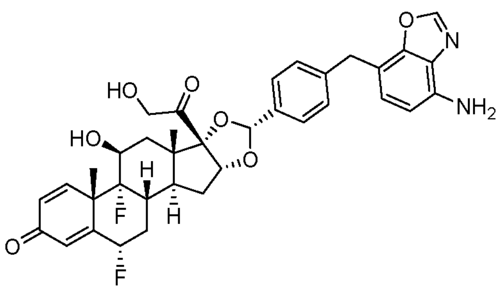

In another aspect, the present invention provides a pharmaceutical composition comprising the compound of formula (I) or the pharmaceutically acceptable salt thereof disclosed herein and a pharmaceutically acceptable excipient.

In another aspect, the present invention provides a method for treating a disease mediated by a glucocorticoid receptor in a subject (e.g., a mammal), comprising administering to a subject (e.g., a mammal, preferably a human) in need of such treatment a therapeutically effective amount of the compound of formula (I) or the pharmaceutically acceptable salt thereof, or the pharmaceutical composition thereof.

In another aspect, the present invention provides use of the compound of formula (I) or the pharmaceutically acceptable salt thereof, or the pharmaceutical composition thereof in preparing a medicament for use in preventing or treating a disease mediated by a glucocorticoid receptor.

In another aspect, the present invention provides use of the compound of formula (I) or the pharmaceutically acceptable salt thereof, or the pharmaceutical composition thereof in preventing or treating a disease mediated by a glucocorticoid receptor.

In another aspect, the present invention provides the compound of formula (I) or the pharmaceutically acceptable salt thereof, or the pharmaceutical composition thereof for use in preventing or treating a disease mediated by a glucocorticoid receptor.

In another aspect, the present invention provides a method for treating a tumor, an inflammatory disease, or an autoimmune disease in a subject (e.g., a mammal), comprising administering to a subject (e.g., a mammal, preferably a human) in need of such treatment a therapeutically effective amount of the compound of formula (I) or the pharmaceutically acceptable salt thereof, or the pharmaceutical composition thereof.

In another aspect, the present invention provides use of the compound of formula (I) or the pharmaceutically acceptable salt thereof, or the pharmaceutical composition thereof in preparing a medicament for use in treating a tumor, an inflammatory disease, or an autoimmune disease.

In another aspect, the present invention provides use of the compound of formula (I) or the pharmaceutically acceptable salt thereof, or the pharmaceutical composition thereof in treating a tumor, an inflammatory disease, or an autoimmune disease.

In another aspect, the present invention provides the compound of formula (I) or the pharmaceutically acceptable salt thereof, or the pharmaceutical composition thereof for use in treating a tumor, an inflammatory disease, or an autoimmune disease.

In some embodiments, the disease mediated by a glucocorticoid receptor is selected from a tumor, an inflammatory disease, or an autoimmune disease.

BENEFICIAL EFFECTS OF PRESENT INVENTION

The compounds of the present invention have strong binding activity to glucocorticoid receptors and weak or inactive binding to other hormone receptors, and the strong selectivity of the compounds of the present invention is expected to reduce the side effects associated with acting on other hormone receptors. The compounds of the present invention also have good metabolic stability in liver microsomes. In addition, the compounds of the present invention have a low potential risk of drug-drug interaction (DDI) and have no obvious inhibitory effect on the hERG potassium channel, which suggests that the compounds of the present invention have a low risk of cardiotoxicity due to the inhibition of hERG potassium channel.

TERMINOLOGY AND DEFINITIONS

Unless otherwise stated, the terms used in the present invention have the following meanings, and the definitions of groups and terms described in the present invention, including definitions thereof as examples, exemplary definitions, preferred definitions, definitions documented in tables, definitions of specific compounds in the examples, and the like, may be arbitrarily combined and incorporated with each other. A certain term, unless otherwise specifically defined, should not be considered uncertain or unclear, but construed according to its common meaning in the field. When referring to a trade name, it is intended to refer to its corresponding commercial product or its active ingredient.

Herein,

“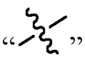”

represents a connection site.

The illustration of the pure compounds of the racemate or the enantiomer herein is from Maehr, JChem.Ed. 1985, 62: 114-120. Unless otherwise stated, the absolute configuration of a stereogenic center is represented by a wedged bond and a wedged dashed bond

(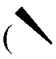 and 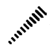),

and the relative configuration of a stereogenic center (e.g., *cis*- or *trans*-configuration of alicyclic compounds) is represented by a black solid bond and a dashed bond

(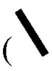 and 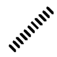).

The term "tautomer" refers to functional isomers resulting from the rapid movement of an atom in a molecule between two positions. The compounds of the present invention may exhibit the tautomerism. Tautomeric compounds may exist in two or more interconvertible forms. Tautomers generally exist in an equilibrium form. Trying to separate a single tautomer usually leads to a mixture, the physicochemical properties of which are consistent with the mixture of the compound. The position of the equilibrium depends on the chemical properties of the molecule. For example, in many aliphatic aldehydes and ketones such as acetaldehyde, the keto form predominates; whereas in phenol, the enol form predominates. In the present invention, all tautomeric forms of the compound are included.

The term "stereoisomer" refers to isomers resulting from different spatial arrangements of atoms in a molecule, including *cis-trans* isomers, enantiomers, and diastereoisomers.

The compound of the present invention may have an asymmetric atom such as a carbon atom, a sulfur atom, a nitrogen atom, a phosphorus atom, or an asymmetric double bond, and thus the compound of the present invention may exist in the form of a particular geometric isomer or stereoisomer. The form of a particular geometric isomer or stereoisomer may be *cis* and *trans* isomers, E and Z geometric isomers, (-)- and (+)- enantiomers, (R)- and (S)- enantiomers, diastereoisomers, (D)-isomers, (L)-isomers, and racemic mixtures or other mixtures thereof, such as an enantiomer or diastereoisomer enriched mixture, and all of the above isomers as well as mixtures thereof are encompassed within the definition scope of the compound of the present invention. An additional asymmetric carbon atom, asymmetric sulfur atom, asymmetric nitrogen atom, or asymmetric phosphorus atom may be present in substituents such as alkyl. All of these isomers and mixtures thereof referred to in the substituents are also encompassed within the definition scope of the compound of the present invention. The compound with asymmetric atoms disclosed herein can be separated in an optically active pure form or in a racemic form. The optically active pure form can be isolated from a racemic mixture or synthesized by using chiral starting materials or chiral reagents.

The term "substituted" means that any one or more hydrogen atoms on a specific atom are substituted by substituents, as long as the valence of the specific atom is normal and the resulting compound is stable. When the substituent is oxo (namely =O), it means that two hydrogen atoms are substituted, and oxo is not available on an aromatic group.

The term "optional" or "optionally" means that the subsequently described event or circumstance may, but not necessarily, occur. The description includes instances where the event or circumstance occurs and instances where it does not. For example, an ethyl "optionally" substituted with halogen, means that the ethyl may be unsubstituted ($CH_2CH_3$), monosubstituted ($CH_2CH_2F$, $CH_2CH_2Cl$, or the like), polysubstituted ($CHFCH_2F$, $CH_2CHF_2$, $CHFCH_2Cl$, $CH_2CHCl_2$, or the like), or fully substituted ($CF_2CF_3$, $CF_2CCl_3$, $CCl_2CCl_3$, or the like). It will be understood by those skilled in the art that for any group comprising one or more substituents, no substitution or substituting pattern that is spatially impossible and/or cannot be synthesized will be introduced.

When any variable (e.g., $R^a$ or $R^b$) occurs more than once in the constitution or structure of a compound, the variable is independently defined in each case. For example, if a group is substituted with 2 $R^b$, the definition of each $R^b$ is independent.

When the number of a linking group is 0, such as $-(CH_2)_0-$, it means that the linking group is a bond. When one of variables is selected from a chemical bond or is absent, it means that the two groups which it links are linked directly. For example, when L in A-L-Z represents a bond, it means that the structure is actually A-Z.

When the linking direction of the linking group referred to herein is not specified, the linking direction is arbitrary. For example, when X in the structural unit

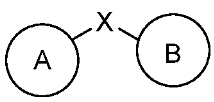

is selected from "$C_1$-$C_3$ alkylene-O", then X may link ring A and ring B in a direction from left to right to constitute "ring A-$C_1$-$C_3$ alkylene-O-ring B", or link ring A and ring B in a direction from right to left to constitute "ring A-O-$C_1$-$C_3$ alkylene-ring B".

When a bond of a substituent is cross-linked to two atoms on a ring, the substituent can be bonded to any atom on the ring. For example, the structural unit

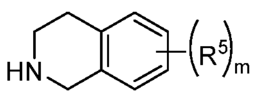

represents that $R^5$ may be substituted at any position on the benzene ring.

$C_m$-$C_n$ used herein means that the portion has an integer number of carbon atoms in the range of m-n. For example, "$C_1$-$C_{10}$" means that the group may have 1 carbon atom, 2 carbon atoms, 3 carbon atoms, 4 carbon atoms, 5 carbon atoms, 6 carbon atoms, 7 carbon atoms, 8 carbon atoms, 9 carbon atoms, or 10 carbon atoms.

The term "alkyl" refers to hydrocarbyl with a general formula of $C_nH_{2n+1}$. The alkyl may be linear or branched. The term "$C_1$-$C_6$ alkyl" may be understood to represent a linear or branched saturated hydrocarbyl having 1, 2, 3, 4, 5, or 6 carbon atoms. Specific examples of the alkyl include, but are not limited to, methyl, ethyl, n-propyl, isopropyl, n-butyl, isobutyl, sec-butyl, tert-butyl, n-pentyl, 1-methylbutyl, 2-methylbutyl, 3-methylbutyl, neopentyl, hexyl, 2-methylpentyl, and the like. The term "$C_1$-$C_3$ alkyl" may be understood to represent a linear or branched saturated alkyl group having 1-3 carbon atoms. The "$C_1$-$C_6$ alkyl" may further include "$C_1$-$C_3$ alkyl".

The term "alkoxy" refers to a group derived from a linear or branched alcohol by loss of a hydrogen atom from hydroxyl, and may be understood as "alkyloxy" or "alkyl-O-". The term "$C_1$-$C_6$ alkoxy" may be understood as "$C_1$-$C_6$ alkyloxy" or "$C_1$-$C_6$ alkyl-O-". The "$C_1$-$C_6$ alkoxy" may further include "$C_1$-$C_3$ alkoxy".

The term "cycloalkyl" refers to a fully saturated carbon ring group that exists in the form of a monocyclic ring, a fused ring, a bridged ring, a spiro ring, or the like. Unless otherwise specified, the carbon ring is generally a 3-10 membered ring (e.g., a 3, 4, 5, 6, 7, 8, 9, or 10 membered ring). The term "$C_3$-$C_{10}$ cycloalkyl" may be understood as a

saturated monocyclic, fused, spiro, or bridged ring having 3-10 carbon atoms. Specific examples of the cycloalkyl include, but are not limited to, cyclopropyl, cyclobutyl, cyclopentyl, cyclohexyl, cycloheptyl, cyclooctyl, cyclononyl, cyclodecyl, norbornyl (bicyclo[2.2.1]heptyl), bicyclo[2.2.2]octyl, adamantyl, spiro[4.5]decyl, and the like. The term "$C_3$-$C_{10}$ cycloalkyl" may include "$C_3$-$C_6$ cycloalkyl", and the term "$C_3$-$C_6$ cycloalkyl" may be understood to mean a saturated monocyclic or bicyclic hydrocarbon ring having 3-6 carbon atoms. Specific examples include, but are not limited to, cyclopropyl, cyclobutyl, cyclopentyl, cyclohexyl, or the like.

The term "cycloalkyloxy" may be understood as "cycloalkyl-O-".

The term "cycloalkenyl" refers to a non-aromatic carbon ring group that is not fully saturated, has at least one carbon-carbon double bond, and exists in the form of a monocyclic ring, a fused ring, a bridged ring, a spiro ring, or the like. Unless otherwise specified, the carbon ring is generally a 5-8 membered ring. The term "$C_5$-$C_6$ cycloalkenyl" refers to cycloalkenyl with 5 or 6 ring carbon atoms. Specific examples include, but are not limited to, cyclopentenyl, cyclopentadienyl, cyclohexenyl, cyclohexadienyl, and the like.

The term "heterocyclyl" refers to a fully saturated or partially saturated (non-aromatic heteroaromatic group on the whole) monocyclic, fused cyclic, spiro cyclic, or bridged cyclic group, and ring atoms of the group include 1-5 (e.g., 1-3 or 1-2) heteroatoms or heteroatom groups (i.e., heteroatom-containing atom groups). The "heteroatom or heteroatom group" includes, but is not limited to, a nitrogen atom (N), an oxygen atom (O), a sulfur atom (S), a phosphorus atom (P), a boron atom (B), $-S(=O)_2-$, -S(=O)-, $-P(=O)_2-$, -P(=O)-, -NH-, -S(=O)(=NH)-, -C(=O)NH-, -NHC(=O)NH-, and the like. The term "4-14 membered heterocyclyl" refers to a heterocyclyl group having a ring atom number of 4, 5, 6, 7, 8, 9, 10, 11, 12, 13, or 14, and ring atoms of the heterocyclyl group include 1-5 heteroatoms or heteroatom groups independently selected from those described above. "4-14 membered heterocyclyl" includes "4-10 membered heterocyclyl", "4-7 membered heterocyclyl", or the like, wherein specific examples of 4-membered heterocyclyl include, but are not limited to, azetidinyl or oxetanyl; specific examples of 5-membered heterocyclyl include, but are not limited to, tetrahydrofuranyl, dioxolyl, pyrrolidinyl, imidazolidinyl, pyrazolidinyl, pyrrolinyl, 4,5-dihydrooxazolyl, or 2,5-dihydro-1*H*-pyrrolyl; specific examples of 6-membered heterocyclyl include, but are not limited to, tetrahydropyranyl, piperidyl, morpholinyl, dithianyl, thiomorpholinyl, piperazinyl, trithianyl, tetrahydropyridinyl, or 4*H*-[1,3,4]thiadiazinyl; and specific examples of 7-membered heterocyclyl include, but are not limited to, diazepanyl. The heterocyclyl may also be a bicyclic group, wherein specific examples of 5,5-membered bicyclic groups include, but are not limited to, hexahydrocyclopenta[*c*]pyrrol-2(1*H*)-yl; and specific examples of 5,6-membered bicyclic groups include, but are not limited to, hexahydropyrrolo[1,2-*a*]pyrazin-2(1*H*)-yl, 5,6,7,8-tetrahydro-[1,2,4]triazolo[4,3-*a*]pyrazinyl, and 5,6,7,8-tetrahydroimidazo[1,5-*a*]pyrazinyl. Optionally, the heterocyclyl may be a benzo-fused ring group of the 4-7 membered heterocyclyl described above. Specific examples include, but are not limited to, dihydroisoquinolyl and the like. "4-10 membered heterocyclyl" may include "5-10 membered heterocyclyl", "4-7 membered heterocyclyl", "5-6 membered heterocyclyl", "6-8 membered heterocyclyl", "4-10 membered heterocycloalkyl", "5-10 membered heterocycloalkyl", "4-7 membered heterocycloalkyl", "5-6 membered heterocycloalkyl", "6-8 membered heterocycloalkyl", and the like, and the "4-7 membered heterocyclyl" may further include "4-6 membered heterocyclyl", "5-6 membered heterocyclyl", "4-7 membered heterocycloalkyl", "4-6 membered heterocycloalkyl", "5-6 membered heterocycloalkyl", and the like. Although some bicyclic heterocyclyl herein comprise, in part, a benzene ring or a heteroaromatic ring, the heterocyclyl is still non-aromatic on the whole.

The term "aryl" refers to an aromatic monocyclic or fused polycyclic group of carbon atoms with the conjugated pi-electron system. The term "$C_6$-$C_{10}$ aryl" may be understood as an aryl group having 6-10 carbon atoms, in particular a ring having 6 carbon atoms ("$C_6$ aryl"), such as phenyl, a ring having 9 carbon atoms ("$C_9$ aryl"), such as indanyl or indenyl, or a ring having 10 carbon atoms ("$C_{10}$ aryl"), such as tetrahydronaphthyl, dihydronaphthyl, or naphthyl.

The term "aryloxy" may be understood as "aryl-O-".

The term "heteroaryl" refers to an aromatic cyclic group having an aromatic monocyclic or fused polycyclic system in which at least one ring atom selected from N, O, and S is comprised, while the remaining ring atoms are C. The term "5-10 membered heteroaryl" may be understood to include an aromatic monocyclic or bicyclic ring system which has 5, 6, 7, 8, 9, or 10 ring atoms, in particular 5, 6, 9, or 10 ring atoms, and comprises 1-5, preferably 1-3, heteroatoms independently selected from N, O, and S. In particular, the heteroaryl is selected from thienyl, furanyl, pyrrolyl, oxazolyl, thiazolyl, imidazolyl, pyrazolyl, isoxazolyl, isothiazolyl, oxadiazolyl, triazolyl, thiadiazolyl, or the like, and benzo derivatives thereof, such as benzofuranyl, benzothienyl, benzothiazolyl, benzoxazolyl, benzoisoxazolyl, benzimidazolyl, benzotriazolyl, indazolyl, indolyl, isoindolyl, or the like; or pyridyl, pyridazinyl, pyrimidinyl, pyrazinyl, triazinyl, or the like, and benzo derivatives thereof, such as quinolyl, quinazolinyl, isoquinolyl, or the like; or azocinyl, indolizinyl, purinyl, and the like, and benzo derivatives thereof; or cinnolinyl, phthalazinyl, quinazolinyl, quinoxalinyl, naphthyridinyl, pteridinyl, carbazolyl, acridinyl, phenazinyl, phenothiazinyl, phenoxazinyl, or the like. The term "5-6 membered heteroaryl" refers to an aromatic ring system which has 5 or 6 ring atoms, and comprises 1-3, preferably 1-2, heteroatoms independently selected from N, O, and S.

The term "heteroaryloxy" may be understood as "heteroaryl-O-".

The term "halo" or "halogen" refers to fluorine, chlorine, bromine, or iodine.

The term "hydroxy" refers to -OH group.

The term "cyano" refers to -CN group.

The term "sulfydryl" refers to -SH group.

The term "amino" refers to $-NH_2$ group.

The term "therapeutically effective amount" refers to an amount of the compound of the present invention for (i) treating a specific disease, condition or disorder; (ii) alleviating, ameliorating or eliminating one or more symptoms of a specific disease, condition or disorder, or (iii) delaying onset of one or more symptoms of a specific disease, condition or disorder described herein. The amount of the compound of the present invention constituting the "therapeutically effective amount" varies dependently on the compound, the disease state and its severity, the administration regimen, and the age of the mammal to be treated, but can be determined routinely by those skilled in the art in accordance with their knowledge and the present disclosure.

The term "prevent" or "prevention" means administering the compound or formulation described herein to prevent a disease or one or more symptoms associated with the disease, and includes: preventing the occurrence of the disease or disease state in an individual (e.g., a mammal), particularly when such an individual (e.g., a mammal) is predisposed to the disease state but has not yet been diagnosed with it.

The term "pharmaceutically acceptable" is used herein for those compounds, materials, compositions, and/or dosage forms which are, within the scope of sound medical judgment, suitable for use in contact with the tissues of human beings and animals without excessive toxicity, irritation, allergic response, or other problems or complications, and commensurate with a reasonable benefit/risk ratio. The term "pharmaceutically acceptable salt" refers to salts of pharmaceutically acceptable acids or bases, including salts formed from the compound and an inorganic or organic acid, and salts formed from the compound and an inorganic or organic base.

The term "pharmaceutical composition" refers to a mixture consisting of one or more of the compounds or the salts thereof of the present invention and a pharmaceutically acceptable excipient. The pharmaceutical composition is intended to facilitate the administration of the compound of the present invention to an organism.

The term "pharmaceutically acceptable excipients" refers to those which do not have a significant irritating effect on an organism and do not impair the biological activity and properties of the active compound. Suitable excipients are well known to those skilled in the art, such as carbohydrate, wax, water-soluble and/or water-swellable polymers, hydrophilic or hydrophobic materials, gelatin, oil, solvent, water, and the like.

The term "individual" includes mammals and non-mammals. Examples of mammals include, but are not limited to, any member of the class Mammalia: humans, non-human primates (e.g., chimpanzees and other apes and monkeys); livestock animals, such as cattle, horses, sheep, goats, and pigs; domestic animals, such as rabbits, dogs, and cats; laboratory animals, including rodents, such as rats, mice, guinea pigs, and the like. Examples of non-human mammals include, but are not limited to, birds, fish, and the like. In one embodiment associated with the methods and compositions provided herein, the mammal is a human.

The word "comprise" and variations thereof such as "comprises" or "comprising" may be understood in an open, non-exclusive sense, i.e., "including but not limited to".

The phosphate ester compounds (for example, $R^{10}$ is selected from a compound of

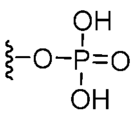 )

described herein are ester prodrugs, which are hydrolyzed by phosphatase *in vivo* to release compounds with biological activity. For example, the phosphate ester compound

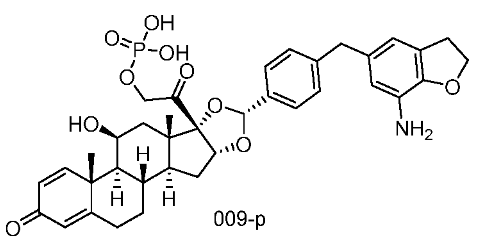

009-p

is hydrolyzed by phosphatase *in vivo* to release a glucocorticoid receptor agonist

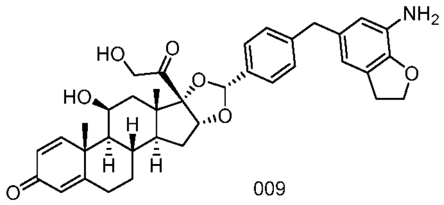

009

. Thus, it will be understood or expected by those skilled in the art that if compound 009 has some biological activity (e.g., glucocorticoid receptor agonistic activity), then the corresponding ester compound 009-p also has the same or similar biological activity *in vivo.*

The present invention also comprises isotopically-labeled compounds which are identical to those documented herein but have one or more atoms replaced by an atom having an atomic mass or mass number different from the atomic mass or mass number usually found in nature. Examples of isotopes that can be incorporated into the compounds of the present invention include isotopes of hydrogen, carbon, nitrogen, oxygen, phosphorus, sulfur, fluorine, iodine and chlorine, such as $^{2}H$, $^{3}H$, $^{11}C$, $^{13}C$, $^{14}C$, $^{13}N$, $^{15}N$, $^{15}O$, $^{17}O$, $^{18}O$, $^{31}P$, $^{32}P$, $^{35}S$, $^{18}F$, $^{123}I$, $^{125}I$ and $^{36}Cl$, respectively, and the like.

Certain isotopically-labeled compounds of the present invention (e.g., those labeled with $^{3}H$ and $^{14}C$) can be used to analyze compounds and/or substrate tissue distribution. Tritiated (i.e., $^{3}H$) and carbon-14 (i.e., $^{14}C$) isotopes are particularly preferred for their ease of preparation and detectability. Positron emitting isotopes, such as $^{15}O$, $^{13}N$, $^{11}C$ and $^{18}F$ can be used in positron emission tomography (PET) studies to determine substrate occupancy. Isotopically-labeled compounds of the present invention can generally be prepared by following procedures analogous to those disclosed in the schemes and/or examples below while substituting a non-isotopically labeled reagent with an isotopically-labeled reagent.

The pharmaceutical composition of the present invention can be prepared by combining the compound of the present invention with a suitable pharmaceutically acceptable excipient, and can be formulated, for example, into a solid, semisolid, liquid, or gaseous formulation such as tablet, pill, capsule, powder, granule, ointment, emulsion, suspension, suppository, injection, inhalant, gel, microsphere, aerosol, and the like.

Typical routes of administration of the compound or the pharmaceutically acceptable salt thereof or the pharmaceutical composition thereof of the present invention include, but are not limited to, oral, rectal, local, inhalation, parenteral, sublingual, intravaginal, intranasal, intraocular, intraperitoneal, intramuscular, subcutaneous and intravenous administration.

The pharmaceutical composition disclosed herein can be manufactured by methods well known in the art, such as by conventional mixing, dissolving, granulating, emulsifying, lyophilizing, and the like.

In some embodiments, the pharmaceutical composition is in an oral form. For oral administration, the pharmaceutical composition can be formulated by mixing the active compounds with pharmaceutically acceptable excipients well known in the art. These excipients enable the compounds of the present invention to be formulated into tablets, pills, pastilles, dragees, capsules, liquids, gels, slurries, suspensions and the like for oral administration to a patient.

A solid oral composition can be prepared by conventional mixing, filling or tableting. For example, it can be obtained by the following method: mixing the active compounds with solid excipients, optionally grinding the resulting mixture, adding additional suitable excipients if desired, and processing the mixture into granules to get the core parts of tablets or dragees. Suitable excipients include, but are not limited to: binders, diluents, disintegrants, lubricants, glidants, or flavoring agents, and the like.

The pharmaceutical compositions may also be suitable for parenteral administration, such as sterile solutions, suspensions or lyophilized products in suitable unit dosage forms.

In all of the administration methods of the compound described herein, the daily dose administered is from 0.001 mg/kg to 200 mg/kg body weight, preferably from 0.05 mg/kg to 50 mg/kg body weight, and more preferably from 0.1 mg/kg to 30 mg/kg body weight, given in individual or separated doses. The following abbreviations are used in the present invention:

| Abbreviation | Chemical name | Abbreviation | Chemical name |
|---|---|---|---|
| BPin | 4,4,5,5-tetramethyl-1,3,2-dioxaborane-2-yl | $B_2Pin_2$ | 4,4,5,5-tetramethyl-2-(4,4,5,5-tetramethyl-1,3,2-dioxaborane-2-yl)-1,3,2-dioxaborane |
| DCM | Dichlormethane | DMF | *N,N*-dimethylformamide |
| $Pd(dppf)Cl_2$ | 1,1'-bis(diphenylphosphino) ferrocene palladium chloride | HCl | Hydrochloric acid |

(continued)

| Abbreviation | Chemical name | Abbreviation | Chemical name |
|---|---|---|---|
| DMAP | 4-Dimethylaminopyridine | TFA | Trifluoroacetic acid |
| THF | Tetrahydrofuran | MeCN | Acetonitrile |
| TEA | Triethylamine | NBS | *N*-Bromosuccinimide |
| M | mol/L | Boc | tert-Butyloxycarbonyl |
| DIEA | *N,N*-Diisopropylethylamine | MeOH | Methanol |
| DMSO | Dimethyl sulfoxide | TLC | Thin layer chromatography |
| KOAc | Potassium acetate | DIPEA | *N,N*-Diisopropylethylamine |
| PE | Petroleum ether | EA | Ethyl acetate |
| ACN | Acetonitrile | $Pd(PPh_3)_4$ | Tetrakis(triphenylphosphine)palladium(0) |

## BRIEF DESCRIPTION OF THE DRAWINGS

FIG. 1 is a COSY diagram of intermediate 1-5 in Example 1 of the present invention.
FIG. 2 is a graph showing the results of the efficacy test of the test compound in Test Example 6 of the present invention in a CHS mouse model.

## DETAILED DESCRIPTION

The present invention is described in detail below by way of examples. However, this is by no means disadvantageously limiting the scope of the present invention. Although the present invention has been described in detail herein and specific examples have also been disclosed, it will be apparent to those skilled in the art that various modifications can be made to the specific examples without departing from the spirit and scope of the present invention. All reagents used in the present invention are commercially available and can be used without further purification.
Unless otherwise stated, the ratio represented by a mixed solvent is a volume mixing ratio.
Unless otherwise stated, % refers to wt%.
The eluent or the mobile phase may be a mixed eluent or mobile phase consisting of two or more solvents, and the ratio of the mixed eluent or mobile phase is the volume ratio of the solvents. For example, "0-10% methanol/dichloromethane" represents that the volume ratio of methanol to dichloromethane in the mixed eluent or mobile phase is 0:100-10:100.
Compounds are named either manually or by ChemDraw® software, and supplier's catalog names are given for commercially available compounds.
The structures of the compounds are determined by nuclear magnetic resonance (NMR) and/or mass spectrometry (MS). NMR shifts are given in $10^{-6}$ (ppm). Solvents for NMR determination are deuterated dimethyl sulfoxide, deuterated chloroform, deuterated methanol or the like, and internal standard is tetramethylsilane (TMS); "$IC_{50}$" refers to the half maximal inhibitory concentration, which is the concentration at which half of the maximal inhibitory effect is achieved; "$EC_{50}$" refers to the half maximal effect concentration, a concentration that causes 50% of the maximal effect.
**Example 1:** Synthesis of (6a*R*,6b*S*,7*S*,8a*S*,8b*S*,10*R*,11a*R*,12a*S*,12b*S*)-10-(4-((7-amino-2,3-dihydrobenzofuran-4-yl)methyl)phenyl)-7-hydroxy-8b-(2-hydroxyacetyl)-6a,8a-dimethyl-6a,6b,7,8,8a,8b,11a,12,12a,12b-decahydro-1*H*-naphtho[2',1':4,5]indeno[1,2-*d*][1,3]dioxolan-4(2*H*)-one (compound **001)**

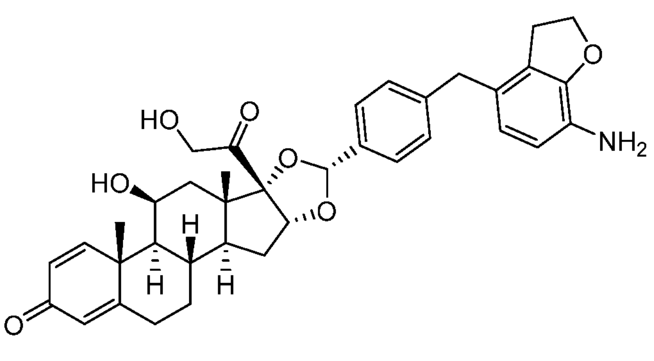

The synthetic route and the specific synthetic steps were as follows.

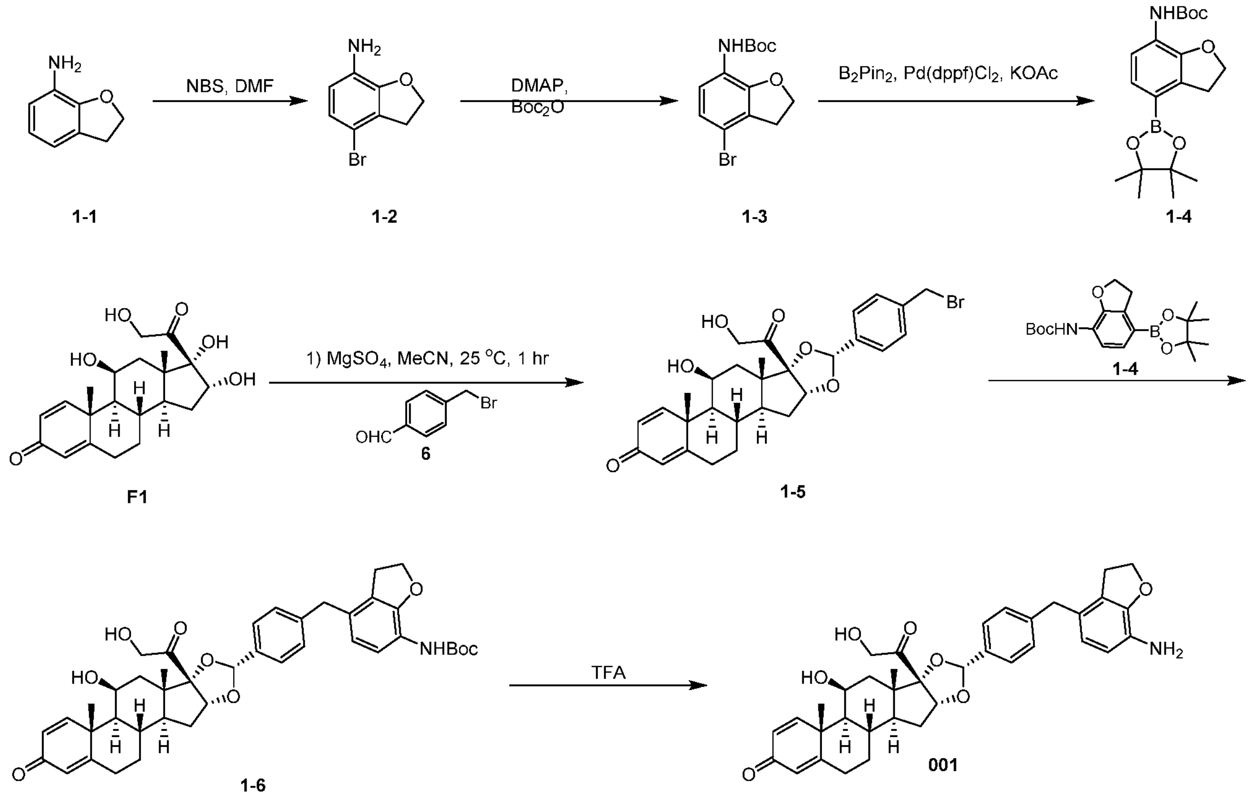


Step 1: synthesis of 4-bromo-2,3-dihydrobenzofuran-7-amine **1-2**

According to the method reported in the literature (WO2016169504 A1), 2,3-dihydrobenzofuran-7-amine (1.0 g, 7.40 mmol) was dissolved in dimethylformamide (20 mL), N-bromosuccinimide (1.48 g, 8.33 mmol) was added, and the mixture was stirred at 25 °C for 3 h. After the reaction was completed as detected by LCMS, water (20 mL) was added to dilute the mixture, the mixture was extracted with ethyl acetate (100 mL × 2), the organic phase was washed with a saturated aqueous sodium chloride solution (30 mL × 2), the organic phase was dried, filtered, and concentrated under reduced pressure, and the crude product was separated and purified by column chromatography (tetrahydrofuran/petroleum ether = **1/4)** to give 4-bromo-2,3-dihydrobenzofuran-7-amine **1-2** (1.0 g). LC-MS: Rt: 0.444 min; MS m/z (ESI): 213.9 $[M+H]^+$;

Step 2: synthesis of *tert*-butyl (4-bromo-2,3-dihydrobenzofuran-7-yl)carbamate

4-Bromo-2,3-dihydrobenzofuran-7-amine **1-2** (1.0 g, 4.67 mmol) was dissolved in dichloromethane (20 mL), di-*tert*-butyl dicarbonate (1.41 g, 6.46 mmol) and 4-dimethylaminopyridine (571 mg, 4.67 mmol) were added, and the mixture was stirred at 25 °C for 16 h. After the reaction was completed as detected by LCMS, water (20 mL) was added to dilute the mixture, the mixture was extracted with ethyl acetate (100 mL × 2), the organic phase was washed with a

saturated aqueous sodium chloride solution (30 mL), the organic phase was dried, filtered, and concentrated under reduced pressure, and the crude product was separated and purified by column chromatography (tetrahydrofuran/petroleum ether = **1/5)** to give *tert*-butyl (4-bromo-2,3-dihydrobenzofuran-7-yl)carbamate **1-3** (600 mg, 38.8%). LC-MS: Rt: 1.756 min; MS m/z (ESI): 258.1 [M-56+H] $^+$;

$^1$H NMR (400 MHz, CHLOROFORM-d) δ = 7.71 - 7.54 (m, 1H), 6.87 (d, *J* = 8.6 Hz, 1H), 6.44 (s, 1H), 4.55 (t, *J*= 8.8 Hz, 2H), 3.15 (t, *J*= 8.8 Hz, 2H), 1.43 (s, 9H).

Step 3: synthesis of *tert*-butyl (4-(4,4,5,5-tetramethyl-1,3,2-dioxaborolan-2-yl)-2,3-dihydrobenzofuran-7-yl)carbamate **1-4**

*tert*-Butyl (4-bromo-2,3-dihydrobenzofuran-7-yl)carbamate **1-3** (450 mg, 1.43 mmol) was dissolved in dioxane (10 mL), bis(pinacolato)diboron (909 mg, 3.58 mmol), [1,1'-bis(diphenylphosphino)ferrocene]palladium dichloride (105 mg, 0.14 mmol), and potassium acetate (422 mg, 4.30 mmol) were added, and the mixture was stirred at 80 °C for 16 h under nitrogen atmosphere. After the reaction was completed as detected by LCMS, the reaction mixture was filtered and concentrated, and the residue was separated and purified by column chromatography (tetrahydrofuran/petroleum ether = 1/6) to give compound **1-4** (410 mg, 79.2%).

LC-MS: Rt: 1.937 min; MS m/z (ESI): 306.2 [M-56+H] $^+$.

Step 4: synthesis of (6a*R*,6b*S*,7*S*,8a*S*,8b*S*,10*R*,11a*R*,12a*S*,12b*S*)-10-(4-(bromomethyl)phenyl)-7-hydroxy-8b-(2-hydroxyacetyl)-6a,8a-dimethyl-6a,6b,7,8,8a,8b,11a,12,12a,12b-decahydro-1*H*-naphtho[2',1':4,5]indeno[1,2-*d*][1,3]dioxolan-4(2*H*)-one **1-5**

The reactant 16α -hydroxyprednisolone **F1** (1 g, 2.66 mmol) and anhydrous magnesium sulfate (1.22 g, 10.1 mmol) were dissolved in acetonitrile (20 mL), and the mixture was stirred at 25 °C for 1 h. 4-(Bromomethyl)benzaldehyde (529 mg, 2.66 mmol) was dissolved in acetonitrile (2 mL) and added to the reaction mixture, the mixture was cooled to 0 °C, trifluoromethanesulfonic acid (1.88 g, 12.5 mmol) was added dropwise, and the mixture was stirred at 25 °C for 2 h. After the reaction was completed as detected by LCMS, the reaction mixture was quenched with an aqueous sodium bicarbonate solution (20 mL), the mixture was extracted with ethyl acetate (100 mL × 2), the organic phase was washed with a saturated aqueous sodium chloride solution (50 mL), the organic phase was dried, filtered, and concentrated under reduced pressure, and the crude product was separated and purified by column chromatography (tetrahydrofuran/petroleum ether = 1/2) to give a crude product which was separated and purified by SFC [column: DAICEL CHIRALCEL OD (250 mm × 50 mm, 10 μm); mobile phase: A: a 0.1% solution of ammonia in methanol; B: $CO_2$, 50%] to give intermediate **1-5** (1.2 g, 80.9%).

LC-MS: Rt: 1.551 min; MS m/z (ESI): 557.1 [M+H] $^+$;

$^1$H NMR (400 MHz, DMSO-d6) δ = 7.45 (s, 3H), 7.31 (d, *J*= 10.1 Hz, 1H), 6.16 (dd, *J* = 1.8, 10.1 Hz, 1H), 5.93 (s, 1H), 5.75 (s, 1H), 5.45 (s, 1H), 5.16 - 5.03 (m, 1H), 4.94 (d, *J* = 4.9 Hz, 1H), 4.79 (d, *J* = 3.0 Hz, 1H), 4.68 (s, 2H), 4.53 ( d, *J* = 19.5 Hz, 1H), 4.30 ( s, 1H), 4.19 ( d, *J* = 19.4 Hz, 1H), 2.61 - 2.53 (m, 1H), 2.31-2.25 (m, 1H), 2.17 - 2.07 (m, 1H), 2.06 - 1.98 (m, 1H), 1.81 - 1.61 (m, 5H), 1.39 (s, 3H), 1.10 - 0.99 (m, 2H), 0.87 (s, 3H).

The absolute configuration of intermediate 1-5 was verified by two-dimensional nuclear magnetic resonance (see FIG. 1).

Step 5: synthesis of tert-butyl (4-(4-((6a*R*,6b*S*,7*S*,8a*S*,8b*S*,10*R*,11a*R*,12a*S*,12b*S*)-7-hydroxy-8b-(2-hydroxyacetyl)-6a,8a-dimethyl-4-oxo-2,4,6a,6b,7,8,8a,8b,11a,12,12a,12b-dodecahydro-1*H*-naphtho[2',1':4,5]indeno[1,2-*d*][1,3]dioxolan-10-yl)benzyl)-2,3-dihydrobenzofuran-7-yl)carbamate **1-6**

Compound **1-5** (300 mg, 0.54 mmol) was dissolved in tetrahydrofuran (6 mL) and water (0.6 mL), *tert*-butyl (4-(4,4,5,5-tetramethyl-1,3,2-dioxaborolan-2-yl)-2,3-dihydrobenzofuran-7-yl)carbamate (194 mg, 0.54 mmol), [1,1'-bis(diphenylphosphino)ferrocene]palladium dichloride (78.8 mg, 0.11 mmol), and potassium carbonate (223 mg, 1.61 mmol) were added, and the mixture was stirred at 80 °C for 16 h under nitrogen atmosphere. After the reaction was completed as detected by LCMS, the reaction mixture was filtered and concentrated, and the residue was separated and purified by column chromatography (tetrahydrofuran/petroleum ether = **1/1)** to give compound **1-6** (300 mg, 54.8%). LC-MS: Rt: 0.584 min; MS m/z (ESI): 712.4 [M+H] $^+$;

Step 6: synthesis of (6a*R*,6b*S*,7*S*,8a*S*,8b*S*,10*R*,11a*R*,12a*S*,12b*S*)-10-(4-((7-amino-2,3-dihydrobenzofuran-4-yl)methyl)phenyl)-7-hydroxy-8b-(2-hydroxyacetyl)-6a,8a-dimethyl-6a,6b,7,8,8a,8b,11a,12,12a,12b-decahydro-1*H*-naphtho[2',1':4,5]indeno[1,2-*d*][1,3]dioxolan-4(2*H*)-one **001**

**1-6** (280 mg, 0.39 mmol) was dissolved in dichloromethane (6 mL), trifluoroacetic acid (2 mL) was added, and the mixture was stirred at 25 °C for 1 h. After the reaction was completed as detected by LCMS, the reaction mixture was concentrated, and the crude product was separated and purified by preparative-HPLC [column: Phenomenex luna 30 × 30 mm × 10 μm; YMC AQ 100 × 30 × 10 μm; mobile phase: A: water (HCl); B: ACN, B%, 30%-60%, 15 min] to give compound **001** (10 mg, 19.0%).

LC-MS: Rt: 1.511 min; MS m/z (ESI): 612.5 [M+H] $^+$;
$^1$H NMR (400 MHz, DMSO-$d_6$) δ = 9.52 - 8.19 (m, 2H), 7.38 (d, *J*= 8.1 Hz, 2H), 7.32 (d, *J*= 10.1 Hz, 1H), 7.20 (d, *J*= 8.0 Hz, 2H), 6.89 (d, *J*= 8.0 Hz, 1H), 6.65 (d, *J*= 8.0 Hz, 1H), 6.17 (dd, *J*= 1.8, 10.1 Hz, 1H), 5.93 (s, 1H), 5.41 (s, 1H), 4.92 (d, *J* = 5.0 Hz, 1H), 4.81 (br s, 1H), 4.60 (t, *J* = 8.8 Hz, 2H), 4.50 (d, *J* = 19.4 Hz, 1H), 4.29 (br s, 1H), 4.18 (d, *J* = 19.4 Hz, 1H), 3.86 (s, 2H), 3.16 - 3.08 (m, 2H), 2.62 - 2.54 (m, 1H), 2.30 (m, 1H), 2.19 - 2.08 (m, 1H), 2.07 - 1.97 (m, 1H), 1.81 - 1.59 (m, 5H), 1.40 (s, 3H), 1.12 - 0.96 (m, 2H), 0.87 (s, 3H).

**Example 2:** synthesis of (6a*R*,6b*S*,7*S*,8a*S*,8b*S*,10*R*,11a*R*,12a*S*,12b*S*)-10-(4-((1*H*-benzo[*d*]imidazol-6-yl)methyl)phenyl)-7-hydroxy-8b-(2-hydroxyacetyl)-6a,8a-dimethyl-6a,6b,7,8,8a,8b,11a,12,12a,12b-decahydro-1*H*-naphtho[2',1':4,5]indeno[1,2-*d*][1,3]dioxolan-4(2*H*)-one (compound **002)**

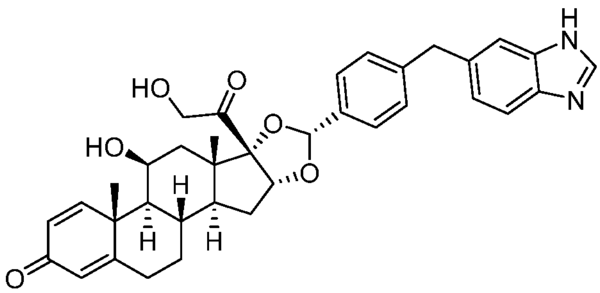

The synthetic route and the specific synthetic steps were as follows.

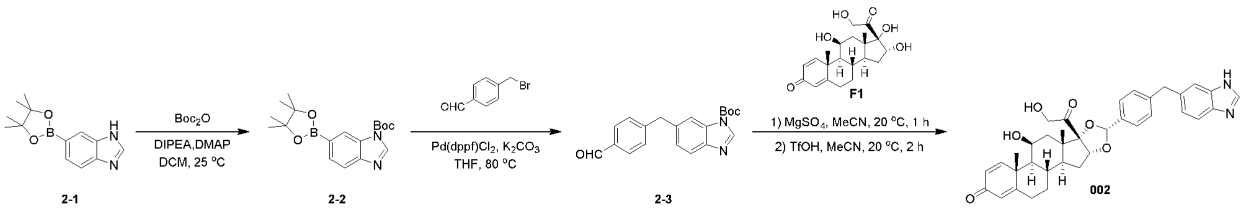


Step 1: synthesis of *tert*-butyl 6-(4,4,5,5-tetramethyl-1,3,2-dioxaborolan-2-yl)-1*H*-benzo[*d*]imidazole-1-formate **2-2**

6-(4,4,5-Trimethyl-1,3,2-dioxaborolan-2-yl)-1*H*-benzo[*d*]imidazole (1.00 g, 4.10 mmol), *N,N*-diisopropylethylamine (1.06 g, 8.19 mmol), 4-dimethylaminopyridine (50 mg, 0.41 mmol), and di-*tert*-butyl dicarbonate (894 mg, 4.10 mmol) were dissolved in anhydrous dichloromethane (20 mL), and the mixture was reacted at 25 °C for 6 h. After the reaction was completed as detected by LCMS, water (20 mL) was added to quench the reaction, the mixture was extracted with dichloromethane (50 mL × 2), the organic phase was washed with a saturated aqueous sodium chloride solution (30 mL), the organic phase was dried, filtered, and concentrated under reduced pressure, and the crude product was separated and purified by column chromatography (ethyl acetate/petroleum ether = 1/5) to give **2-2** (1.0 g, 69.5%).

LC-MS: Rt: 1.793 min; MS m/z (ESI): 345.3 [M+H] $^+$;
$^1$H NMR (400 MHz, DMSO-$d_6$) δ= 8.50 - 8.24 (m, 2H), 8.00 - 7.75 (m, 2H), 1.70 (d, *J* = 3.5 Hz, 9H), 1.36 (d, *J* = 5.1 Hz, 12H).

Step 2: synthesis of *tert*-butyl 6-(4-formylbenzyl)-1*H*-benzo[*d*]imidazole-1-formate **2-3**

**2-2** (500 mg, 1.45 mmol), 4-(bromomethyl)benzaldehyde (376 mg, 1.89 mmol), potassium carbonate (602 mg, 4.36 mmol), and [1,1'-bis(diphenylphosphino)ferrocene]palladium dichloride (106 mg, 0.14 mmol) were dissolved in anhydrous tetrahydrofuran (10 mL), and the mixture was reacted at 80 °C for 16 h under nitrogen atmosphere. After the reaction was completed as detected by LCMS, the reaction mixture was filtered and concentrated, and the residue was separated and purified by column chromatography (ethyl acetate/petroleum ether = 1/5) to give **2-3** (380 mg, crude). LC-MS: Rt: 1.563 min and 1.593; MS m/z (ESI): 337.3 $[M+H]^+$;

Step 3: synthesis of (6a*R*,6b*S*,7*S*,8a*S*,8b*S*,10*R*,11a*R*,12a*S*,12b*S*)-10-(4-((1*H*-benzo[*d*]imidazol-6-yl)methyl)phenyl)-7-hydroxy-8b-(2-hydroxyacetyl)-6a,8a-dimethyl-6a,6b,7,8,8a,8b,11a,12,12a,12b-decahydro-1*H*-naphtho[2',1':4,5]indeno[1,2-*d*][1,3]dioxolan-4(2*H*)-one

16α-Hydroxyprednisolone **F1** (230 mg, 0.61 mmol) was dissolved in anhydrous acetonitrile (1.5 mL), anhydrous magnesium sulfate (279 mg, 2.32 mmol) was added, and the mixture was stirred at 25 °C for 1 h. **2-3** (308 mg, 0.92 mmol) was dissolved in anhydrous acetonitrile (1.5 mL) and added to the reaction system, the mixture was cooled to 0 °C, trifluoromethanesulfonic acid (431 mg, 2.87 mmol) was added dropwise, and the mixture was stirred at 25 °C for 2 h. After the reaction was completed as detected by LCMS, a saturated sodium bicarbonate solution (5 mL) was added to quench the reaction, the mixture was extracted with ethyl acetate (30 mL × 2), the organic phase was washed with a saturated aqueous sodium chloride solution (20 mL), the organic phase was dried, filtered, and concentrated under reduced pressure, and the crude product was purified by Prep-HPLC [column: Phenomenex Luna 30 × 30 mm × 10 μm; YMC AQ 100 × 30 × 10 μm; mobile phase: A: water (containing 0.225% formic acid); B: MeCN, B%, 15%-45%, 20 min] to give compound **002** (35.8 mg, 9.75%).

LC-MS: Rt: 1.390 min; MS m/z (ESI): 595.5 [M+H]; Chiral-HPLC: Rt: 2.211 min, chiral purity = 97.56%.

$^1$H NMR (400MHz, DMSO-$d_6$) $\delta$ = 8.39 (br s, 1H), 7.52 (d, *J*= 8.3 Hz, 1H), 7.44 (s, 1H), 7.40 - 7.35 (m, 2H), 7.33 - 7.26 (m, 3H), 7.12 (d, *J* = 8.4 Hz, 1H), 6.16 (dd, *J* = 1.6, 10.1 Hz, 1H), 5.93 (s, 1H), 5.40 (s, 1H), 5.08 (br s, 1H), 4.92 (d, *J*= 4.9 Hz, 1H), 4.78 (d, *J* = 3.0 Hz, 1H), 4.50 (br d, *J* = 16.0 Hz, 1H), 4.28 (s, 1H), 4.17 (d, *J* = 19.5 Hz, 1H), 4.05 (s, 2H), 2.30 (s, 1H), 2.15 - 1.98 (m, 2H), 1.81 - 1.60 (m, 5H), 1.39 (s, 3H), 1.10 - 0.97 (m, 2H), 0.86 (s, 3H).

**Example 3:** synthesis of (6a*R*,6b*S*,7*S*,8a*S*,8b*S*,10*R*,11a*R*,12a*S*,12b*S*)-10-(4-((1*H*-indol-6-yl)methyl)phenyl)-7-hydroxy-8b-(2-hydroxyacetyl)-6a,8a-dimethyl-6a,6b,7,8,8a,8b,11a,12,12a,12b-decahydro-1*H*-naphtho[2',1':4,5]indeno[1,2-*d*][1,3]dioxolan-4(2*H*)-one (compound **003)**

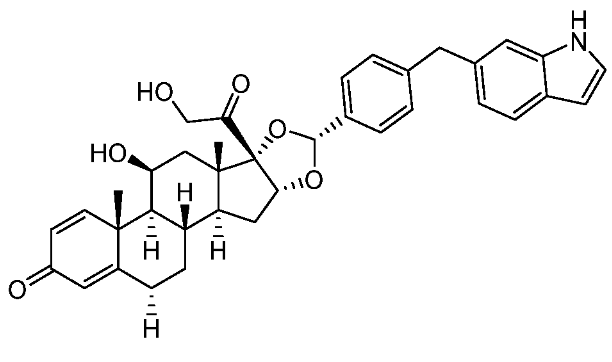

The synthetic route and the specific synthetic steps were as follows.

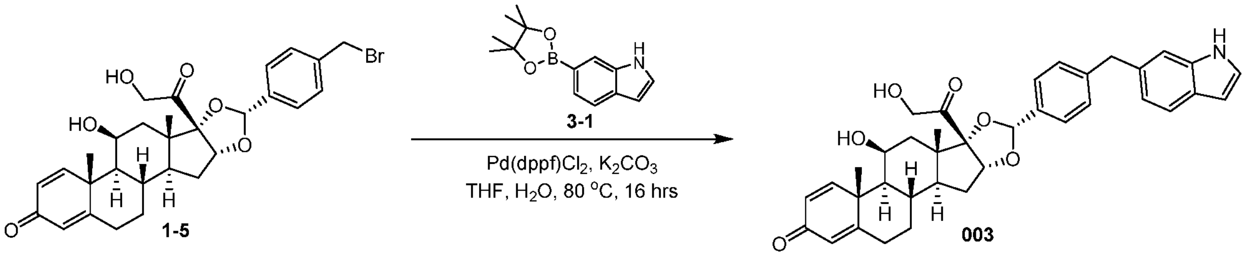

Compound **1-5** (500 mg, 0.89 mmol) was dissolved in tetrahydrofuran (5 mL) and water (0.5 mL), 6-(4,4,5,5-tetramethyl-1,3,2-dioxaborolan-2-yl)-1*H*-indole **3-1** (262 mg, 1.08 mmol), [1,1'-bis(diphenylphosphino)ferrocene]palladium dichloride (66 mg, 0.09 mmol), and potassium carbonate (372 mg, 2.69 mmol) were added, and the mixture was stirred at 80 °C for 16 h under nitrogen atmosphere. After the reaction was completed as detected by LCMS, the mixture was diluted with ethyl acetate (100 mL), the organic phase was washed with a saturated aqueous sodium chloride solution (30 mL), the organic phase was dried, filtered, and concentrated under reduced pressure, and the crude product was separated and purified by column chromatography (tetrahydrofuran/petroleum ether = 1/1) to give a crude product which was separated and purified by Prep-HPLC [column: Phenomenex luna 30 × 30 mm × 10 μm + YMC AQ 100 × 30 × 10 μm; mobile phase: A: water (0.225% FA); B: MeCN, B%, 50%-80%, 20 min] to give compound **003** (60 mg 11.2%).

LC-MS: Rt: 2.098 min; MS m/z (ESI): 594.3 [M+H] $^+$; Chiral-HPLC: Rt: 1.204 min, chiral purity = 100%.

$^1$H NMR (400 MHz, DMSO-$d_6$) δ = 10.92 (s, 1H), 7.41 (d, *J* = 8.1 Hz, 1H), 7.37 (*d, J* = 8.0 Hz, 2H), 7.30 (d, *J*= 10.1 Hz, 1H), 7.28 - 7.23 (m, 3H), 7.18 (s, 1H), 6.85 (dd, *J*= 1.3, 8.1 Hz, 1H), 6.34 ( s, 1H), 6.16 (dd, *J*= 1.8, 10.1 Hz, 1H), 5.93 (s, 1H), 5.40 (s, 1H), 5.08 (t, *J* = 5.9 Hz, 1H), 4.92 (d, *J*= 5.0 Hz, 1H), 4.78 (d, *J* = 3.1 Hz, 1H), 4.50 (dd, *J*= 6.3, 19.4 Hz, 1H), 4.29 (s, 1H), 4.17 (dd, *J*= 5.4, 19.4 Hz, 1H), 4.00 (s, 2H), 2.59 - 2.53 (m, 1H), 2.36 - 2.26 (m, 1H), 2.18 - 2.06 (m, 1H), 2.05 - 1.96 (m, 1H), 1.80 - 1.54 (m, 5H), 1.39 (s, 3H), 1.11 - 0.97 (m, 2H), 0.86 (s, 3H).

**Example 4:** synthesis of (6a*R*,6b*S*,7*S*,8a*S*,8b*S*,10*R*,11a*R*,12a*S*,12b*S*)-7-hydroxy-8b-(2-hydroxyacetyl)-10-(4-(indolin-6-ylmethyl)phenyl)-6a,8a-dimethyl-6a,6b,7,8,8a,8b,11a,12,12a,12b-decahydro-1*H*-naphtho[2',1':4,5]indeno[1,2-*d*][1,3]dioxolan-4(2*H*)-one (compound **004)**

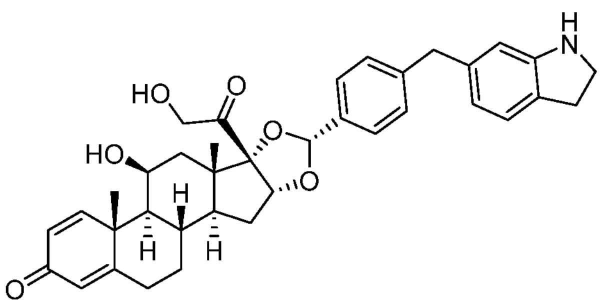

The synthetic route and the specific synthetic steps were as follows.

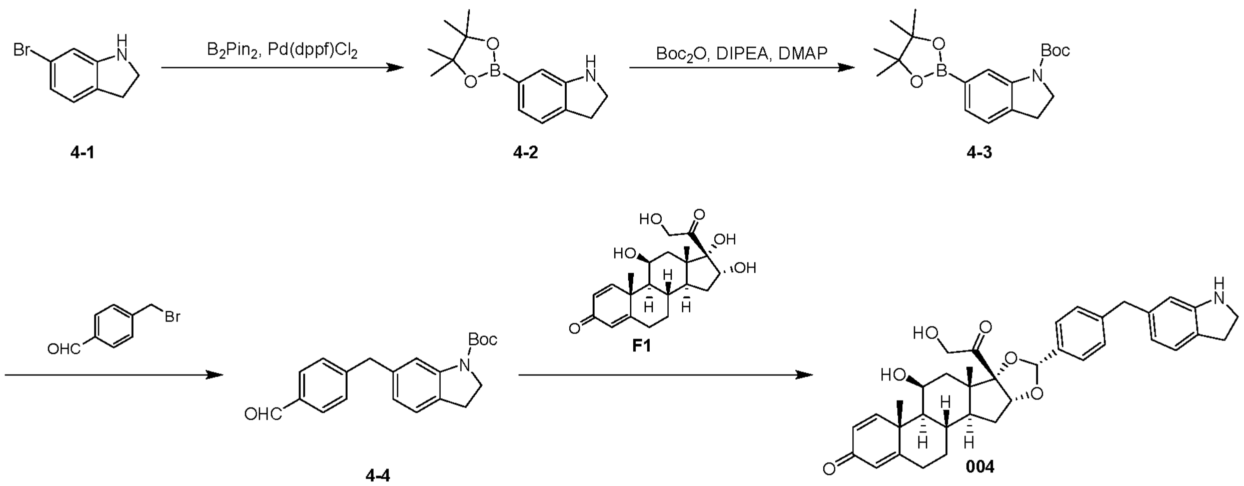


4-1 4-2 4-3 F1 4-4 004

Step 1: synthesis of 6-(4,4,5,5-tetramethyl-1,3,2-dioxaborolan-2-yl)indoline **4-2**

6-Bromoindoline (1.0 g, 5.05 mmol) was dissolved in dioxane (20 mL), bis(pinacolato)diboron (2.56 g, 10.1 mmol), [1,1'-bis(diphenylphosphino)ferrocene]palladium dichloride (369 mg, 504 μmol), and potassium acetate (1.49 g, 15.2 mmol) were added, and the mixture was reacted at 80 °C for 12 h under nitrogen atmosphere. After the reaction was completed as detected by LCMS, the reaction mixture was filtered and concentrated, and the residue was separated

and purified by column chromatography (tetrahydrofuran/petroleum ether = 1/3) to give 6-(4,4,5,5-tetramethyl-1,3,2-dioxaborolan-2-yl)indoline **4-2** (1.2 g, 96.9%).

LC-MS: Rt: 1.509 min; MS m/z (ESI): 246.3 [M+H] $^+$;

Step 2: synthesis of *tert*-butyl 6-(4,4,5,5-tetramethyl-1,3,2-dioxaborolan-2-yl)indoline-1-formate **4-3 4-2** (500 mg, 2.04 mmol) was dissolved in dichloromethane (8 mL), di-*tert*-butyl dicarbonate (891 mg, 4.08 mmol), diisopropylethylamine (528 mg, 4.08 mmol), and 4-dimethylaminopyridine (49.8 mg, 408 μmol) were added, and the mixture was stirred at 25 °C for 6 h. After the reaction was completed as detected by LCMS, the mixture was diluted with dichloromethane (50 mL), the organic phase was washed with a saturated aqueous sodium chloride solution (15 mL), the organic phase was dried, filtered, and concentrated under reduced pressure, and the residue was separated and purified by column chromatography (tetrahydrofuran/petroleum ether = 1/5) to give **4-3** (260 mg, 36.9%).

LC-MS: Rt: 2.340 min; MS m/z (ESI): 290.3 [M-56+H];

Step 3: synthesis of *tert*-butyl 6-(4-formylbenzyl)indoline-1-formate **4-4**

*tert*-Butyl 6-(4,4,5,5-tetramethyl-1,3,2-dioxaborolan-2-yl)indoline-1-formate (260 mg, 753 μmol) was dissolved in tetrahydrofuran (5 mL), 4-(bromomethyl)benzaldehyde (299 mg, 1.51 mmol), [1,1'-bis(diphenylphosphino)ferrocene]palladium dichloride (165 mg, 226 μmol), and potassium carbonate (520 mg, 3.77 mmol) were added, and the mixture was stirred at 80 °C for 12 h under nitrogen atmosphere. After the reaction was completed as detected by LCMS, the reaction mixture was filtered and concentrated, and the residue was separated and purified by column chromatography (tetrahydrofuran/petroleum ether = 1/5) to give **4-4** (190 mg, 74.8%).

LC-MS: Rt: 1.810 min; MS m/z (ESI): 282.2 [M-56+H] $^+$;

Step 4: synthesis of (6a*R*,6b*S*,7*S*,8a*S*,8b*S*,10*R*,11a*R*,12a*S*,12b*S*)-7-hydroxy-8b-(2-hydroxyacetyl)-10-(4-(indolin-6-ylmethyl)phenyl)-6a,8a-dimethyl-6a,6b,7,8,8a,8b,11a,12,12a,12b-decahydro-1*H*-naphtho[2',1'4,5]indeno[1,2-*d*][1,3]dioxolan-4(2*H*)-one **004**

16α-Hydroxyprednisolone **F1** (165 mg, 438 μmol) was dissolved in acetonitrile (3 mL), anhydrous magnesium sulfate (258 mg, 2.15 mmol) was added, and the mixture was stirred at 25 °C for 1 h. *tert*-Butyl 6-(4-formylbenzyl)indoline-1-formate (178 mg, 526 μmol) was dissolved in acetonitrile (1 mL) and added to the reaction mixture, the mixture was cooled to 0 °C, trifluoromethanesulfonic acid (322 mg, 2.15 mmol) was added dropwise, and the mixture was stirred at 25 °C for 2 h. After the reaction was completed as detected by LCMS, a saturated aqueous sodium bicarbonate solution (10 mL) was added to quench the reaction, the mixture was extracted with ethyl acetate (30 mL × 3), the organic phase was washed with a saturated aqueous sodium chloride solution (20 mL), the organic phase was dried, filtered, and concentrated under reduced pressure, and the crude product was separated and purified by Prep-HPLC [column: Phenomenex luna 30 × 30 mm × 10 μm + YMC AQ 100 × 30 × 10 μm; mobile phase: A: water (0.225% FA); B: MeCN, B%, 18%-48%, 20 min] to give compound **004** (42.9 mg, 6.45%).

LC-MS: Rt: 1.532 min; MS m/z (ESI): 596.3 [M+H] $^+$; Chiral-HPLC: Rt: 2.502 min, chiral purity = 100%.

$^1$H NMR (400 MHz, DMSO-$d_6$) δ = 7.37 - 7.33 (m, 2H), 7.30 (d, *J* = 10.1 Hz, 1H), 7.20 (d, *J*= 8.0 Hz, 2H), 6.88 (d, *J* = 7.3 Hz, 1H), 6.37 (d, *J* = 7.4 Hz, 1H), 6.28 (s, 1H), 6.18 - 6.13 (m, 1H), 5.95 - 5.91 (m, 1H), 5.39 (s, 2H), 5.12 - 5.06 (m, 1H), 4.91 (d, *J* = 5.0 Hz, 1H), 4.80 - 4.75 (m, 1H), 4.54 - 4.45 (m, 1H), 4.32 - 4.25 (m, 1H), 4.21 - 4.12 (m, 1H), 3.75 (s, 2H), 3.38 - 3.34 (m, 2H), 2.84 - 2.77 (m, 2H), 2.55-2.47 (m 1H), 2.34 - 2.27 (m, 1H), 2.16 - 2.07 (m, 1H), 2.06 - 1.97 (m, 1H), 1.79 - 1.59 (m, 5H), 1.39 (s, 3H), 1.12 - 0.97 (m, 2H), 0.89 - 0.82 (m, 3H).

**Example 5:** synthesis of (6a*R*,6b*S*,7*S*,8a*S*,8b*S*,10*R*,11a*R*,12a*S*,12b*S*)-10-(4-((3,4-dihydro-2*H*-benzo[*b*][1,4]oxazin-7-yl)methyl)phenyl)-7-hydroxy-8b-(2-hydroxyacetyl)-6a,8a-dimethyl-1,2,6a,6b,7,8,8a,8b,11a,12,12a,12b-decahydro-1*H*-naphtho[2',1':4,5]indeno[1,2-*d*][1,3]dioxolan-4(2*H*)-one (compound **005)**

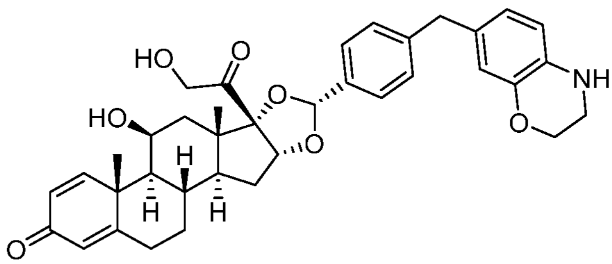

The synthetic route and the specific synthetic steps were as follows.

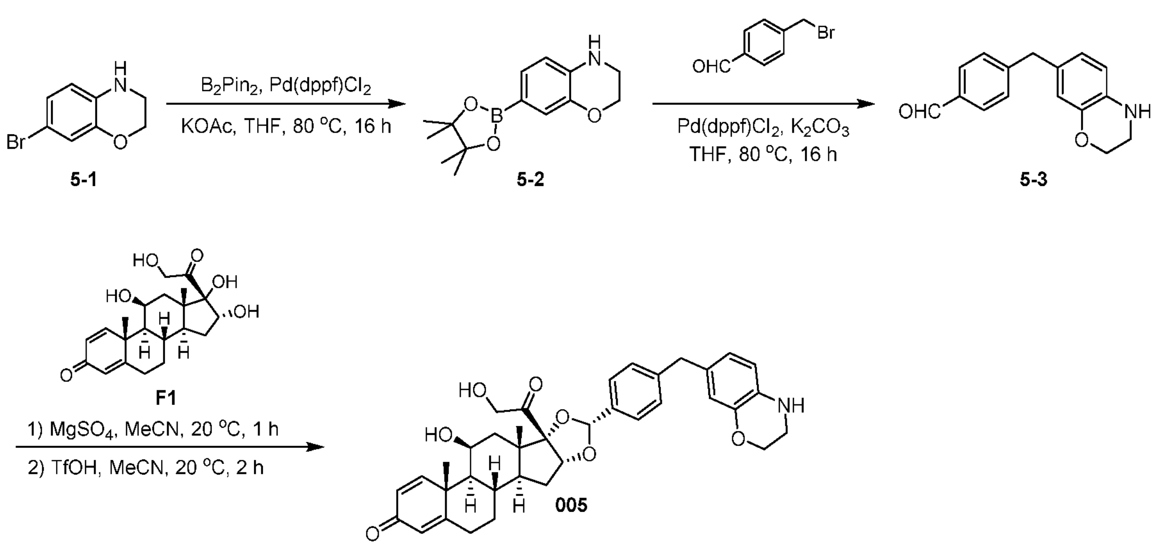


5-1 5-2 5-3 F1 005

Step 1: synthesis of 7-(4,4,5,5-tetramethyl-1,3,2-dioxaborolan-2-yl)-3,4-dihydro-2*H*-benzo[*b*][1,4]oxazine **5-2**

7-Bromo-3,4-dihydro-2*H*-benzo[b][1,4]oxazine (1.00 g, 4.67 mmol) **5-1** was dissolved in tetrahydrofuran (20 mL), bis(pinacolato)diboron (1.30 g, 5.14 mmol), potassium acetate (1.38 g, 14.0 mmol), and [1,1'-bis(diphenylphosphino)ferrocene]palladium dichloride (341 mg, 467 $\mu$mol) were added, and the mixture was reacted at 80 °C for 16 h under nitrogen atmosphere. After the reaction was completed as detected by LCMS, the reaction mixture was filtered and concentrated, and the residue was purified by column chromatography (ethyl acetate/petroleum ether = 1/5) to give 7-(4,4,5,5-tetramethyl-1,3,2-dioxaborolan-2-yl)-3,4-dihydro-2*H*-benzo[*b*][1,4]oxazine **5-2** (950 mg, 77.9%).
LC-MS: Rt: 1.454min; MS m/z (ESI):262.3 [M+H] $^+$;

Step 2: synthesis of 4-((3,4-dihydro-2*H*-benzo[*b*][1,4]oxazin-7-yl)methyl)benzaldehyde **5-3**

**5-2** (900 mg, 3.45 mmol) was dissolved in tetrahydrofuran (20 mL), 4-(bromomethyl)benzaldehyde (1.03 g, 5.17 mmol), potassium carbonate (1.43 g, 10.3 mmol), and [1,1'-bis(diphenylphosphino)ferrocene]palladium dichloride (252 mg, 345 $\mu$mol) were added, and the mixture was reacted at 80 °C for 16 h under nitrogen atmosphere. After the reaction was completed as detected by LCMS, the reaction mixture was filtered and concentrated, and the residue was purified by column chromatography (ethyl acetate/petroleum ether = 1/3) to give 4-((3,4-dihydro-2*H*-benzo[*b*][1,4]oxazin-7-yl)methyl)benzaldehyde **5-3** (397 mg).
LC-MS: Rt: 1.202 min; MS m/z (ESI):254.1 [M+H] $^+$;

Step 3: synthesis of (6a*R*,6b*S*,7*S*,8a*S*,8b*S*,10*R*,11a*R*,12a*S*,12b*S*)-10-(4-((3,4-dihydro-2*H*-benzo[*b*][1,4]oxazin-7-yl)methyl)phenyl)-7-hydroxy-8b-(2-hydroxyacetyl)-6a,8a-dimethyl-1,2,6a,6b,7,8,8a,8b,11a,12,12a,12b-decahydro-1*H*-naphtho[2',1':4,5]indeno[1,2-*d*][1,3]dioxolan-4(2*H*)-one (compound **005)**

16$\alpha$-Hydroxyprednisolone **F1** (350 mg, 929 $\mu$mol) was dissolved in acetonitrile (15 mL), magnesium sulfate (425 mg, 3.53 mmol) was added, and the mixture was stirred at 25 °C for 1 h. 4-((3,4-Dihydro-2*H*-benzo[*b*][1,4]oxazin-7-yl)methyl)benzaldehyde (235 mg, 929 $\mu$mol) **5-3** was dissolved in acetonitrile (5 mL) and added to the reaction system, the mixture was cooled to 0 °C, trifluoromethanesulfonic acid (655 mg, 4.37 mmol) was added dropwise, and the mixture was stirred at 25 °C for 2 h. After the reaction was completed as detected by LCMS, a saturated aqueous sodium bicarbonate solution (15 mL) was added to quench the reaction, the mixture was extracted with ethyl acetate (50 mL $\times$ 3), the organic phase was washed with a saturated aqueous sodium chloride solution (20 mL), the organic phase was dried, filtered, and concentrated under reduced pressure, and the crude product was separated and purified by prep-HPLC [column: Xtimate C18 150 $\times$ 40 mm $\times$ 10 $\mu$m; mobile phase: A: water (10 mM $NH_4HCO_3$); B: MeCN, B%, 40%-70%, 10 min] to give compound **005** (20 mg, 21.1%).

LC-MS: Rt: 1.874 min; MS m/z (ESI):612.3 [M+H]$^+$;
$^1$H NMR (400 MHz, DMSO-$d_6$) $\delta$ = 7.41 - 7.27 (m, 3H), 7.19 (br d, *J*= 7.9 Hz, 2H), 6.56 - 6.38 (m, 3H), 6.16 (dd, *J* = 1.1, 10.0 Hz, 1H), 5.93 (s, 1H), 5.52 (br s, 1H), 5.39 (s, 1H), 5.05 (t, *J*= 5.9 Hz, 1H), 4.92 (br d, *J* = 5.0 Hz, 1H),

4.76 (br d, *J* = 2.6 Hz, 1H), 4.49 (br dd, *J* = 6.3, 19.4 Hz, 1H), 4.29 (br s, 1H), 4.17 (br dd, *J* = 5.6, 19.4 Hz, 1H), 4.10 - 4.01 (m, 2H), 3.70 (s, 2H), 3.29-3.25 (m, 1H), 3.21 (br s, 2H), 2.37 - 2.25 (m, 1H), 2.18 - 1.96 (m, 2H), 1.84 - 1.56 (m, 5H), 1.39 (s, 3H), 1.14 - 0.97 (m, 2H), 0.86 (s, 3H).

**Example 6:** synthesis of (2*S*,6a*S*,6b*R*,7*S*,8a*S*,8b*S*,10*R*,11a*R*,12a*S*,12b*S*)-10-(4-((3,4-dihydro-2*H*-benzo[*b*][1,4]oxazin-7-yl)methyl)phenyl)-2,6b-difluoro-7-hydroxy-8b-(2-hydroxyacetyl)-6a,8a-dimethyl-6a,6b,7,8,8a,8b,11a,12,12a,12b-decahydro-1*H*-naphtho[2',1':4,5]indeno[1,2-*d*][1,3]dioxolan-4(2*H*)-one (compound **006)**

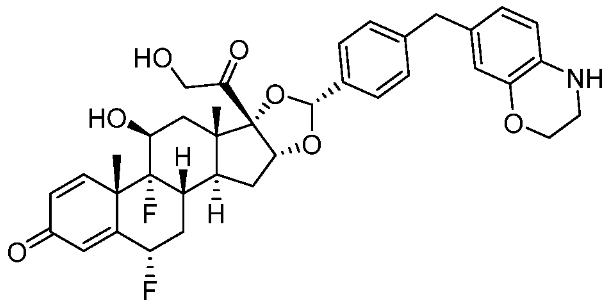

The synthetic route and the specific synthetic steps were as follows.

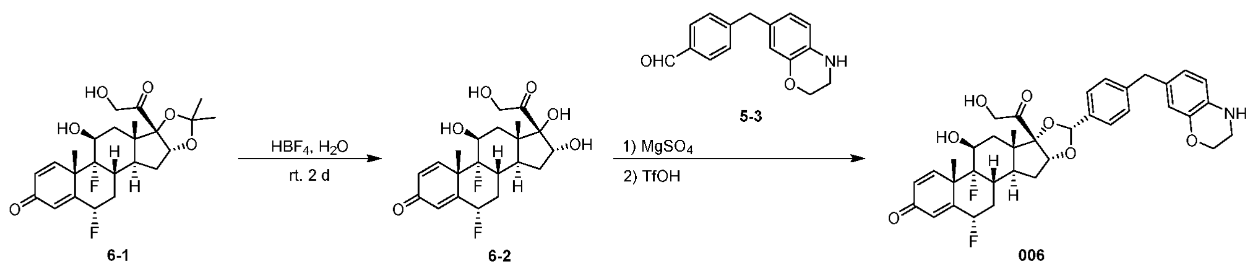


**6-1** **6-2** **006**

Step 1: synthesis of (6S,8S,9R,10*S*,11*S*,13*S*,14*S*,16*R*,17*S*)-6,9-difluoro-11,16,17-trihydroxy-17-(2-hydroxyacetyl)-10,13-dimethyl-6,7,8,9,10,11,12,13,14,15,16,17-dodecahydro-3*H*-cyclopenta[a]phenanthren-3-one **6-2**

The compound (2*S*,6a*S*,6b*R*,7*S*,8a*S*,8b*S*,11a*R*,12a*S*,12b*S*)-2,6b-difluoro-7-hydroxy-8b-(2-hydroxyacetyl)-6a,8a, 10, 10-tetramethyl-6a,6b, 7,8,8a,8b, 11a, 12, 12a, 12b-decahydro-1*H*-naphtho[2',1':4,5]indeno[1,2-*d*][1,3]dioxolan-4(2*H*)-one **6-1** (1.0 g, 2.21 mmol) was dissolved in a 30% aqueous fluoroboric acid solution (20 mL), and the mixture was stirred at room temperature for 48 h. After the reaction was completed as detected by LCMS, 40 mL of water was added, the mixture was stirred for 5 min and filtered, and the residue was washed with water (10 mL × 3) and ethanol (10 mL × 3) sequentially to give a crude product of (6*S*,8*S*,9*R*,10*S*,11*S*,13*S*,14*S*,16*R*,17*S*)-6,9-difluoro-11,16,17-trihydroxy-17-(2-hydroxyacetyl)-10,13-dimethyl-6,7,8,9,10,11,12,13,14,15, 16, 17-dodecahydro-3*H*-cyclopenta[a]phenanthren-3-one **6-2** (0.7 g). LC-MS: Rt: 0.426 min; MS m/z (ESI): 413.1 $[M+H]^+$;

Step 2: synthesis of (2*S*,6a*S*,6b*R*,7*S*,8a*S*,8b*S*,10*R*,11a*R*,12a*S*,12b*S*)-10-(4-((3,4-dihydro-2*H*-benzo[*b*][1,4]oxazin-7-yl)methyl)phenyl)-2,6b-difluoro-7-hydroxy-8b-(2-hydroxyacetyl)-6a,8a-dimethyl-6a,6b,7,8,8a,8b,11a,12,12a,12b-decahydro-1*H*-naphtho[2',1:4,5]indeno[1,2-*d*][1,3]dioxolan-4(2*H*)-one **006**

**6-2** (150 mg, 0.36 mmol) was dissolved in anhydrous acetonitrile (1.5 mL), magnesium sulfate (166 mg, 1.38 mmol) was added, and the mixture was stirred at 25 °C for 1 h. 4-((3,4-Dihydro-2*H*-benzo[*b*][1,4]oxazin-7-yl)methyl)benzaldehyde (138 mg, 0.55 mmol) was dissolved in anhydrous acetonitrile (1.5 mL) and added to the reaction system, the mixture was cooled to 0 °C, trifluoromethanesulfonic acid (257 mg, 1.71 mmol) was added dropwise, and the mixture was reacted at 25 °C for 2 h. After the reaction was completed as detected by LCMS, a saturated aqueous sodium bicarbonate solution (10 mL) was added to quench the reaction, the mixture was extracted with ethyl acetate (50 mL × 2), the organic phase was washed with a saturated aqueous sodium chloride solution (20 mL), the organic phase was dried, filtered, and concentrated under reduced pressure, and the crude product was separated and purified by Prep-HPLC [column: Phenomenex Luna C18 100 × 30 mm × 3 μm; mobile phase: A: water (0.225% FA); B: MeCN, B%:

40%-70%, 8 min] to give compound **006** (82.7 mg, 34.6%).

LC-MS: Rt: 1.553min; MS m/z (ESI): 648.2 $[M+H]^+$; Chiral-HPLC: Rt: 3.241 min, chiral purity = 100%.

$^1H$ NMR (400 MHz, DMSO-$d_6$) $\delta$ = 7.32 (d, *J* = 8.1 Hz, 2H), 7.26 (d, *J* = 10.1 Hz, 1H), 7.20 (d, *J* = 8.1 Hz, 2H), 6.53 - 6.48 (m, 1H), 6.47 - 6.43 (m, 2H), 6.33 - 6.27 (m, 1H), 6.13 (s, 1H), 5.72 - 5.57 (m, 1H), 5.50 (d, *J* = 3.0 Hz, 1H), 5.43 (s, 1H), 4.94 (d, *J* = 4.9 Hz, 1H), 4.51 (d, *J* = 19.5 Hz, 1H), 4.19-4.04 (m, 2H), 4.07 - 4.03 (m, 2H), 3.69 (s, 2H), 3.22 - 3.19 (m, 2H), 2.65 - 2.56 (m, 1H), 2.28-2.07 (m, 2H), 2.05-1.98 (m, 1H), 1.75 - 1.65 (m, 3H), 1.51-1.49 (m, 1H), 1.50 (s, 3H), 0.86 (s, 3H).

**Example 7:** synthesis of (6a*R*,6b*S*,7*S*,8a*S*,8b*S*,10*R*,11a*R*,12a*S*,12b*S*)-10-(4-((4-aminobenzo[*d*]oxazol-7-yl)methyl)phenyl)-7-hydroxy-8*b*-(2-hydroxyacetyl)-6*a*,8*a*-dimethyl-6*a*,6*b*,7,8,8*a*,8*b*,11*a*,12,12*a*,12*b*-decahydro-1*H*-naphtho[2',1':4,5]indeno[1,2-*d*][1,3]dioxolan-4(2*H*)-one (compound 007)

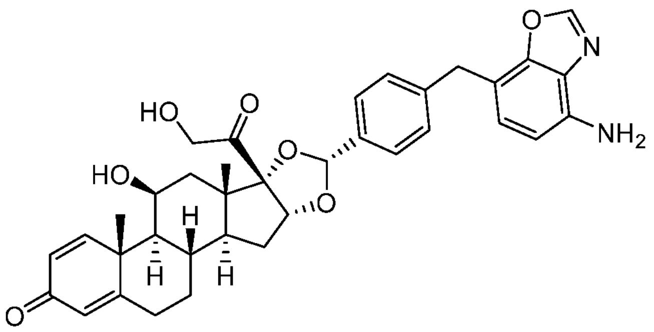

The synthetic route and the specific synthetic steps were as follows.

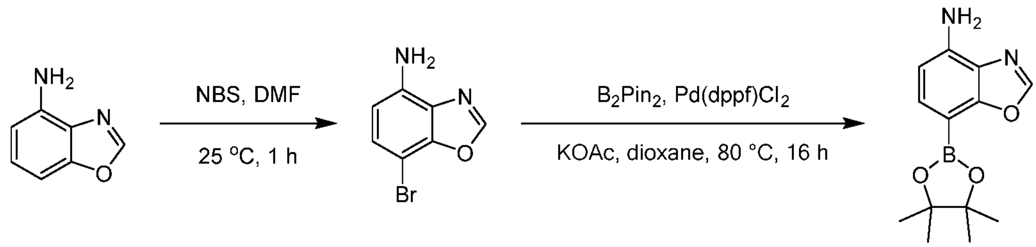


7-1 7-2 7-3

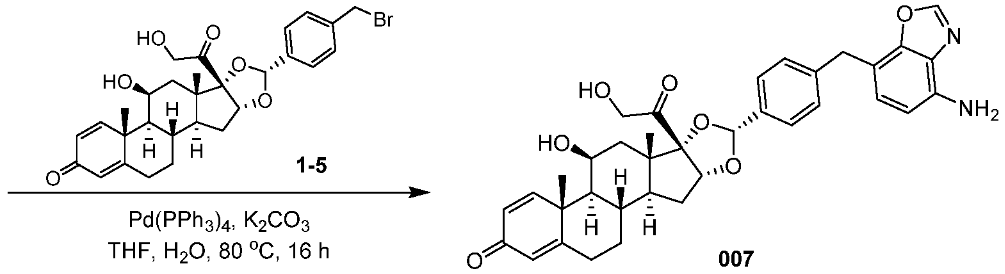


1-5

007

Step 1: synthesis of 7-bromobenzo[*d*]oxazole-4-amine **7-2**

According to the method reported in the literature (CN110903258), benzo[d]oxazol-4-amine (1.0 g, 7.5 mmol) was dissolved in dimethylformamide (20 mL), NBS (1.3 g, 7.1 mmol) was added, and the mixture was stirred at 25 °C for 1 h. After the reaction was completed as detected by LCMS, water (20 mL) was added to dilute the reaction mixture, the mixture was extracted with ethyl acetate (100 mL × 2), the organic phase was washed with a saturated aqueous sodium chloride solution (30 mL × 2), the organic phase was dried, filtered, and concentrated under reduced pressure, and the crude product was separated and purified by column chromatography (tetrahydrofuran/petroleum ether = **1/2)** to give 7-bromobenzo[*d*]oxazol-4-amine **7-2** (1.3 g, 64.5%).

LC-MS: Rt: 1.181 min; MS m/z (ESI): 214.9 [M+H]$^+$;
$^1$H NMR (400 MHz, DMSO-$d_6$) δ = 8.59 (s, 1H), 7.24 (d, *J* = 8.5 Hz, 1H), 6.52 (d, *J* = 8.6 Hz, 1H), 5.86 (s, 2H).

Step 2: synthesis of 7-(4,4,5,5-tetramethyl-1,3,2-dioxaborolan-2-yl)benzo[*d*]oxazol-4-amine **7-3**

7-Bromobenzo[*d*]oxazol-4-amine **7-2** (1.3 g, 6.1 mmol) and bis(pinacolato)diboron (3.1 g, 12.2 mmol) were dissolved in anhydrous dioxane (26 mL), potassium acetate (1.8 g, 18.3 mmol) and [1,1'-bis(diphenylphosphino)ferrocene]palladium dichloride (447 mg, 0.6 mmol) were added, and the mixture was stirred at 80 °C for 16 h under nitrogen atmosphere. After the reaction was completed as detected by LCMS, the reaction mixture was filtered and concentrated, and the residue was purified by column chromatography (tetrahydrofuran/petroleum ether = **1/2)** to give 7-(4,4,5,5-tetramethyl-1,3,2-dioxaborolan-2-yl)benzo[*d*]oxazol-4-amine **7-3** (1.4 g, 64.3%).
LC-MS: Rt: 1.368 min; MS m/z (ESI): 261.2 [M+H];

Step 3: synthesis of (6a*R*,6b*S*,7*S*,8a*S*,8b*S*,10*R*,11a*R*,12a*S*,12b*S*)-10-(4-((4-aminobenzo[*d*]oxazol-7-yl)methyl)phenyl)-7-hydroxy-8*b*-(2-hydroxyacetyl)-6*a*,8*a*-dimethyl-6*a*,6*b*,7,8,8*a*,8*b*,11*a*,12,12*a*,12*b*-decahydro-1*H*-naphtho[2',1':4,5]indeno[1,2-*d*][1,3]dioxolan-4(2*H*)-one (compound **007)**

Compound **7-3** (112 mg, 0.4 mmol) and compound **1-5** (200 mg, 0.4 mmol) were dissolved in tetrahydrofuran (4 mL) and water (0.4 mL), potassium carbonate (149 mg, 1.1 mmol) and tetrakis(triphenylphosphine)palladium(0) (124 mg, 0.1 mmol) were added, and the mixture was stirred at 80 °C for 16 h under nitrogen atmosphere. After the reaction was completed as detected by LCMS, the reaction mixture was filtered, the residue was separated and purified by column chromatography (tetrahydrofuran/petroleum ether = 1/2), and the resulting crude product was separated and purified by preparative HPLC [column: Phenomenex C18 75 × 30 mm × 3 μm; mobile phase: A: water ($NH_4HCO_3$); B: acetonitrile, B%, 34%-58%, 10 min] to give compound **007** (37.7 mg, 20.52%).

LC-MS: Rt: 1.832 min; MS m/z (ESI): 611.5 [M+H] $^+$;
$^1$H NMR (400 MHz, DMSO-$d_6$) δ = 8.45 (s, 1H), 7.36 (d, *J* = 8.1 Hz, 2H), 7.31 (d, *J* = 10.1 Hz, 1H), 7.25 (d, *J* = 8.1 Hz, 2H), 6.93 (d, *J* = 8.0 Hz, 1H), 6.48 (d, *J* = 8.0 Hz, 1H), 6.17 (dd, *J* = 1.8, 10.1 Hz, 1H), 5.94 (s, 1H), 5.48 (s, 2H), 5.39 (s, 1H), 5.08 (s, 1H), 4.92 (d, *J* = 5.0 Hz, 1H), 4.78 (d, *J* = 2.9 Hz, 1H), 4.50 (dd, *J* = 3.0, 19.3 Hz, 1H), 4.29 (s, 1H), 4.17 (d, *J* = 18.4 Hz, 1H), 4.03 (s, 2H), 2.61 - 2.53 (m, 1H), 2.37 - 2.29 (m, 1H), 2.16 - 1.97 (m, 2H), 1.82 - 1.59 (m, 5H), 1.40 (s, 3H), 1.13 - 1.00 (m, 2H), 0.86 (s, 3H).

**Example 8:** synthesis of (6a*R*,6b*S*,7*S*,8a*S*,8b*S*,10*R*,11a*R*,12a*S*,12b*S*)-10-(4-((7-aminobenzo[*d*][1,3]dioxolan-4-yl)methyl)phenyl)-7-hydroxy-8b-(2-hydroxyacetyl)-6a,8a-dimethyl-6a,6b,7,8,8a,8b,11a,12,12a,12b-decahydro-1*H*-naphtho[2',1':4,5]indeno[1,2-*d*][1,3]dioxolan-4(2*H*)-one (compound **008)**

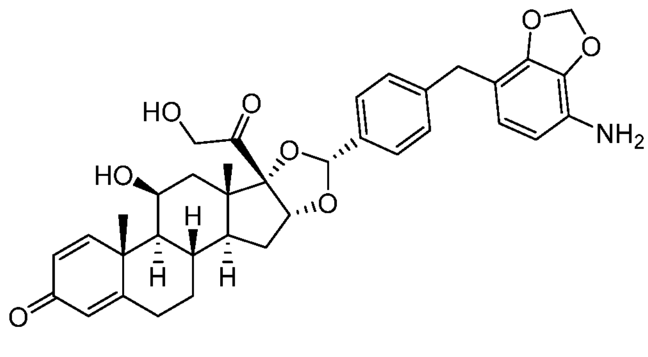

The synthetic route and the specific synthetic steps were as follows.

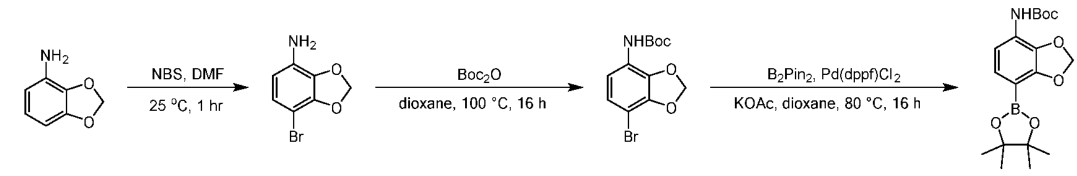


8-1 8-2 8-3 8-4

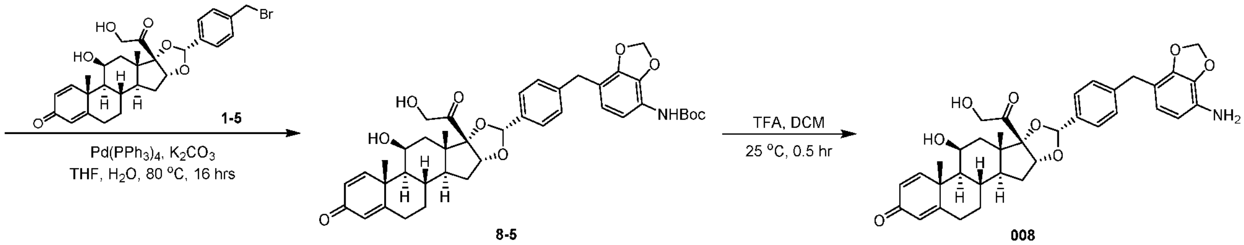


8-5 008

Step 1: synthesis of 7-bromobenzo[*d*][1,3]dioxolan-4-amine **8-2**

The starting material benzo[*d*][1,3]dioxolan-4-amine **8-1** (2.00 g, 14.6 mmol) was dissolved in *N,N*-dimethylformamide (50 mL), N-bromosuccinimide (2.50 g, 13.9 mmol) was added at 0 °C, and the mixture was stirred at 0 °C for 1 h. After the reaction was completed as detected by LCMS, ethyl acetate (200 mL) was added to dilute the reaction mixture, the mixture was washed with water (100 mL × 2) and saturated brine (50 mL), the organic phase was dried, filtered, and concentrated, and the residue was purified by column chromatography (petroleum ether/tetrahydrofuran = 2/1) to give 7-bromobenzo[*d*][1,3]dioxolan-4-amine **8-2** (1.30 g, 94.8%).
LC-MS: Rt: 1.311 min; MS m/z (ESI): 218.1 $[M+H]^+$;

Step 2: synthesis of *tert*-butyl (7-bromobenzo[*d*][1,3]dioxolan-4-yl)carbamate **8-3**

7-Bromo-benzo[*d*][1,3]dioxolan-4-amine **8-2** (1.00 g, 4.6 mmol) and di-*tert*-butyl dicarbonate (3.0 g, 13.9 mmol) were dissolved in 1,4-dioxane (20 mL), and the mixture was stirred at 100 °C for 16 h. After the reaction was completed as detected by LCMS, the reaction mixture was concentrated, and the residue was purified by column chromatography (petroleum ether/ethyl acetate = 6/1) to give *tert*-butyl (7-bromobenzo[*d*][1,3]dioxolan-4-yl)carbamate **8-3** (820 mg, 43.5%).

LC-MS: Rt: 1.707 min; MS m/z (ESI): 262.1 $[M+H-56]^+$;
$^1$H NMR (400 MHz, DMSO-$d_6$) $\delta$ = 8.95 (s, 1H), 6.98 - 6.89 (m, 2H), 6.10 (s, 2H), 1.44 (s, 9H). Step 3: synthesis of *tert*-butyl (7-(4,4,5,5-tetramethyl-1,3,2-dioxaborolan-2-yl)benzo[*d*][1,3]dioxolan-4-yl)carbamate **8-4**

*tert*-Butyl (7-bromobenzo[*d*][1,3]dioxolan-4-yl)carbamate **8-3** (820 mg, 2.60 mmol) and bis(pinacolato)diboron (1.30 g, 5.20 mmol) were dissolved in 1,4-dioxane (25 mL), potassium acetate (764 mg, 7.80 mmol) and [1,1' bis(diphenylphosphino)ferrocene]palladium dichloride (190 mg, 0.30 mmol) were added, and the mixture was stirred at 80 °C for 16 h under nitrogen atmosphere. After the reaction was completed as detected by LCMS, the reaction mixture was filtered and concentrated, and the residue was purified by column chromatography (petroleum ether/tetrahydrofuran = 6/1) to give *tert*-butyl (7-(4,4,5,5-tetramethyl-1,3,2-dioxaborolan-2-yl)benzo[*d*][1,3]dioxolan-4-yl)carbamate **8-4** (1.00 g, 83.8%).
LC-MS: Rt: 1.782 min; MS m/z (ESI): 308.3 $[M+H-56]^+$;

Step 4: synthesis of *tert*-butyl (7-(4-((6a*R*,6b*S*,7*S*,8a*S*,8b*S*,10*R*,11a*R*,12a*S*,12b*S*)-7-hydroxy-8b-(2-hydroxyacetyl)-6a,8a-dimethyl-4-oxo-2,4,6a,6b,7,8,8a,8b,11a,12,12a,12b-dodecahydro-1*H*-naphtho[2',1':4,5]indeno[1,2-*d*][1,3]dioxolan-10-yl)benzyl)benzo[*d*][1,3]dioxolan-4-yl)carbamate **8-5**

*tert*-Butyl (7-(4,4,5,5-tetramethyl-1,3,2-dioxaborolan-2-yl)benzo[*d*][1,3]dioxolan-4-yl)carbamate **8-4** (489 mg, 1.35 mmol) and (6a*R*,6b*S*,7*S*,8a*S*,8b*S*,10*R*,11a*R*,12a*S*,12b*S*)-10-(4-(bromomethyl)phenyl)-7-hydroxy-8b-(2-hydroxyacetyl)-6a,8a-dimethyl-6a,6b,7,8,8a,8b,11a,12,12a,12b-decahydro-1*H*-naphtho[2',1':4,5]indeno[1,2-*d*][1,3]dioxolan-4(2*H*)-one **1-5** (500 mg, 0.9 mmol) were dissolved in a mixed solvent of tetrahydrofuran (20 mL) and water (2 mL), potassium carbonate (372 mg, 2.70 mmol) and tetrakis(triphenylphosphine)palladium(0) (311 mg, 0.3 mmol) were added, and the mixture was stirred at 80 °C for 16 h under nitrogen atmosphere. After the reaction was completed as detected

by LCMS, water was added to dilute the reaction mixture, the mixture was extracted with ethyl acetate (100 mL), the organic phase was washed with saturated brine (30 mL), dried, filtered, and concentrated, and the residue was purified by column chromatography (petroleum ether/tetrahydrofuran = 1/1) and then separated and purified by preparative high performance liquid chromatography [column: Gemini NX C18 5 μm × 10 mm × 150 mm; mobile phase: A: 0.1% aqueous formic acid solution; B: acetonitrile, 49%-79%, 10 min] to give intermediate *tert*-butyl (7-(4-((6a*R*,6b*S*,7*S*,8a*S*,8b*S*,10*R*,11a*R*,12a*S*,12b*S*)-7-hydroxy-8b-(2-hydroxyacetyl)-6a,8a-dimethyl-4-oxo-2,4,6a,6b,7,8,8a,8b,11a,12,12a,12b-dodecahydro-1*H*-naphtho[2',1':4,5]indeno[1,2-*d*][1,3]dioxolan-10-yl)benzyl)benzo[*d*][1,3]dioxolan-4-yl)carbamate **8-5** (20 mg, 2.8%).
LC-MS: Rt: 1.742 min; MS m/z (ESI): 714.4 $[M+H]^+$;

Step 5: synthesis of (6a*R*,6b*S*,7*S*,8a*S*,8b*S*,10*R*,11a*R*,12a*S*,12b*S*)-10-(4-((7-aminobenzo[*d*][1,3]dioxolan-4-yl)methyl)phenyl)-7-hydroxy-8b-(2-hydroxyacetyl)-6a,8a-dimethyl-6a,6b,7,8,8a,8b,11a,12,12a,12b-decahydro-1*H*-naphtho[2',1':4,5]indeno[1,2-*d*][1,3]dioxolan-4(2*H*)-one (compound **008)**

*tert*-Butyl (7-(4-((6a*R*,6b*S*,7*S*,8a*S*,8b*S*,10*R*,11a*R*,12a*S*,12b*S*)-7-hydroxy-8b-(2-hydroxyacetyl)-6a,8a-dimethyl-4-oxo-2,4,6a,6b,7,8,8a,8b,11a,12,12a,12b-dodecahydro-1*H*-naphtho[2',1':4,5]indeno[1,2-*d*][1,3]dioxolan-10-yl)benzyl)benzo[*d*][1,3]dioxolan-4-yl)carbamate **8-5** (20 mg, 0.03 mmol) was dissolved in dichloromethane (4 mL), trifluoroacetic acid (1 mL) was added, and the mixture was stirred at 25 °C for 0.5 h. After the reaction was completed as detected by LCMS, The reaction mixture was concentrated, and the residue was separated and purified by preparative high performance liquid chromatography [column: Xtimate C18 100 × 30 mm × 10 μm; mobile phase: A: 0.1% aqueous ammonia solution; B: acetonitrile, B%, 20%-70%, 35 min] to give (6a*R*,6b*S*,7*S*,8a*S*,8b*S*,10*R*,11a*R*,12a*S*,12b*S*)-10-(4-((7-aminobenzo[*d*][1,3]dioxolan-4-yl)methyl)phenyl)-7-hydroxy-8b-(2-hydroxyacetyl)-6a,8a-dimethyl-6a,6b,7,8,8a,8b,11a,12,12a,12b-decahydro-1*H*-naphtho[2',1':4,5]indeno[1,2-*d*][1,3]dioxolan-4(2*H*)-one (compound **008)** (3.1 mg, 17.3%).

LC-MS: Rt: 1.681 min; MS m/z (ESI): 614.5 $[M+H]^+$;
$^1$H NMR (400 MHz, DMSO-$d_6$) δ = 7.36 (d, *J* = 8.0 Hz, 2H), 7.32 (d, *J* = 10.1 Hz, 1H), 7.20 (d, *J* = 7.9 Hz, 2H), 6.42 (d, *J* = 8.3 Hz, 1H), 6.22 - 6.15 (m, 2H), 5.94 (s, 1H), 5.88 (s, 2H), 5.40 (s, 1H), 5.11 (t, *J* = 5.9 Hz, 1H), 4.92 (d, *J* = 5.0 Hz, 1H), 4.83 - 4.75 (m, 3H), 4.51 (dd, *J* = 6.3, 19.4 Hz, 1H), 4.30 (s, 1H), 4.18 (dd, *J* = 5.6, 19.4 Hz, 1H), 3.73 (s, 2H), 2.31 (d, *J* = 2.3 Hz, 1H), 2.15 - 2.00 (m, 2H), 1.81 - 1.61 (m, 5H), 1.40 (s, 3H), 1.25 (s, 1H), 1.13 - 1.01 (m, 2H), 0.87 (s, 3H).

**Example 9:** synthesis of (6a*R*,6b*S*,7*S*,8a*S*,8b*S*,10*R*,11a*R*,12a*S*,12b*S*)-10-(4-((7-amino-2,3-dihydrobenzofuran-5-yl)methyl)phenyl)-7-hydroxy-8b-(2-hydroxyacetyl)-6a,8a-dimethyl-6a,6b,7,8,8a,8b,11a,12,12a,12b-decahydro-1*H*-naphtho[2',1':4,5]indeno[1,2-*d*][1,3]dioxolan-4(2*H*)-one (compound **009)**

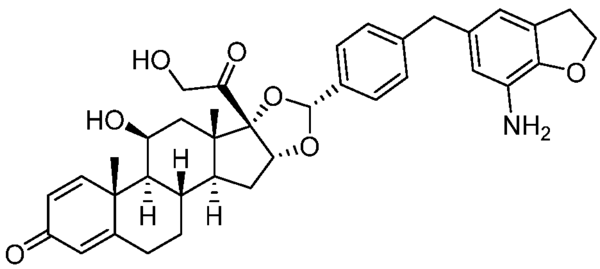

The synthetic route and the specific synthetic steps were as follows.

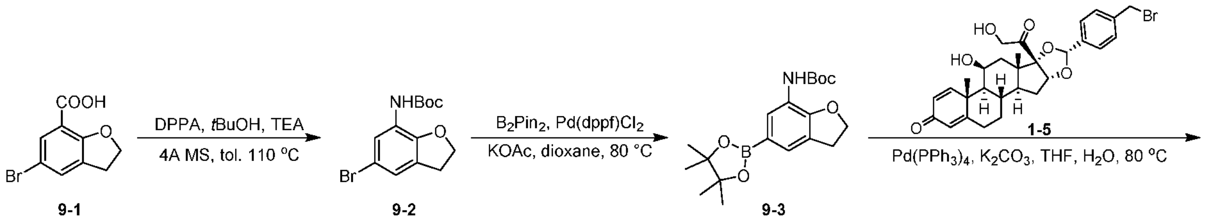


9-1 9-2 9-3

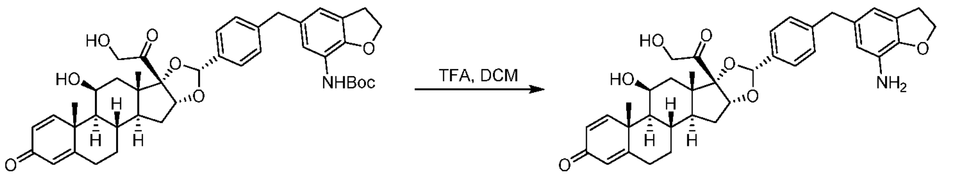


9-4 009

Step 1: synthesis of *tert*-butyl (5-bromo-2,3-dihydrobenzofuran-7-yl)carbamate **9-2**

5-Bromo-2,3-dihydrobenzofuran-7-carboxylic acid **9-1** (1.00 g, 4.11 mmol) was dissolved in toluene (10 mL) and *tert*-butanol (10 mL), 4A molecular sieves (200 mg) and triethylamine (1.25 g, 12.3 mmol) were added at room temperature, and the mixture was stirred at 110 °C for 0.5 h and cooled to room temperature. Diphenylphosphoryl azide (1.70 g, 6.17 mmol) was added dropwise, and the mixture was warmed to 110 °C and stirred for 10 h. After the reaction was completed as detected by LCMS, the reaction mixture was filtered and diluted with ethyl acetate (150 mL), the organic phase was washed with saturated brine (30 mL), dried, filtered, and concentrated, and the residue was separated and purified by column chromatography (0-40% ethyl acetate/petroleum ether) to give intermediate *tert*-butyl (5-bromo-2,3-dihydrobenzofuran-7-yl)carbamate **9-2** (1.03 g, 79.7%).

LC-MS: Rt: 1.065 min; MS m/z (ESI): 259.7 $[M+H-56]^+$;
$^1H$ NMR (400 MHz, DMSO-$d_6$) $\delta$ = 8.59 (s, 1H), 7.50 (br s, 1H), 7.13 -7.11 (m, 1H), 4.61 - 4.50 (m, 2H), 3.24 - 3.17 (m, 2H), 1.48 - 1.41 (m, 9H).

Step 2: synthesis of *tert*-butyl (5-(4,4,5,5-tetramethyl-1,3,2-dioxaborolan-2-yl)-2,3-dihydrobenzofuran-7-yl)carbamate **9-3**

*tert*-Butyl (5-bromo-2,3-dihydrobenzofuran-7-yl)carbamate **9-2** (600 mg, 1.91 mmol), bis(pinacolato)diboron (533 mg, 2.10 mmol), potassium acetate (562 mg, 5.73 mmol), and 1,1'-bis(diphenylphosphino)ferrocene palladium chloride (139 mg, 190 μmol) were dissolved in anhydrous 1,4-dioxane (10 mL), and the mixture was reacted at 80 °C for 16 h under nitrogen atmosphere. After the reaction was completed as detected by LCMS, the reaction mixture was filtered and concentrated, and the residue was separated and purified by column chromatography (0-10% tetrahydrofuran/petroleum ether) to give intermediate *tert*-butyl (5-(4,4,5,5-tetramethyl-1,3,2-dioxaborolan-2-yl)-2,3-dihydrobenzofuran-7-yl)carbamate **9-3** (990 mg, 71.8%).

LC-MS: Rt: 4.417 min; MS m/z (ESI): 306.2 $[M+H-56]^+$;
$^1H$ NMR (400 MHz, CHLOROFORM-*d*) $\delta$ = 8.17 - 8.27 (m, 1 H) 7.38 (s, 1 H) 7.25 - 7.31 (m, 1 H) 4.59 - 4.66 (m, 2 H) 3.20 - 3.29 (m, 2 H) 1.33 (s, 12 H) 1.31 (s, 9 H)

Step 3: synthesis of *tert*-butyl (5-(4-((6a*R*,6b*S*,7*S*,8a*S*,8b*S*,10*R*,11a*R*,12a*S*,12b*S*)-7-hydroxy-8b-(2-hydroxyacetyl)-6a,8a-dimethyl-4-oxo-2,4,6a,6b, 7,8,8a,8b, 11a, 12, 12a, 12b-dodecahydro-1*H*-naphtho[2',1':4,5]indeno[1,2-*d*][1,3]dioxolan-10-yl)benzyl)-2,3-dihydrobenzofuran-7-yl)carbamate **9-4**

(6a*R*,6b*S*,7*S*,8a*S*,8b*S*,10*R*,11a*R*,12a*S*,12b*S*)-10-(4-(Bromomethyl)phenyl)-7-hydroxy-8b-(2-hydroxyacetyl)-6a,8a-dimethyl-6a,6b,7,8,8a,8b, 11a, 12, 12a, 12b-decahydro-1*H*-naphtho[2',1':4,5]indeno[1,2-*d*][1,3]dioxolan-4(2*H*)-one **1-5** (300 mg, 538 μmol), *tert*-butyl (5-(4,4,5,5-tetramethyl-1,3,2-dioxaborolan-2-yl)-2,3-dihydrobenzofuran-7-yl)carbamate **9-3** (300 mg, 830 μmol), tetrakis(triphenylphosphine)palladium(0) (62.2 mg, 53.8 μmol), and potassium carbonate (148 mg, 1.08 mmol) were dissolved in anhydrous tetrahydrofuran (5 mL) and water (1 mL), and the mixture was stirred at 80 °C for 2 h under nitrogen atmosphere. After the reaction was completed as detected by LCMS, the reaction mixture was extracted with ethyl acetate (50 mL), the organic phase was washed with saturated brine (10 mL),

dried, filtered, and concentrated, and the residue was separated and purified by column chromatography (0-50% tetrahydrofuran/petroleum ether) to give intermediate (5-(4-((6a*R*,6b*S*,7*S*,8a*S*,8b*S*,10*R*,11a*R*,12a*S*,12b*S*)-7-hydroxy-8b-(2-hydroxyacetyl)-6a,8a-dimethyl-4-oxo-2,4,6a,6b, 7,8,8a,8b, 11a, 12, 12a, 12b-dodecahydro-1*H*-naphtho[2',1':4,5]indeno[1,2-*d*][1,3]dioxolan-10-yl)benzyl)-2,3-dihydrobenzofuran-7-yl)carbamate **9-4** (300 mg, 78.3%). LC-MS: Rt: 2.156 min; MS m/z (ESI): 712.3 $[M+H]^+$;

Step 4: synthesis of (6a*R*,6b*S*,7*S*,8a*S*,8b*S*,10*R*,11a*R*,12a*S*,12b*S*)-10-(4-((7-amino-2,3-dihydrobenzofuran-5-yl)methyl)phenyl)-7-hydroxy-8b-(2-hydroxyacetyl)-6a,8a-dimethyl-6a,6b,7,8,8a,8b,11a,12,12a,12b-decahydro-1*H*-naphtho[2',1':4,5]indeno[1,2-*d*][1,3]dioxolan-4(2*H*)-one (compound **009)**

The intermediate *tert*-butyl (5-(4-((6a*R*,6b*S*,7*S*,8a*S*,8b*S*,10*R*,11a*R*,12a*S*,12b*S*)-7-hydroxy-8b-(2-hydroxyacetyl)-6a,8a-dimethyl-4-oxo-2,4,6a,6b,7,8,8a,8b,11a,12,12a,12b-dodecahydro-1*H*-naphtho[2',1':4,5]indeno[1,2-*d*][1,3]dioxolan-10-yl)benzyl)-2,3-dihydrobenzofuran-7-yl)carbamate **9-4** (220 mg, 309 μmol) was dissolved in anhydrous dichloromethane (4 mL), trifluoroacetic acid (1 mL) was added, and the mixture was stirred at 25 °C for 40 min. After the reaction was completed as detected by LCMS, The reaction mixture was blow-dried with nitrogen, and the resulting residue was separated and purified by preparative high performance liquid chromatography [column: YMC-Pack CN 150 × 30 mm × 5 μm; mobile phase: A: 0.1% aqueous formic acid solution; B: acetonitrile, B%, 40%-60%, 12 min] to give the compound (6a*R*,6b*S*,7*S*,8a*S*,8b*S*,10*R*,11a*R*,12a*S*,12b*S*)-10-(4-((7-amino-2,3-dihydrobenzofuran-5-yl)methyl)phenyl)-7-hydroxy-8b-(2-hydroxyacetyl)-6a, 8a-dimethyl-6a,6b,7,8,8a,8b,11a,12,12a,12b-decahydro-1*H*-naphtho[2',1':4,5]indeno[1,2-*d*][1,3]dioxolan-4(2*H*)-one 009 (2.00 mg, 1.06%).

LC-MS: Rt: 2.576 min; MS m/z (ESI): 612.3 $[M+H]^+$;
$^1$H NMR (400 MHz, DMSO-$d_6$) $\delta$ = 7.33 - 7.38 (m, 2 H) 7.31 (d, *J* = 10.13 Hz, 1 H) 7.19 (d, *J* = 8.13 Hz, 2 H) 6.29 (d, *J* = 17.26 Hz, 2 H) 6.17 (dd, *J* = 10.13, 1.88 Hz, 1 H) 5.94 (s, 1 H) 5.39 (s, 1 H) 5.08 (s, 1 H) 4.90 - 4.94 (m, 1 H) 4.77 - 4.81 (m, 1 H) 4.45 - 4.73 (m, 3 H) 4.42 (t, *J* = 8.63 Hz, 2 H) 4.29 (d, *J* = 2.88 Hz, 1 H) 4.13 - 4.23 (m, 1 H) 3.71 (s, 2 H) 3.04 (t, *J* = 8.69 Hz, 2 H) 2.30 (s, 1 H) 2.09 - 2.17 (m, 1 H) 1.98 - 2.06 (m, 1 H) 1.56 - 1.84 (m, 6 H) 1.40 (s, 3 H) 0.97 - 1.11 (m, 2 H) 0.86 (s, 3 H).

**Example 10:** synthesis of (2*S*,6a*S*,6b*R*,7*S*,8a*S*,8b*S*,10*R*,11a*R*,12a*S*,12b*S*)-10-(4-((7-amino-2,3-dihydrobenzofuran-4-yl)methyl)phenyl)-2, 6b-difluoro-7-hydroxy-8b-(2-hydroxyacetyl)-6a, 8a-dimethyl-6a,6b,7,8,8a,8b,11a,12,12a,12b-decahydro-1*H*-naphtho[2',1':4,5]indeno[1,2-*d*][1,3]dioxolan-4(2*H*)-one (compound **010)**

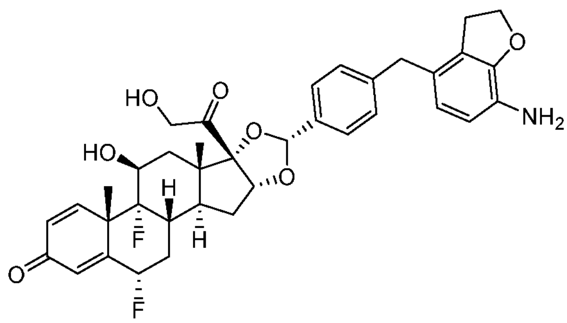

The synthetic route and the specific synthetic steps were as follows.

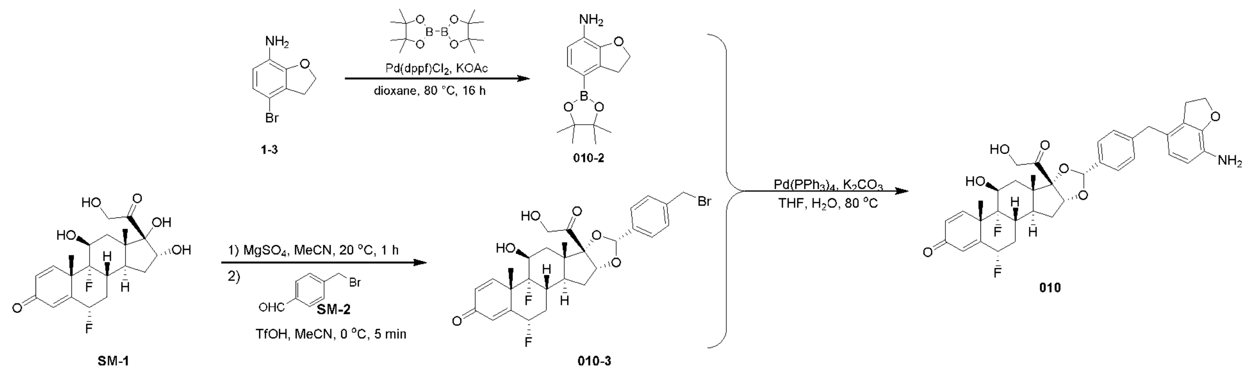


1-3 010-2 SM-1 010-3 010

Step 1: synthesis of (2*S*,6a*S*,6b*R*,7*S*,8a*S*,8b*S*,10*R*,11a*R*,12a*S*,12b*S*)-10-(4-(bromomethyl)phenyl)-2,6b-difluoro-7-hydroxy-8b-(2-hydroxyacetyl)-6a,8a-dimethyl-6a,6b,7,8,8a,8b, 11a, 12, 12a, 12b-decahydro-1*H*-naphtho[2',1':4,5]indeno[1,2-*d*][1,3]dioxolan-4(2*H*)-one **010-3**

The starting material 6-alpha,9-alpha-difluoro-11-beta, 16-alpha, 17-alpha,21-tetrahydroxypregna-1,4-diene-3,20-dione **SM-1** (1.00 g, 2.42 mmol) was dissolved in anhydrous acetonitrile (10 mL), anhydrous magnesium sulfate (1.46 g, 12.1 mmol) was added, and the mixture was stirred at 25 °C for 1 h. A solution of 4-bromomethylbenzaldehyde (579 mg, 2.91 mmol) in acetonitrile (2 mL) was added at 0 °C, a solution of trifluoromethanesulfonic acid (1.82 g, 12.12 mmol) in acetonitrile (2 mL) was slowly added dropwise, and the mixture was stirred at 0 °C for 5 min. After the reaction was completed as detected by LCMS, the reaction was quenched with a saturated aqueous sodium bicarbonate solution (20 mL), the mixture was extracted with ethyl acetate (50 mL × 2), the organic phase was washed with a saturated aqueous sodium chloride solution (20 mL), dried, filtered, and concentrated, and the residue was separated and purified by Prep-HPLC [column: YMC-Pack CN 150 × 30 mm × 5 μm; mobile phase: A, 0.1% aqueous formic acid solution; B, acetonitrile, B%, 53%-63%, 12 min] to give intermediate (2*S*,6a*S*,6b*R*,7*S*,8a*S*,8b*S*,10*R*,11a*R*,12a*S*,12b*S*)-10-(4-(bromomethyl)phenyl)-2, 6b-difluoro-7-hydroxy-8b-(2-hydroxyacetyl)-6a, 8a-dimethyl-6a,6b,7,8,8a,8b,11a,12,12a,12b-decahydro-1*H*-naphtho[2',1':4,5]indeno[1,2-*d*][1,3]dioxolan-4(2*H*)-one **010-3** (640 mg, 42%).

LC-MS: Rt: 2.845 min; MS m/z (ESI): 595.1 $[M+H]^+$;
$^1$H NMR (400MHz, DMSO-$d_6$) $\delta$ = 7.62 - 7.39 (m, 4H), 7.27 (d, *J*= 10.3 Hz, 1H), 6.30 (dd, *J* = 1.8, 10.0 Hz, 1H), 6.13 (s, 1H), 5.77 - 5.45 (m, 3H), 5.01 - 4.95 (m, 1H), 4.68 (s, 2H), 4.55 (br d, *J* = 19.3 Hz, 1H), 4.22 (br d, *J* = 19.3 Hz, 2H), 2.74 - 2.56 (m, 1H), 2.37 - 2.12 (m, 3H), 2.09 - 1.95 (m, 1H), 1.77 - 1.64 (m, 3H), 1.56 - 1.42 (m, 4H), 0.87 (s, 3H);
$^{19}$F NMR (376MHz, DMSO-$d_6$) $\delta$ = -164.99 (s, 1F), -186.39 (s, 1F).

Step 2: synthesis of 4-(4,4,5,5-tetramethyl-1,3,2-dioxaborolan-2-yl)-2,3-dihydrobenzofuran-7-amine **010-2**

4-Bromo-2,3-dihydrobenzofuran-7-amine **1-3** (7.30 g, 34.1 mmol) was dissolved in 1,4-dioxane (150 mL), bis(pinacolato)diboron (20.0 g, 78.8 mmol), 1,1'-bis(diphenylphosphino)ferrocene palladium chloride (2.50 g, 3.4 mmol), and potassium acetate (10.0 g, 102.3 mmol) were added, and the mixture was stirred at 80 °C for 16 h under nitrogen atmosphere. After the reaction was completed as detected by LCMS, the reaction mixture was filtered and concentrated, and the residue was separated and purified by column chromatography (tetrahydrofuran/petroleum ether = 1/6) to give intermediate 4-(4,4,5,5-tetramethyl-1,3,2-dioxaborolan-2-yl)-2,3-dihydrobenzofuran-7-amine **010-2** (9.50 g, 96.0%).
LC-MS: Rt: 1.470 min; MS m/z (ESI): 262.0 [M+H];

Step 3: synthesis of (2*S*,6a*S*,6b*R*,7*S*,8a*S*,8b*S*,10*R*,11a*R*,12a*S*,12b*S*)-10-(4-((7-amino-2,3-dihydrobenzofuran-4-yl)methyl)phenyl)-2, 6b-difluoro-7-hydroxy-8b-(2-hydroxyacetyl)-6a, 8a-dimethyl-6a,6b,7,8,8a,8b,11a,12,12a,12b-decahydro-1*H*-naphtho[2',1':4,5]indeno[1,2-*d*][1,3]dioxolan-4(2*H*)-one **010**

(2*S*,6a*S*,6b*R*,7*S*,8a*S*,8b*S*,10*R*,11a*R*,12a*S*,12b*S*)-10-(4-(bromomethyl)phenyl)-2,6b-difluoro-7-hydroxy-8b-(2-hydroxyacetyl)-6a,8a-dimethyl-6a,6b,7,8,8a,8b,11a,12,12a,12b-decahydro-1*H*-naphtho[2',1':4,5]indeno[1,2-*d*][1,3]dioxolan-4(2*H*)-one **010-3** (150 mg, 253 μmol) was dissolved in tetrahydrofuran (2 mL) and water (0.5 mL), 4-(4,4,5,5-tetramethyl-1,3,2-dioxaborolan-2-yl)-2,3-dihydrobenzofuran-7-amine (79 mg, 303 μmol), potassium carbonate (79 mg, 303 μmol), and tetrakis(triphenylphosphine)palladium(0) (29 mg, 25.3 μmol) were added sequentially, and the mixture was reacted at 80 °C for 2 h under nitrogen atmosphere. After the reaction was completed as detected by LCMS, the reaction mixture was extracted with ethyl acetate (10 mL × 3), the organic phase was washed with a saturated aqueous sodium chloride solution (5 mL), dried, filtered, and concentrated, and the residue was separated and purified by preparative high performance liquid chromatography [column: Phenomenex C18 75 × 30 mm × 3 μm; mobile phase: A: 0.1% aqueous ammonia solution; B: acetonitrile, B%, 30%-70%, 9 min] to give the compound (2*S*,6a*S*,6b*R*,7*S*,8a*S*,8b*S*,10*R*,11a*R*,12a*S*,12b*S*)-10-(4-((7-amino-2,3-dihydrobenzofuran-4-yl)methyl)phenyl)-2, 6b-difluoro-7-hydroxy-8b-(2-hydroxyacetyl)-6a, 8a-dimethyl-6a,6b,7,8,8a,8b,11a,12,12a,12b-decahydro-1*H*-naphtho[2',1':4,5]indeno[1,2-*d*][1,3]dioxolan-4(2*H*)-one **010** (20.0 mg).

LC-MS: Rt: 2.092 min; MS m/z (ESI): 648.3 $[M+H]^+$;
$^1$H NMR (400MHz, DMSO-$d_6$) $\delta$ = 7.33 (d, *J* = 8.0 Hz, 2H), 7.27 (d, *J* = 9.8 Hz, 1H), 7.18 (d, *J* = 8.0 Hz, 2H), 6.42 - 6.37 (m, 2H), 6.30 (dd, *J*= 1.8, 10.3 Hz, 1H), 6.13 (s, 1H), 5.75 - 5.56 (m, 1H), 5.52 (d, *J* = 2.8 Hz, 1H), 5.45 (s, 1H), 5.11 (t, *J* = 6.0 Hz, 1H), 4.95 (d, *J* = 4.8 Hz, 1H), 4.56 - 4.38 (m, 5H), 4.25 - 4.15 (m, 2H), 3.73 (s, 2H), 2.97 (t, *J* = 8.7 Hz, 2H), 2.44 (br s, 1H), 2.32 - 2.19 (m, 2H), 2.10 - 2.00 (m, 1H), 1.78 - 1.64 (m, 3H), 1.50 (s, 4H), 0.86 (s, 3H);
$^{19}$F NMR (376MHz, DMSO-$d_6$) $\delta$ = -164.96 (s, 1F), -186.39 (s, 1F).

**Example 11:** synthesis of (6a*R*,6b*S*,7*S*,8a*S*,8b*S*,10*R*,11a*R*,12a*S*,12b*S*)-10-(4-((7-amino-2,3-dihydro-1*H*-inden-4-yl)methyl)phenyl)-7-hydroxy-8b-(2-hydroxyacetyl)-6a,8a-dimethyl-6a,6b,7,8,8a,8b,11a,12,12a,12b-decahydro-1*H*-naphtho[2',1':4,5]indeno[1,2-*d*][1,3]dioxolan-4(2*H*)-one (compound **011)**

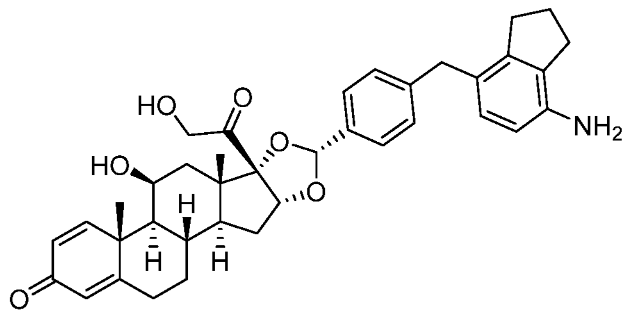

The synthetic route and the specific synthetic steps were as follows.

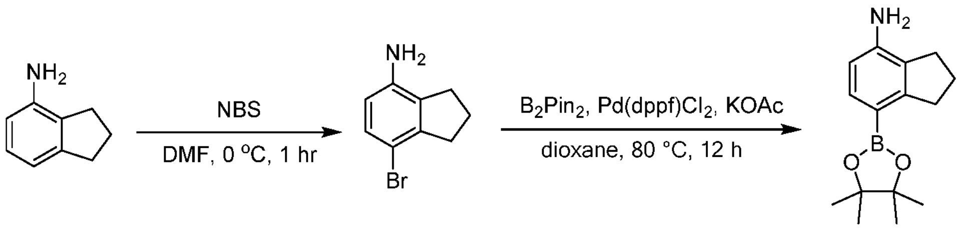


**11-1** **11-2** **11_3**

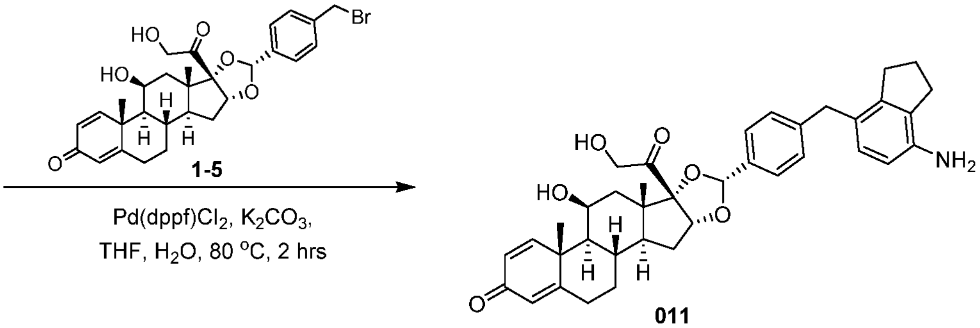


**011**

Step 1: synthesis of 7-bromo-2,3-dihydro-1*H*-inden-4-amine **11-2**

2,3-Dihydro-1*H*-inden-4-amine (2.00 g, 15.0 mmol) was dissolved in *N,N*-dimethylformamide (20 mL), N-bromosuccinimide (NBS) (2.67 g, 15.0 mmol) was added at 0 °C, and the mixture was stirred at 0 °C for 1 h. After the

reaction was completed as detected by LCMS, the mixture was quenched with a saturated sodium bicarbonate solution (50 mL) at 0 °C and extracted with ethyl acetate (100 mL × 2), the organic phase was washed with water (50 mL × 3) and saturated brine (50 mL), dried, filtered, and concentrated, and the resulting residue was separated and purified by column chromatography (0-15% ethyl acetate/petroleum ether) to give 7-bromo-2,3-dihydro-1*H*-inden-4-amine **11-2** (1.70 g, 53.4%).
LC-MS: Rt: 0.800 min; MS m/z (ESI): 213.8 $[M+H]^+$;

Step 2: synthesis of 7-(4,4,5,5-tetramethyl-1,3,2-dioxaborolan-2-yl)-2,3-dihydro-1*H*-inden-4-amine **11-3**

7-Bromo-2,3-dihydro-1*H*-inden-4-amine **11-2** (100 mg, 471 μmol) and bis(pinacolato)diboron (239 mg, 943 μmol) were dissolved in 1,4-dioxane (5 mL), 1,1'-bis(diphenylphosphino)ferrocene palladium chloride (34.5 mg, 47.2 μmol) and potassium acetate (92.5 mg, 943 μmol) were added, and the mixture was stirred at 80 °C for 12 h under nitrogen atmosphere. After the reaction was completed as detected by LCMS, the reaction mixture was filtered and concentrated, and the resulting residue was separated and purified by column chromatography (0-60% ethyl acetate/petroleum ether) to give intermediate 7-(4,4,5,5-tetramethyl-1,3,2-dioxaborolan-2-yl)-2,3-dihydro-1*H*-inden-4-amine **11-3** (77.0 mg, 63.0%).
LC-MS: Rt: 0.914 min; MS m/z (ESI): 260.3 $[M+H]^+$;

Step 3: synthesis of (6a*R*,6b*S*,7*S*,8a*S*,8b*S*,10*R*,11a*R*,12a*S*,12b*S*)-10-(4-((7-amino-2,3-dihydro-1*H*-inden-4-yl)methyl)phenyl)-7-hydroxy-8b-(2-hydroxyacetyl)-6a,8a-dimethyl-6a,6b,7,8,8a,8b,11a,12,12a,12b-decahydro-1*H*-naphtho[2',1':4,5]indeno[1,2-*d*][1,3]dioxolan-4(2*H*)-one **011**

7-(4,4,5,5-Tetramethyl-1,3,2-dioxaborolan-2-yl)-2,3-dihydro-1*H*-inden-4-amine **11-3** (77 mg, 295.98 μmol) and (6a*R*,6b*S*,7*S*,8a*S*,8b*S*,10*R*,11a*R*,12a*S*,12b*S*)-10-(4-(bromomethyl)phenyl)-7-hydroxy-8b-(2-hydroxyacetyl)-6a,8a-dimethyl-6a,6b,7,8,8a,8b,11a,12,12a,12b-decahydro-1*H*-naphtho[2',1':4,5]indeno[1,2-*d*][1,3]dioxolan-4(2*H*)-one **1-5** (110 mg, 197 μmol) were dissolved in tetrahydrofuran (5 mL) and water (1 mL), 1,1'-bis(diphenylphosphino)ferrocene palladium chloride (14.4 mg, 19.7 μmol) and potassium carbonate (54.5 mg, 394 μmol) were added, and the mixture was stirred for reaction at 80 °C for 2 h under nitrogen atmosphere. After the reaction was completed as detected by LCMS, the mixture was diluted with water (20 mL) and then extracted with ethyl acetate (50 mL × 2), the organic phase was washed with saturated brine (20 mL), dried, filtered, and concentrated, and the resulting residue was separated and purified by preparative high performance liquid chromatography [column: Boston Prime C18 150 × 30 mm × 5 μm; mobile phase: A: 0.1% aqueous formic acid solution; B: ACN, 29%-49%, 12 min] to give (6a*R*,6b*S*,7*S*,8a*S*,8b*S*,10*R*,11a*R*,12a*S*,12*b*S)-10-(4-((7-amino-2,3-dihydro-1*H*-inden-4-yl)methyl)phenyl)-7-hydroxy-8b-(2-hydroxyacetyl)-6a,8a-dimethyl-6a,6b,7,8,8a,8b,11a,12,12a,12b-decahydro-1*H*-naphtho[2',1':4,5]indeno[1,2-*d*][1,3]dioxolan-4(2*H*)-one (55.0 mg, 45.7%).

LC-MS: Rt: 0.856 min; MS m/z (ESI): 610.4 $[M+H]^+$;
$^1$H NMR (400 MHz, DMSO-$d_6$) $\delta$ = 7.37 - 7.28 (m, 3H), 7.16 - 7.11 (m, 1H), 7.14 (d, *J* = 7.8 Hz, 1H), 6.66 (d, *J* = 7.8 Hz, 1H), 6.33 (d, *J* = 8.0 Hz, 1H), 6.16 (dd, *J* = 1.6, 10.2 Hz, 1H), 5.93 (s, 1H), 5.39 (s, 1H), 5.08 (s, 1H), 4.91 (d, *J*= 5.3 Hz, 1H), 4.80 - 4.75 (m, 1H), 4.75 - 4.55 (m, 2H), 4.50 (d, *J* = 17.8 Hz, 1H), 4.29 (s, 1H), 4.17 (d, *J*= 18.1 Hz, 1H), 3.73 (s, 2H), 2.66 (t, *J* = 7.5 Hz, 2H), 2.63 - 2.57 (m, 2H), 2.57 - 2.53 (m, 1H), 2.30 (s, 1H), 2.16 - 1.98 (m, 2H), 1.91 (quin, *J* = 7.4 Hz, 2H), 1.81 - 1.60 (m, 5H), 1.40 (s, 3H), 1.11 - 0.97 (m, 2H), 0.90 - 0.81 (m, 3H).

**Example 12:** synthesis of (6a*R*,6b*S*,7*S*,8a*S*,8b*S*,10*R*,11a*R*,12a*S*,12b*S*)-10-(4-((6-amino-2,3-dihydrobenzofuran-4-yl)methyl)phenyl)-7-hydroxy-8b-(2-hydroxyacetyl)-6a,8a-dimethyl-6a,6b,7,8,8a,8b,11a,12,12a,12b-decahydro-1*H*-naphtho[2',1':4,5]indeno[1,2-*d*][1,3]dioxolan-4(2*H*)-one (compound **012)**

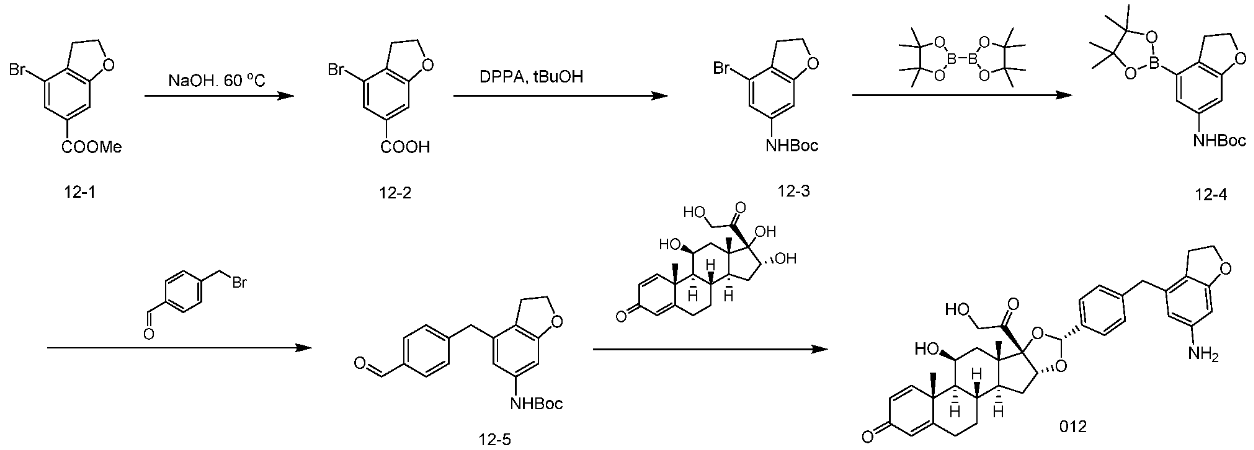


12-1 12-2 12-3 12-4 12-5 012

Compound 012 was prepared by referring to Example 5 and Example 9 and using compound 12-1 (CAS 1677706-25-0) as the starting material.

LC-MS: MS m/z (ESI): 612.3 [M+H]$^+$.

**Example 13:** synthesis of (6a*R*,6b*S*,7*S*,8a*S*,8b*S*,10*R*,11a*R*,12a*S*,12b*S*)-10-(4-((5-aminobenzo[*d*]oxazol-7-yl)methyl)phenyl)-7-hydroxy-8*b*-(2-hydroxyacetyl)-6*a*,8*a*-dimethyl-6*a*,6*b*,7,8,8*a*,8*b*,11*a*,12,12*a*,12*b*-decahydro-1*H*-naphtho[2',1':4,5]indeno[1,2-*d*][1,3]dioxolan-4(2*H*)-one (compound **013)**

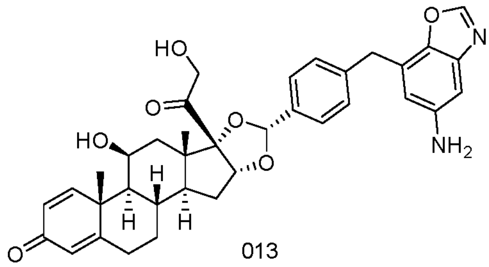

013

Compound 013 was prepared by referring to Example 7 and using compound

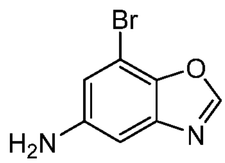

(CAS 1267216-18-1) as the starting material.

LC-MS: MS m/z (ESI): 611.5 [M+H] $^+$.

**Example 14:** synthesis of 2-((6a*R*,6b*S*,7*S*,8a*S*,8b*S*,10*R*,11a*R*,12a*S*,12b*S*)-10-(4-((7-amino-2,3-dihydrobenzofuran-5-yl)methyl)phenyl)-7-hydroxy-6a,8a-dimethyl-4-oxo-2,4,6a,6b,7,8,8a,8b,11a,12,12a,12b-dodecahydro-1*H*-naphtho[2',1':4,5]indeno[1,2-*d*][1,3]dioxolan-8b-yl)-2-oxoethyl dihydrogen phosphate (compound **009-p)**

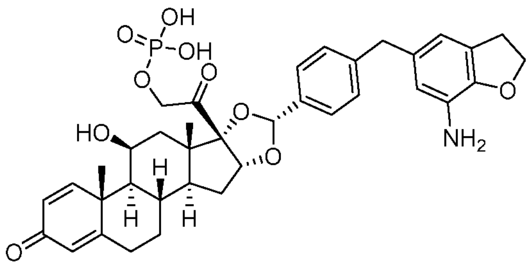

The synthetic route and the specific synthetic steps were as follows.

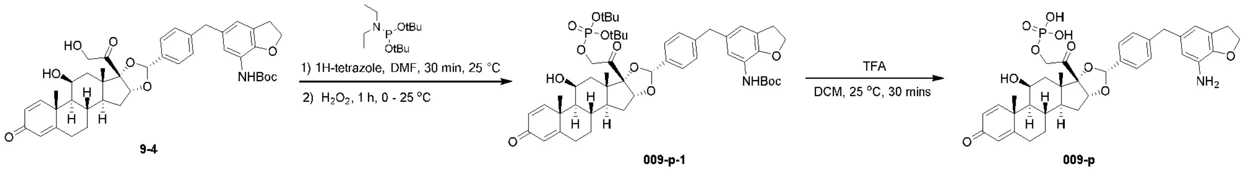


Step 1: synthesis of tert-butyl (5-(4-((6a*R*,6b*S*,7*S*,8a*S*,8b*S*,10*R*,11a*R*,12a*S*,12b*S*)-8b-(2-((di-*tert*-butoxyphosphoryl)oxo)acetyl)-7-hydroxy-6a,8a-dimethyl-4-oxo-2,4,6a,6b,7,8,8a,8b,11a,12,12a,12b-dodecahydro-1*H*-naphtho[2',1:'4,5]indeno[1,2-*d*][1,3]dioxolan-10-yl)benzyl)-2,3-dihydrobenzofuran-7-yl)carbamate **(009-p-1)**

Intermediate **9-4** (80.0 mg, 112 μmol) was dissolved in *N,N*-dimethylformamide (4 mL), di-*tert*-butyl *N,N*-diethylphosphoramidite (336 mg, 1.35 mmol) and tetrazole (78.7 mg, 1.12 mmol) were added, and the mixture was stirred at room temperature for 30 min. After the reaction was completed as monitored by TLC, the mixture was cooled to 0 °C, hydrogen peroxide (580 mg, 5.12 mmol) was added to the reaction mixture, and the mixture was returned to room temperature and stirred for 1 h. After the reaction was completed as detected by LCMS, the reaction was quenched with an aqueous sodium sulfite solution (10 mL) at 0 °C, the mixture was extracted with ethyl acetate (30 mL), the organic phase was washed with saturated brine (10 mL), dried, filtered, and concentrated, and the resulting residue was separated and purified by column chromatography (0-60% ethyl acetate/petroleum ether) to give intermediate **009-p-1** (50.0 mg, 49.2%).
LC-MS: Rt: 1.216 min; MS m/z (ESI): 904.6 $[M+H]^+$;

Step 2: synthesis of 2-((6a*R*,6b*S*,7*S*,8a*S*,8b*S*,10*R*,11a*R*,12a*S*,12b*S*)-10-(4-((7-amino-2,3-dihydrobenzofuran-5-yl)methyl)phenyl)-7-hydroxy-6a,8a-dimethyl-4-oxo-2,4,6a,6b,7,8,8a,8b,11a,12,12a,12b-dodecahydro-1*H*-naphtho[2',1':4,5]indeno[1,2-*d*][1,3]dioxolan-8b-yl)-2-oxoethyl dihydrogen phosphate **009-p**

Intermediate **009-p-1** (50.0 mg, 55.3 μmol) was dissolved in dichloromethane (3 mL), trifluoroacetic acid (630 mg, 5.53 mmol) was added, and the mixture was stirred at room temperature for reaction for 30 min. After the reaction was completed as detected by LCMS, the reaction mixture was blow-dried with nitrogen, and the resulting residue was separated and purified by preparative high performance liquid chromatography [column: Boston Prime C18 150 × 30 mm × 5 μm; mobile phase: A, 0.1% aqueous formic acid solution; B, acetonitrile, B%, 38%-58%,12 min) to give compound **009-p** (10.0 mg, 26.1%).

LC-MS: Rt: 2.752 min; MS m/z (ESI): 692.3 [M+H];
$^1$H NMR (400 MHz, DMSO-$d_6$) $\delta$ = 7.37 - 7.24 (m, 3H), 7.14 (d, *J* = 6.8 Hz, 2H), 6.27 (s, 2H), 6.16 (dd, *J* = 1.6, 10.0 Hz, 1H), 5.93 (s, 1H), 5.47 (s, 1H), 4.94 - 4.84 (m, 2H), 4.66 - 4.56 (m, 1H), 4.40 (t, *J* = 8.6 Hz, 2H), 4.28 (s, 1H), 3.68 (s, 2H), 3.00 (t, *J* = 8.7 Hz, 2H), 2.29 (d, *J* = 2.9 Hz, 1H), 2.16 - 1.94 (m, 3H), 1.83 - 1.60 (m, 5H), 1.39 (s, 3H), 1.04 - 0.83 (m, 5H).

**Example 15:** synthesis of phosphate ester compounds 001-p, 002-p, 003-p, 004-p, 005-p, 006-p, 007-p, 008-p, 010-p, 011-p, 012-p, and 013-p

Phosphate ester compounds 001-p, 002-p, 003-p, 004-p, 005-p, 006-p, 007-p, 008-p, 010-p, 011-p, 012-p, and 013-p can be prepared by referring to the phosphorylation procedure described in Example 14 and using compounds 001, 002, 003, 004, 005, 006, 007, 008, 010, 011, 012, 013, or N-Boc intermediates of the compounds described above (e.g., intermediate 1-6 in Example 1) as the starting material.

Compounds other than the compounds synthesized in Examples 1-15 were synthesized by referring to the synthetic route and source material in Examples 1-15.

**Biological Activity and Related Properties Test Example**

The compounds in the following test examples were prepared according to the methods in the above examples disclosed herein.

**Test Example 1. Activity of Compound of the Present Invention Determined by K562-GRE Reporter Gene**

To generate the parental K562-GRE cell line, K562 cells were seeded at $5 \times 10^5$ cells/well onto a 6-well plate (brand: Costar, Cat. No: 3516) containing 2 mL of complete medium (RPM1640, 10% FBS, penicillin-streptomycin) and cultured at 37 °C with 5% $CO_2$ for 24 h. The next day, 3 μg of pNL2.2[NLucP/MMTV/Hygro-NANO] (Promega) and 3 μL of PLUS reagent (brand: Invitrogen, Cat. No: 11514-015) were diluted in 150 μL of Opti-MEM (brand: Gibco, Cat. No: 11058021) and incubated at room temperature for 5 min. The pNL2.2[NLucP/MMTV/Hygro-NANO] vector contains MMTV LTR (mouse mammary tumor virus long terminal repeat), which drives transcription of the luciferase reporter gene NanoLuc in response to activation of several nuclear receptors (e.g. glucocorticoid receptor and androgen receptor). After incubation, the diluted DNA solution was mixed with Lipofectamine LTX solution (brand: Invitrogen, Cat. No: 15338-100) (6 μL of Lipofectamine LTX + 144 μL of Opti-MEM) in a 1:1 ratio, pre-incubated, and incubated at room temperature for 15 min to form a DNA-Lipofectamine LTX complex. After incubation, 300 μL of the DNA-Lipofectamine complex was added directly to the cell wells. The K562 cells were transfected at 37 °C with 5% $CO_2$ for 24 h. After transfection, the cells were washed with 3 mL of PBS and selectively grown for two weeks with complete growth medium containing 125 μg/mL hygromycin B (brand: Invitrogen, Cat. No: 10687010). "K562-GRE (pNL2.2[NLucP/MMTV/Hygro-NANO])" cells were generated.

The K562-GRE (pNL2.2[NLucP/MMTV/Hygro-NANO) cells were seeded at 50000 cells per well in 50 μL assay medium (RPMI 1640 medium, 1% FBS, 1% sodium pyruvate, 1% MEM non-essential amino acid and 1% penicillin-streptomycin) on a 96-well tissue culture treated white plate (brand: Costar, Cat. No: 3917), and the cells were treated with 50 μL (2×) of compounds in 5-fold serial dilution with the assay medium, each compound at a total of 8 concentrations with a final concentration of 0.0000128 μM-1 μM in the system. After mixing, the cells were cultured in an incubator at 37 °C for 24 h. After 24 h of incubation, an equal volume of 100 μL of Nano-Glo luciferase assay system (brand: Promega, Cat. No: N1120) was added to the cells, the mixture was reacted in a shaker at 500 g for 10 min, and the chemiluminescence values were detected using the PHERAstar instrument. The antibody binding curves were plotted using the logarithmic value of the antibody concentration as the abscissa and the corresponding chemiluminescence reading as the ordinate, and the $EC_{50}$ value was calculated by four-parameter fitting (GraphPad Prism9). The results are shown in Table 1.

**Test Example 2. Determination of Binding Activity of Compound of the Present Invention to Glucocorticoid Receptor GR**

The binding activity of the compound to GR was determined using the Polarscreen Glucocorticoid Receptor Assay Kit, Red (brand: Thermo, Cat. No: A15898). The test compound was diluted 10-fold with DMSO in a 96-well V-bottom plate (brand: Nunc, Cat. No: 249944). The highest concentration was 100 μM, 8 concentrations in total. The compound was then further diluted 50-fold with the detection buffer Complete GR Screening buffer provided in the kit, 10 μL of the diluted compound was transferred to a 384 microplate (brand: Corning, Cat. No: 4514), 5 μL of Fluormone GS Red (4× concentration) was added to the test compound, then a mixture of 5 μL of GR Full length (4× concentration) was added, and the experiment was performed in duplicate. The 384 microplate were incubated at room temperature in the dark for 2 h, and the fluorescence polarization (mP) was detected using the EnVision multifunctional microplate reader (manufacturer: Perkinelmer). With the log value of the final concentration of the compound as the X axis and the mP value as the Y axis, data was input into the processing software Graphpad Prism 9, and the $EC_{50}$ value was calculated by four-parameter fitting.

**Test Example 3. Determination of Binding Activity of Compound of the Present Invention to Estrogen Receptor ER**

The binding activity of the compound to ER was determined using the LanthaScreen® TR-FRET ER Alpha Coactivator Assay kit (brand: Thermo, Cat. No: A15885). The ER receptor agonist Estradiol (brand: Sigma, Cat. No: E8875-25) was diluted 10-fold with DMSO in a 96-well V-bottom plate. The highest concentration was 100 μM, 8 concentrations in total. The test compound was diluted 10-fold with DMSO. The highest concentration was 3000 μM, 8 concentrations in total. The compound was then further diluted 50-fold with the detection buffer Nuclear Receptor Buffer

E (containing 5 mM DTT) provided in the kit, 10 μL of the diluted compound was transferred to a 96-well half-area microplate (brand: Corning, Cat. No: 3694), 5 μL of ER-LBD protein (4× concentration) was added to the test compound, then a mixture of 5 μL of fluorescein-coactivator peptide and Tb-labeled anti-GST antibody (4× concentration) was added, and the experiment was performed in duplicate. The plate was incubated at room temperature for 2 h, the fluorescence values (Excitation 337, Emission 520/495 nm) were detected using the PHERAstar instrument, and the 520:495 ratio was calculated. With the log value of the final concentration of the compound as the X axis and the 520/495 ratio as the Y axis, data was input into the software Graphpad Prism 9, and the $EC_{50}$ value was calculated by four-parameter fitting. The results are shown in Table 1. The test compounds have relatively weak binding activity to estrogen receptors.

**Test Example 4. Determination of Binding Activity of Compound to Androgen Receptor AR**

The binding activity of the compound to AR was determined using the LanthaScreen® TR-FRET Androgen Receptor Coactivator Assay kit (brand: Thermo, Cat. No: A15878). The AR receptor agonist dihydrotestosterone (DHT) (brand: Sigma, Cat. No: D-073) was diluted 10-fold with DMSO in a 96-well V-bottom plate. The highest concentration was 100 μM, 8 concentrations in total. The test compound was diluted 10-fold with DMSO. The highest concentration was 3000 μM, 8 concentrations in total. The compound was further diluted 50-fold with the detection buffer Nuclear Receptor Buffer A (containing 5 mM DTT) provided in the kit, 10 μL of the diluted compound was transferred to a 96-well half-area microplate, 5 μL of AR-LBD protein (4× concentration) was added to the test compound, then a mixture of 5 μL of fluorescein-coactivator peptide and Tb-labeled anti-GST antibody (4× concentration) was added, and the experiment was performed in duplicate. The plate was incubated at room temperature for 2 h, the fluorescence values (Excitation 337, Emission 520/495 nm) were detected using the PHERAstar instrument, and the 520:495 ratio was calculated. With the log value of the final concentration of the compound as the X axis and the 520/495 ratio as the Y axis, data was input into the processing software Graphpad Prism 9, and the $EC_{50}$ value was calculated by four-parameter fitting. The results are shown in Table 1. The test compounds have relatively weak binding activity to androgen receptors.

**Test Example 5. Determination of Binding Activity of Compound to Progesterone Receptor PR**

The binding activity of the compound to PR was determined using the LanthaScreen® TR-FRET Progesterone Receptor Coactivator Assay kit (brand: Thermo, Cat. No: A15903). The PR receptor agonist Progesterone (brand: Sigma, Cat. No: P0130) was diluted 10-fold with DMSO in a 96-well V-bottom plate. The highest concentration was 100 μM, 8 concentrations in total. The test compound was diluted 10-fold with DMSO. The highest concentration was 3000 μM, 8 concentrations in total. The compound was then further diluted 50-fold with the detection buffer Nuclear Receptor Buffer F (containing 5 mM DTT) provided in the kit, 10 μL of the diluted compound was transferred to a 96-well half-area microplate, 5 μL of PR-LBD protein (4× concentration) was added to the test compound, then a mixture of 5 μL of fluorescein-coactivator peptide and Tb-labeled anti-GST antibody (4× concentration) was added, and the experiment was performed in duplicate. The plate was incubated at room temperature for 2 h, the fluorescence values (Excitation 337, Emission 520/495 nm) were detected using the PHERAstar instrument, and the 520:495 ratio was calculated. With the log value of the final concentration of the compound as the X axis and the 520/495 ratio as the Y axis, data was input into the processing software Graphpad Prism 9, and the $EC_{50}$ value was calculated by four-parameter fitting. The results are shown in Table 1. The test compounds have a certain binding activity to progesterone receptors.

**Table 1. Summary of activity *in vitro***

| Compound No. | K562-GRE Reporter $EC_{50}$ (μM) | GR binding $EC_{50}$ (μM) | PR binding $EC_{50}$ (μM) | ER binding $EC_{50}$ (μM) | AR binding $EC_{50}$ (μM) |
|---|---|---|---|---|---|
| 001 | 0.0035 | 0.01304 | 0.1911 | >30 | >30 |
| 002 | 0.0090 | N/A | N/A | N/A | N/A |
| 003 | 0.0038 | 0.1207 | 0.4899 | >30 | >30 |
| 004 | 0.0075 | 0.04252 | N/A | N/A | N/A |
| 005 | 0.0056 | N/A | N/A | N/A | N/A |
| 006 | 0.0017 | 0.0423 | 0.1111 | >30 | >30 |
| 007 | 0.0021 | 0.01712 | N/A | N/A | N/A |
| 008 | 0.00781 | N/A | N/A | N/A | N/A |

(continued)

| Compound No. | K562-GRE Reporter $EC_{50}$ ($\mu$M) | GR binding $EC_{50}$ ($\mu$M) | PR binding $EC_{50}$ ($\mu$M) | ER binding $EC_{50}$ ($\mu$M) | AR binding $EC_{50}$ ($\mu$M) |
|---|---|---|---|---|---|
| 009 | 0.00576 | 0.02024 | 0.427 | >30 | >30 |
| 010 | 0.00107 | 0.01643 | 0.052 | >30 | >30 |
| 011 | 0.00425 | N/A | N/A | N/A | N/A |
| 012 | 0.00958 | N/A | N/A | N/A | N/A |
| N/A represents untested. | | | | | |

The test results show that the test compounds have strong GR binding activity to GR, weak binding activity to PR, and no ER and AR binding, and are expected to reduce the side effects associated with acting on other hormone receptors.

**Test Example 6: Efficacy Test in CHS Mouse Model**

Construction of CHS mouse model: 6- to 8-week-old female C57BL/6N mice (Beijing Vital River Laboratory Animal Technology Co., Ltd.) were selected, and all mice were shaved off abdominal hair using a small animal hair clipper. 400 $\mu$L of the sensitizer was pipetted with a micropipette and applied evenly to the abdomen of the mice for epidermal sensitization. After application, the mice were held for 3-5 s and the solvent was allowed to evaporate from the skin as clean as possible. The specific preparation method for the sensitizer was: 0.5 g of FITC (fluorescein isothiocyanate; sigma-Aldrich) powder was weighed and fully dissolved in a solvent of 50 mL of acetone (SinoPharm reagent) and 50 mL of DBP (dibutyl phthalate; sigma-Aldrich) in equal proportion to give the sensitizer with the FITC content of 0.5%. On the 6th day after sensitization, the thickness of the right ear of the mice was measured with a dial thickness gauge as a baseline value, then a freshly prepared sensitizer was pipetted with a micropipette and applied evenly inside and outside of the right ear of the mice respectively for epidermal stimulation, and 10 $\mu$L of the sensitizer was applied on each side. 24 h after stimulation, the thickness of the right ear of the mice was measured again with a dial thickness gauge to obtain the thickness change of the right ear of the mice, $\Delta$ thickness of right ear = (thickness of right ear 24 h after stimulation) - (baseline value of thickness of right ear).

Therapeutic regimen for CHS model mouse: mice were divided into a blank group, a negative control group, a positive control group, and an experimental group. The experimental group was given compound **001** and compound **009** at a dose of 3 $\mu$g/mouse, the positive control group was given compound **a** (synthesized according to the method reported in patent WO2017210471 for compound **41)** at a dose of 3 $\mu$g/mouse, and the negative control group was given blank vehicle (0.5% DMSO/PBS). The drug was administered by one-time intraperitoneal injection 1 h before the right ear epidermal stimulation.

The results are shown in FIG. 2. After administration of compounds **001** and **009,** there is a significant decrease in the thickness of the right ear in CHS mice compared to the negative control group; both compounds **001** and **009** are superior in efficacy to the positive reference compound a at the same dose. (* indicates the significance of the statistical difference between each experimental group and the negative control group by one-way ANOVA, ** indicates P value < 0.01, and **** indicates P value < 0.0001).

**Test Example 7. Determination of Metabolic Stability of Compound of the Present Invention in Liver Microsomes**

The metabolic stability of the compound of the present invention in liver microsomes was determined using the following method.

I. Experimental materials and instruments

1. Human liver microsome (Corning 452117), beagle dog liver microsome (XENOTECH D1000), SD rat liver microsome (XENOTECH R1000), and CD-1 mouse liver microsome (XENOTECH M1000)
2. $Na_2HPO_4$ (Tianjin Guangfu Fine Chemical Research Institute 20180130)
3. $KH_2PO_4$ (Tianjin Guangfu Fine Chemical Research Institute 20180920)
4. $MgCl_2$ (Tianjin Guangfu Fine Chemical Research Institute 20191216)

5. NADPH (Solarbio 1216C022)
6. Positive control compound verapamil (Sigma MKBV4993V)
7. AB Sciex API4000 liquid chromatography-mass spectrometry system

II. Procedures

1. Preparation of 100 mM phosphate-buffered saline (PBS): 7.098 g $Na_2HPO_4$ was weighed. 500 mL pure water was added. The mixture was dissolved by sonication to give solution A. 3.400 g $KH_2PO_4$ was weighed. 250 mL pure water was added. The mixture was dissolved by sonication to give solution B. The solution A was placed in a stirrer, and the solution B was slowly added until the pH reached 7.4, so that the 100 mM PBS buffer was prepared.
2. Preparation of reaction system
The reaction system was prepared as follows:

| Reagent | Stock solution concentration | Volume | Final concentration |
|---|---|---|---|
| Liver microsome | 20 mg/mL | 10 μL | 0.5 mg/mL |
| Phosphate-buffered saline | 100 mM | 346 μL | 100 mM |

3. The reaction system was pre-incubated in a 37 °C water bath for 10 min. 40 μL of 10 mM NADPH solution (NADPH was dissolved by 100 mM phosphate-buffered saline) was added to the reaction system with a final concentration of NADPH being 1 mM. A negative control was made by replacing the NADPH solution with 40 μL phosphate-buffered saline. The negative control was used to exclude the effect of chemical stability of the compound itself.
4. The reaction was initiated by adding 4 μL of 100 μM of the compound of the present invention and verapamil, the positive control compound, to the reaction system with a final concentration of the compound being 1 μM.
5. After being uniformly mixed in a vortex oscillator, 50 μL incubation sample was respectively taken out at 0.5, 15, 30, 45, and 60 min, and the reaction was stopped by 200 μL glacial acetonitrile containing an internal standard. The sample was centrifuged at 3220 g for 45 min. 90 μL of the supernatant was transferred to a feeding plate after the centrifugation was completed, and 90 μL ultrapure water was added. The mixture was well mixed for LC-MS/MS analysis.

All data were calculated by Microsoft Excel software. The peak area was detected through an extracted ion chromatogram. The *in vitro* half-life ($t_{1/2}$) of the compound was determined by linear fitting of the natural logarithm of compound elimination percentage over time.

*In vitro* half-life ($t_{1/2}$) was calculated by the slope *k*:
*in vitro* $t_{1/2} = 0.693 / k$

The *in vitro* intrinsic clearance (in μL/min/mg protein) was calculated using the following formula: *in vitro* $CL_{int} = k \times$ volume of incubation (μL)/amount of proteins (mg)
$CL_{int}$ was intrinsic clearance; *k* was an elimination rate constant; volume of incubation was the incubation volume (μL); amount of proteins was the amount of protein (mg)

The corresponding liver microsome stability of the compound of the present invention is shown in Table 2.

**Table 2**

| Compound | Human liver microsome stability $t_{1/2}$ (min) | Mouse liver microsome stability $t_{1/2}$ (min) |
|---|---|---|
| Compound 009 | 37.8 | 17.3 |

**Test Example 8. Determination of Membrane Permeability and Transport Properties of Compound of the Present Invention**

The membrane permeability and transport properties of the compound of the present invention were determined using the following method.

I. Experimental materials and instruments

1. Caco-2 cells (ATCC)
2. HEPES (Solarbio 804D049), penicillin/streptomycin (Solarbio 20200109), and PBS (Solarbio 20200620)

3. Fetal Bovine Serum (FBS) (Sigma WXBD0055V), lucifer yellow (Sigma MKCJ3738), and $NaHCO_3$ (Sigma SLBZ4647)
4. Hank's balanced salt solution (HBSS) (Gibco 2085528), non-essential amino acid (NEAA) (Gibco 2211548), and Trypsin/EDTA (Gibco 2120732)
5. High glucose DMEM (Corning 20319014)
6. HTS Transwell-96 Well Permeable (Corning, 3391)
7. Resistance detector (Millipore, Millicell® ERS-2)
8. Cellometer® Vision (Nexcelom Bioscience)
9. Infinite 200 PRO microplate reader (Tecan, Infinite M200PRO)
10. Positive control compound metoprolol (Sinopharm 100084-201403), erythromycin (MCE 84550), and cimetidine (Sinopharm 100158-201406)
11. ABI QTrap 5500 liquid chromatography-mass spectrometry system

II. Procedures

1. Caco-2 cell culture

1) Preparation of transport buffer (HBSS containing 25 mM HEPES, pH 7.4): 5.958 g HEPES and 0.35 g $NaHCO_3$ were accurately weighed and dissolved in 900 mL pure water. 100 mL of 10 × HBSS was then added and stirred well. The pH was adjusted to 7.4, and the mixture was filtered.
2) Preparation of Caco-2 cell culture medium: FBS, penicillin/streptomycin, kanamycin, and NEAA were added to a high glucose DMEM (containing L-glutamine) culture medium to prepare a cell culture medium containing 10% FBS, 100 units penicillin/0.1 mg/mL streptomycin, 0.6 μg/mL kanamycin, and 1 × NEAA.
3) Cells were cultured with a T-75 flask at 37 °C with 5% $CO_2$ in an incubator. The culture medium was discarded when cell density reached 80-90%. The cells were rinsed with 5 mL PBS. 1.5 mL Trypsin/EDTA was added. The mixture was incubated at 37 °C for 5-10 min in an incubator until the cells shed like quicksand. Finally the Trypsin/EDTA was neutralized with a FBS-containing culture medium.
4) The cell suspension was centrifuged at 120 g for 10 min, and the supernatant was discarded.
5) The cells were resuspended in a cell culture medium. The cell suspension was adjusted to a density of $6.86 \times 10^5$ cells/mL.

2. Caco-2 cell inoculation

1) 50 μL of culture medium was added to the Transwell chamber per well. 25 mL of culture medium was added to the lower chamber, and the chamber was preheated at 37 °C for 1 h with 5% $CO_2$ in an incubator.
2) 50 μL of the cell suspension was added to the preheated Transwell chamber per well at a final density of $2.4 \times 10^5$ cells/$cm^2$ (cells/mL).
3) The cells were cultured for 14-18 days. The culture medium was replaced every other day, and the culture medium was replaced within 48 h after the initial inoculation. The culture medium had to be replaced the day before the experiment.

3. Evaluation of integrity of monolayer cell membrane

1) The cells fused and differentiated after 14 days of culture and were ready for a transport experiment.
2) The resistance of the monolayer membrane was measured using a resistance detector. The resistance of each well was recorded.
3) After measurement, the Transwell culture plate was re-incubated.
4) The TEER value was calculated:

$$\text{TEER value} = \text{TEER measurement value } (\Omega) \times \text{membrane area } (\text{cm}^2)$$

The resistance of the monolayer cell membrane < 230 Ω $cm^2$ indicates that a monolayer cell membrane is poorly dense and can not be used for experiment.

4. Transport experiment

1) A 10 mM stock solution of the compound of the present invention or the positive control compound was diluted with DMSO to give a 2 mM stock solution. The 2 mM stock solution was then diluted with the transport buffer to give a 10 μM working solution of the compound of the present invention or the positive control compound.
2) The Caco-2 cell plate was taken out from the incubator, washed twice with the pre-heated transport buffer, and incubated at 37 °C in an incubator for 30 min.
3) To determine the transport rate of the compound from the apical end to the basal end (A -> B), 108 μL of the working solution of the compound was added to the Transwell chamber (apical end), while 8 μL of the sample was immediately taken from the apical end to 72 μL of the transport buffer. 240 μL of a stop solution containing an internal standard was added to stop the transport to be an initial apical end sample. At the same time, 300 μL of the transport buffer was added to the receiving end (basal end). The sample in the experiment was in duplicate.
4) To determine the transport rate of the compound from the basal end to the apical end (B -> A), 308 μL of the working solution of the compound was added to the basal end, while 8 μL of the sample was immediately taken from the basal end to 72 μL of the transport buffer. 240 μL of a stop solution containing an internal standard was added to stop the transport to be an initial basal end sample. At the same time, 100 μL of the transport buffer was added to the Transwell chamber (apical end). The sample in the experiment was in duplicate.
5) The cell culture plate was incubated at 37 °C with $CO_2$ in an incubator for 2 h.
6) After the transport experiment was completed, 8 μL of the sample was taken from the administration end (i.e., the apical end in the A → B direction and the basal end in the B → A direction) to 72 μL of the transport buffer. 240 μL of a stop solution containing an internal standard was then added to stop the transport. 80 μL of the sample was taken from the receiving end (i.e., the basal end in the A → B direction and the apical end in the B → A direction) to 240 μL of a stop solution containing an internal standard. The mixture was vortexed at 1000 rpm for 10 min and centrifuged at 3220 g for 30 min. 100 μL of the supernatant was transferred to a feeding plate, and 100 μL of ultrapure water was added. The mixture was well mixed for LC-MS/MS analysis.
7) Fluorescence values were measured after the transport experiment was completed. A 10 mM lucifer yellow stock solution was prepared with water and then diluted to 100 μM with the transport buffer. 100 μL of the lucifer yellow solution was added to the Transwell chamber (apical end). 300 μL of the transport buffer was added to the basal end. The chamber was incubated at 37 °C with $CO_2$ in an incubator for 30 min. 80 μL solution was taken from the apical end and the basal end to a 96-well plate, and the fluorescence value of the cells was measured by a microplate reader at an excitation wavelength of 485 nm and an emission wavelength of 530 nm (to detect integrity of the membrane). The leakage rate (percent leakage (%) or LY (%)) was calculated by the following formula:

$$\text{Percentage Leakage} = \{I_{\text{acceptor}}\times 0.3/(I_{\text{acceptor}}\times 0.3 + I_{\text{donor}}\times 0.1)\} \times 100\%$$

$I_{\text{acceptor}}$ referred to the fluorescence density of the receiving end (0.3 mL), and $I_{\text{donor}}$ referred to the fluorescence density of the administration end (0.1 mL). LY > 1.0% indicates that a monolayer cell membrane is poorly dense and the corresponding results will be excluded from the evaluation.

The peak areas of the compound on the administration end and the receiving end were measured, and the apparent permeability coefficient ($P_{\text{app}}$, in cm/s) and the Efflux ratio (efflux ratio) of the compound were calculated:

$$P_{\text{app}} = \{V_A\times[drug]_{acceptor}/(Area\times incubation\ time\times[drug]_{initial\ donor}\}$$

$V_A$ was the volume of the receiving end solution (A → B was 0.3 mL, and B → A was 0.1 mL). Area was the area of the membrane of the Transwell 96-well plate (0.143 $cm^2$). Incubation time was the time for incubation (in s). $[drug]_{acceptor}$ was the concentration of the drug at the receiving end. $[drug]_{initial\ donor}$ was the initial concentration of the drug at the administration end.

$$Efflux\ Ratio = \frac{P_{app(B\text{-}A)}}{P_{app(A\text{-}B)}}$$

$P_{app\ (B\text{-}A)}$ represented the apparent permeability coefficient from the basal end to the apical end; $P_{app\ (A\text{-}B)}$ represented the apparent permeability coefficient from the apical end to the basal end.

**Table 3**

| Compound | $P_{app}$(A-B) ($10^{-6}$ cm/s) | $P_{app}$(B-A) ($10^{-6}$ cm/s) | Efflux Ratio |
|---|---|---|---|
| Compound 009 | 0.73 | 3.86 | 5.26 |

**Test Example 9. Determination of Plasma Protein Binding Rate of Compound of the Present Invention**

The protein binding rates of the compound of the present invention in plasma of 5 species (humans, monkeys, dogs, rats, and mice) were determined using the following method.

I. Experimental materials and instruments

1. Human plasma (BioIVT), monkey plasma (ADME-plasma-pooled monkey-05212020), beagle dog plasma (Bio-IVT), SD rat plasma (BioIVT), and CD-1 mouse plasma (BioIVT)
2. $Na_2HPO_4$ (Sigma S5136-500G)
3. $NaH_2PO_4$ (Sigma S3139-500G)
4. NaCl (Sigma S5886-IKG)
5. 96-well equilibrium dialysis plate (HTDialysis LLC, Gales Ferry, CT, HTD96B) and equilibrium dialysis membrane (MWCO 12-14K, 1101)
6. Positive control compound warfarin
7. ABI QTrap 5500 liquid chromatography-mass spectrometry system

II. Procedures

1. Preparation of a buffer with a concentration of 100 mM sodium phosphate and 150 mM NaCl: an alkaline solution with a concentration of 14.2 g/L $Na_2HPO_4$ and 8.77 g/L NaCl was prepared with ultrapure water, an acidic solution with a concentration of 12.0 g/L $NaH_2PO_4$ and 8.77 g/L NaCl was prepared with ultrapure water, and then the alkaline solution was titrated with the acidic solution until the pH was 7.4 to prepare a buffer with a concentration of 100 mM sodium phosphate and 150 mM NaCl.
2. Preparation of a dialysis membrane: the dialysis membrane was soaked in ultrapure water for 60 min to separate the membrane into two pieces, then soaked with 20% ethanol for 20 min, and finally soaked with dialysis buffer for 20 min.
3. Preparation of plasma: the frozen plasma was quickly thawed at room temperature and then centrifuged at 3,220 g at 4 °C for 10 min to remove clots, and the supernatant was collected to a new centrifuge tube. The pH of the plasma was measured and recorded. The plasma with pH 7-8 was used.
4. Preparation of a plasma sample containing the compound: A 10 mM stock solution of the compound of the present invention or the positive control compound was diluted with DMSO to give a 200 μM working solution. 3 μL of 200 μM of the compound working solution was added to 597 μL of human, monkey, dog, rat, or mouse plasma to give a plasma sample with a final concentration of 1 μM.
5. Equilibrium dialysis procedures: a dialysis device was assembled according to the instructions. 120 μL of the plasma sample containing 1 μM of the compound was added to one side of the dialysis membrane, and a dialysate (phosphate buffer) in the same volume was added to the other side. The sample in the experiment was in duplicate. The dialysis plate was sealed with a film, placed in an incubation device, and incubated at approximately 100 rpm

at 37 °C with 5% $CO_2$ for 6 h. After the incubation was completed, the film was removed, and 50 μL of the sample was pipetted from the buffer side and the plasma side of each well to different wells of a new plate. 50 μL of blank plasma was added to the phosphate buffer sample. A blank phosphate buffer in the same volume was added to the plasma sample, and then 300 μL of acetonitrile containing an internal standard was added to precipitate the protein. The mixture was vortexed for 5 min and centrifuged at 3220 g at 4 °C for 30 min. 100 μL of the supernatant was transferred to a feeding plate, and 100 μL of ultrapure water was added. The mixture was well mixed for LC-MS/MS analysis.

The peak areas of the compound on the buffer side and the plasma side were measured. The plasma protein binding rate of the compound was calculated by the following formula:

%Free rate = (ratio of compound peak are to internal standard peak area$_{\text{buffer side}}$/ratio of compound peak area to internal standard peak$_{\text{plasma side}}$ × 100%)

$$\%\text{Binding rate} = 100\% - \%\text{Free rate}$$

**Table 4**

| Compound | Human plasma protein binding rate% | Mouse plasma protein binding rate% |
|---|---|---|
| Compound 009 | 99.8 | 99.8 |

**Test Example 10. Inhibitory Effect of Compound of the Present Invention on Enzymatic Activity of CYP2C9 and CYP2D6**

The inhibition of CYP2C9 and CYP2D6 activity by the compound of the present invention was determined using the following method.

I. Experimental materials and instruments

1. Human liver microsome (Corning 452117)
2. NADPH (Solarbio 705Y021)
3. Positive substrates diclofenac (Sigma SLBV3438) and dextromethorphan (TRC 3-EDO-175-1)
4. Positive inhibitors sulfaphenazole (D. Ehrenstorfer GmbH 109012) and quinidine (TCI WEODL-RE)
5. AB Sciex Triple Quad 5500 liquid chromatography-mass spectrometry system

II. Procedures

1. Preparation of 100 mM phosphate-buffered saline (PBS): 7.098 g $Na_2HPO_4$ was weighed. 500 mL pure water was added. The mixture was dissolved by sonication to give solution A. 3.400 g $KH_2PO_4$ was weighed. 250 mL pure water was added. The mixture was dissolved by sonication to give solution B. The solution A was placed in a stirrer, and the solution B was slowly added until the pH reached 7.4, so that the 100 mM PBS buffer was prepared.
2. A 10 mM NADPH solution was prepared with a 100 mM PBS buffer. A 10 mM stock solution of the compound of the present invention was diluted with DMSO to give a compound working solution at a concentration of 200 × (6000, 2000, 600, 200, 60, 20, and 0 μM). The positive inhibitor stock solution was diluted with DMSO to give a positive inhibitor working solution at a concentration of 200 × (sulfaphenazole, 1000, 300, 100, 30, 10, 3, and 0 μM; quinidine, 100, 30, 10, 3, 1, 0.3, and 0 μM). Substrate working solutions (120 μM diclofenac and 400 μM dextromethorphan) at a concentration of 200 × were prepared with water, acetonitrile, or acetonitrile/methanol.
3. 2 μL of 20 mg/mL liver microsome solution, 1 μL of substrate working solution, 1 μL of compound working solution, and 176 μL of PBS buffer were taken, mixed well, and placed in a 37 °C water bath for pre-incubation for 15 min.

1 μL of sulfaphenazole or quinidine working solution was added to the positive control group instead of the compound working solution. At the same time, 10 mM NADPH solution was placed together in the 37 °C water bath for pre-incubation for 15 min. After 15 min, 20 μL of NADPH was added to each well to initiate the reaction. The mixture was incubated at 37 °C for 5 min (CYP2C9) or 20 min (CYP2D6). All incubated samples were in duplicate. After incubation for the corresponding period of time, 400 μL of icy methanol containing internal standard was added to all samples to stop the reaction. The mixture was vortexed, mixed well, and centrifuged for 40 min at 4 °C at 3220 g. 100 μL of the supernatant was transferred to a feeding plate after the centrifugation was completed, and 100 μL ultrapure water was added. The mixture was well mixed for LC-MS/MS analysis.

The reduction of metabolite production in the treatment groups versus the control group was compared by the peak area ratios of the sample to the internal standard, and the $IC_{50}$ value was calculated using Excel XLfit 5.3.1.3.
Percentages of remaining activity were calculated according to the following formula:

Remaining activity% = peak area ratio of metabolite to internal standard$_{\text{test sample}}$/peak area ratio of metabolite to internal standard$_{\text{blank solvent}}$ × 100%.

Drug-drug interaction (DDI) refers to the physical or chemical changes caused by no less than 2 drugs, as well as changes in efficacy due to such changes. Interpretation of the drug-drug interaction can provide better pharmaceutical services for patients, promote reasonable medication, and avoid adverse reactions to the maximum extent. The drug-drug interaction is dominated by metabolic interactions which are primarily associated with CYP450 enzymes involved in drug metabolism. The results in Table 5 show that the compound of the present invention has a weak inhibitory effect on CYP450, indicating that the compound of the present invention has a lower potential risk of developing DDI.

**Table 5**

| Compound | CYP2C9 inhibition $IC_{50}$ (μM) | CYP2D6 inhibition $IC_{50}$ (μM) |
|---|---|---|
| Compound 009 | 7.2 | >50 |

**Test Example 11. Assay for hERG Inhibitory Activity of Compound of the Present Invention**

The inhibition of hERG activity by the compound of the present invention was determined using the following method.

I. Experimental materials and instruments

1. Dialyzed fetal bovine serum Shanghai Bohan Biotechnology Co., Ltd (BS-0005-500)
2. DMEM culture medium Thermo Fisher Scientific (China) Co., Ltd. (10569)
3. HEPES Thermo Fisher Scientific (China) Co., Ltd. (15630080)
4. Trypsin Thermo Fisher Scientific (China) Co., Ltd. (2192509)
5. Penicillin-streptomycin solution Thermo Fisher Scientific (China) Co., Ltd. (15140-122)
6. MEM non-essential amino acid solution Thermo Fisher Scientific (China) Co., Ltd. (11140)
7. Geneticin (G418) Thermo Fisher Scientific (China) Co., Ltd. (11811031)
8. Blasticidin Thermo Fisher Scientific (China) Co., Ltd. (R21001)
9. PolylysineThermo Fisher Scientific (China) Co., Ltd. (P4832)
10. Dofetilide Beijing Express Technology Co., Ltd. (D525700)
11. Doxycycline Sigma-Aldrich (Shanghai) Trading Co., Ltd. (D9891)
12. Carbon dioxide incubator Thermo Fisher Scientific (China) Co., Ltd. (371)
13. Puller Sutter Company, U.S. (P-97)
14. Micromanipulator Siskiyou Company, U.S. (MC1000e)
15. Micromanipulator Sutter Company, U.S. (ROE-200; MP285)
16. Amplifier HEKA Company, Germany (EPC10)
17. Microscope Olympus (China) Co., Ltd. (IX51/71/73)
18. Perfusion system ALA Company, U.S. (VM8 gravity administration system)

II. Culture of cell lines and cells

HEK293 cells (Cat. No: K1236) stably expressing hERG ion channel were purchased from Invitrogen. The cells were cultured in a culture medium containing 85% DMEM, 10% dialyzed fetal bovine serum, 0.1 mM non-essential amino acid solution, 100 U/mL penicillin-streptomycin solution, 25 mM HEPES, 5 μg/mL blasticidin, and 400 μg/mL geneticin. When the cell density increased to 40-80% of the bottom area of the culture dish, the cells were digested with trypsin and subcultured thrice every week. Before the experiment, the cells were cultured at a density of $5 \times 10^5$ in 6-cm culture dishes, induced for 48 h with 1 μg/mL doxycycline, and then digested and inoculated on a slide for the subsequent manual patch-clamp experiment.

III. Preparation of solutions

1) Extracellular fluid: 132 mM sodium chloride, 4 mM potassium chloride, 3 mM calcium chloride, 0.5 mM magnesium chloride, 11.1 mM glucose, and 10 mM HEPES (adjusted to pH 7.35 with sodium hydroxide).
2) Intracellular fluid: 140 mM potassium chloride, 2 mM magnesium chloride, 10 mM EGTA, 5 mM ATP-magnesium salt, and 10 mM HEPES (adjusted to pH 7.35 with potassium hydroxide).

Note: the osmotic pressure of the solutions was controlled at 280-300 mOsmol/kg. The solutions were filtered and stored at 4 °C before use. The ATP-magnesium salt was prepared into a 100 mM stock solution, subpackaged, and stored in a -20 °C refrigerator. On the day of the experiment, a certain amount of the stock solution was added into the intracellular fluid right before use.

IV. Procedures

1) The slides carrying HEK293 cells in the culture dish were placed in the perfusion chamber of the micromanipulation stage.
2) Appropriate cells were placed in the center of the field of view under an Olympus IX51, IX71, or IX73 inverted microscope. The tip of the glass electrode was found using a 10× objective lens and placed in the center of the field of view. The electrode was then moved down using a micromanipulator while the electrode was slowly brought into proximity to the cell using the coarse adjustment.
3) When the electrode was in close proximity to the cell, the lens was switched to a 40× objective lens for observation. The micromanipulator was used for fine adjustment to make the electrode approach the surface of the cell gradually.
4) Negative pressure was applied to form a seal with a resistance higher than 1 GΩ between the electrode tip and the cell membrane.
5) The instantaneous capacitance current Cfast was compensated under the voltage clamp mode. Then negative pressure was repeatedly applied to the membrane for rupture, and finally, a whole-cell recording mode was formed.
6) The slow capacitive current Cslow, the cell membrane capacitance (Cm), and the input membrane resistance (Ra) were compensated under a membrane potential clamp at -60 mV.
7) After the cells were stabilized, the clamp voltage was changed to -90 mV, the sampling frequency was set at 20 kHz, and the filtration frequency was 10 kHz. The detection condition of the drain current was that the clamp voltage was changed to -80 mV, and the time course was 500 ms.
8) The hERG current test method was as follows: a 4.8-second depolarization command voltage was applied to depolarize the membrane potential from -80 mV to +30 mV, and then a 5.2-second repolarization voltage was instantaneously applied to reduce the membrane potential to -50 mV to remove channel inactivation, thus allowing the observation of the hERG tail current. The peak value of the tail current was the magnitude of the hERG current.
9) The hERG current used to detect the test compound was recorded continuously for 120 seconds prior to dosing to assess the stability of hERG current production by the test cells. Only stable cells within the acceptance range of the evaluation criteria were used in the subsequent compound detection.

Assay of the test compound for inhibition of hERG current: The hERG current measured in the extracellular fluid containing 0.1% DMSO was used as the baseline of the assay. The solution containing the test compound was perfused around the cells sequentially in an ascending order of concentration after the hERG current remained stable for at least 5 minutes. After the completion of each perfusion, a suspension of about 5 minutes was set to allow the compound to act sufficiently on the cells and the hERG current was recorded simultaneously. The last 5 hERG current

values were recorded after the recorded current tended to stabilize, and the average value thereof was taken as the final current value at the specific concentration. After the compound was tested, 150 nM dofetilide was added to the same cell to completely inhibit the current as a positive control for the cell. Meanwhile, the positive compound dofetilide was synchronously detected by the same patch-clamp system before and after the test compound experiment was finished, so that the reliability and the sensitivity of the whole detection system were ensured.

V Data analysis

The data was output by PatchMaster software and analyzed as follows:

1) After a blank solvent or a compound gradient solution was perfused, 5 continuous current values were obtained after stabilization, and the average values were calculated and were respectively used as the tail current magnitude$_{blank}$ and the tail current magnitude$_{compound}$.
2) The current inhibition percentages were calculated by the following formula.

$$\text{Tail current inhibition rate} = \left(1 - \frac{\text{tail current magnitude}_{\text{compound}} - \text{tail current magnitude}_{\text{positive drug}}}{\text{tail current magnitude}_{\text{blank}} - \text{tail current magnitude}_{\text{positive drug}}}\right) \times 100$$

3) The dose-response curve was fitted by Graphpad Prism 8.0 software and the $IC_{50}$ value was calculated.

The compound of the present invention has no obvious inhibitory effect on the hERG potassium channel, which suggests that the compound has a low risk of cardiotoxicity due to the inhibition of hERG potassium channel. The hERG potassium channel inhibitory activity of the test compounds is shown in Table 6.

**Table 6**

| Compound | $IC_{50}$ |
|---|---|
| Compound 009 | > 30 μM |

## Claims

1. A compound of formula (I) or a pharmaceutically acceptable salt thereof:

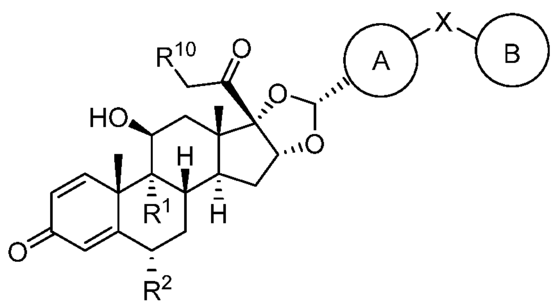

(I)

wherein

$R^1$ and $R^2$ are each independently selected from H, $CH_3$ or halogen;
ring A is selected from phenyl, 5-10 membered heteroaryl, or $C_3$-$C_{10}$ cycloalkyl, wherein the phenyl, 5-10 membered heteroaryl, or $C_3$-$C_{10}$ cycloalkyl is optionally substituted with $R^{1a}$;
X is selected from O, S, $C_1$-$C_3$ alkylene-O, $C_1$-$C_3$ alkylene-S, $NR^6$, or $C(R^7)(R^8)$;
$R^6$ is selected from H, $C_1$-$C_6$ alkyl, $C_3$-$C_6$ cycloalkyl, or 4-7 membered heterocyclyl, wherein the $C_1$-$C_6$ alkyl, $C_3$-$C_6$ cycloalkyl, or 4-7 membered heterocyclyl is optionally substituted with $R^b$;
$R^7$ and $R^8$ are each independently selected from H, halogen, CN, OH, $NH_2$, $C_1$-$C_6$ alkyl, $C_3$-$C_6$ cycloalkyl, or

4-7 membered heterocyclyl, or $R^7$ and $R^8$, together with the atom linked thereto, form $C_3$-$C_6$ cycloalkyl or 4-7 membered heterocyclyl, wherein the OH, $NH_2$, $C_1$-$C_6$ alkyl, $C_3$-$C_6$ cycloalkyl, or 4-7 membered heterocyclyl is optionally substituted with $R^b$;

ring B is selected from $C_6$-$C_{10}$ aryl, 5-10 membered heteroaryl, $C_3$-$C_{10}$ cycloalkyl, or 4-14 membered heterocyclyl, wherein the $C_6$-$C_{10}$ aryl, 5-10 membered heteroaryl, $C_3$-$C_{10}$ cycloalkyl, or 4-14 membered heterocyclyl is optionally substituted with $R^{2a}$;

$R^{10}$ is selected from OH, SH, O($C_1$-$C_6$ alkyl), S($C_1$-$C_6$ alkyl), O-C(=O)-($C_1$-$C_6$ alkyl), or

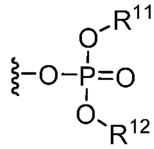
,

wherein the O($C_1$-$C_6$ alkyl), S($C_1$-$C_6$ alkyl), or O-C(=O)-($C_1$-$C_6$ alkyl) is optionally substituted with halogen or CN;

$R^{11}$ and $R^{12}$ are each independently selected from H or $C_1$-$C_6$ alkyl;

$R^{1a}$ and $R^{2a}$ are each independently selected from halogen, CN, =O, OH, $NH_2$, $C_1$-$C_6$ alkyl, $C_3$-$C_6$ cycloalkyl, or 4-7 membered heterocyclyl, wherein the OH, $NH_2$, $C_1$-$C_6$ alkyl, $C_3$-$C_6$ cycloalkyl, or 4-7 membered heterocyclyl is optionally substituted with $R^b$;

each $R^b$ is independently selected from halogen, CN, =O, OH, $NH_2$, $C_1$-$C_6$ alkyl, $C_3$-$C_6$ cycloalkyl, or 4-7 membered heterocyclyl, wherein the OH, $NH_2$, $C_1$-$C_6$ alkyl, $C_3$-$C_6$ cycloalkyl, or 4-7 membered heterocyclyl is optionally substituted with $R^c$;

each $R^c$ is independently selected from halogen, CN, =O, $C_1$-$C_3$ alkyl, OH, O($C_1$-$C_3$ alkyl), $NH_2$, NH($C_1$-$C_3$ alkyl), or N($C_1$-$C_3$ alkyl)$_2$.

**2.** The compound of formula (I) or the pharmaceutically acceptable salt thereof according to claim 1, wherein $R^1$ and $R^2$ are each independently selected from H or F.

**3.** The compound of formula (I) or the pharmaceutically acceptable salt thereof according to claim 1 or 2, wherein

ring A is selected from phenyl or 5-6 membered heteroaryl, wherein the phenyl or 5-6 membered heteroaryl is optionally substituted with $R^{1a}$; or

ring A is selected from phenyl, wherein the phenyl is optionally substituted with $R^{1a}$.

**4.** The compound of formula (I) or the pharmaceutically acceptable salt thereof according to any one of claims 1 to 3, wherein

$R^7$ and $R^8$ are each independently selected from H, halogen, CN, OH, $NH_2$, or $C_1$-$C_3$ alkyl, or $R^7$ and $R^8$, together with the atom linked thereto, form $C_3$-$C_6$ cycloalkyl, wherein the OH, $NH_2$, $C_1$-$C_3$ alkyl, or $C_3$-$C_6$ cycloalkyl is optionally substituted with $R^b$; or

$R^7$ and $R^8$ are each independently selected from H, halogen, or $C_1$-$C_3$ alkyl, or $R^7$ and $R^8$, together with the atom linked thereto, form $C_3$-$C_6$ cycloalkyl.

**5.** The compound of formula (I) or the pharmaceutically acceptable salt thereof according to any one of claims 1 to 4, wherein $R^{10}$ is selected from OH, SH, O($C_1$-$C_3$ alkyl), O-C(=O)-($C_1$-$C_3$ alkyl), or

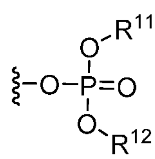
,

wherein the O($C_1$-$C_3$ alkyl) or O-C(=O)-($C_1$-$C_3$ alkyl) is optionally substituted with halogen.

**6.** The compound of formula (I) or the pharmaceutically acceptable salt thereof according to any one of claims 1 to 5, wherein $R^{11}$ and $R^{12}$ are each independently selected from H, methyl, or ethyl.

**7.** The compound of formula (I) or the pharmaceutically acceptable salt thereof according to any one of claims 1 to 6, wherein ring B is selected from $C_6$-$C_{10}$ aryl, 5-10 membered heteroaryl, or 4-14 membered heterocyclyl, wherein the $C_6$-$C_{10}$ aryl, 5-10 membered heteroaryl, or 4-14 membered heterocyclyl is optionally substituted with $R^{2a}$.

**8.** The compound of formula (I) or the pharmaceutically acceptable salt thereof according to any one of claims 1 to 7, wherein

$R^{2a}$ is selected from halogen, CN, =O, OH, $NH_2$, $C_1$-$C_6$ alkyl, $C_3$-$C_6$ cycloalkyl, or 4-7 membered heterocyclyl; or
$R^{2a}$ is selected from $NH_2$ or =O; or
$R^{2a}$ is selected from $NH_2$.

**9.** The compound of formula (I) or the pharmaceutically acceptable salt thereof according to claim 1, wherein the compound of formula (I) or the pharmaceutically acceptable salt thereof is selected from a compound of formula (Ia) or a pharmaceutically acceptable salt thereof:

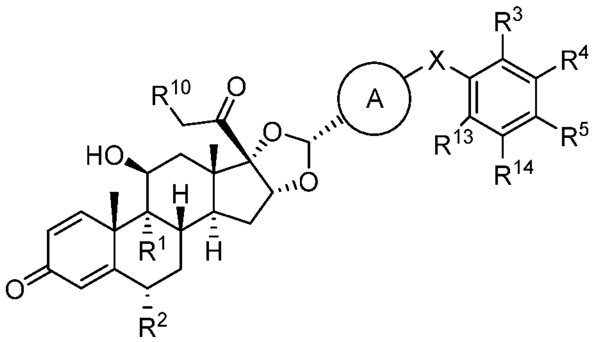

(Ia)

wherein $R^1$, $R^2$, ring A, X, and $R^{10}$ are as defined in claim 1;
$R^3$ is selected from H, OH, or $NHR^9$, $R^4$ and $R^5$, together with the atom linked thereto, form $C_5$-$C_6$ cycloalkenyl, 5-6 membered heterocyclyl, or 5-6 membered heteroaryl, wherein the $C_5$-$C_6$ cycloalkenyl, 5-6 membered heterocyclyl, or 5-6 membered heteroaryl is optionally substituted with $R^{4a}$; or $R^5$ is selected from H, OH, or $NHR^9$, and $R^3$ and $R^4$, together with the atom linked thereto, form $C_5$-$C_6$ cycloalkenyl, 5-6 membered heterocyclyl, or 5-6 membered heteroaryl, wherein the $C_5$-$C_6$ cycloalkenyl, 5-6 membered heterocyclyl, or 5-6 membered heteroaryl is optionally substituted with $R^{4a}$;
$R^9$ is selected from H, $C_1$-$C_6$ alkyl, $C_3$-$C_6$ cycloalkyl, or 4-7 membered heterocyclyl, wherein the $C_1$-$C_6$ alkyl, $C_3$-$C_6$ cycloalkyl, or 4-7 membered heterocyclyl is optionally substituted with $R^d$;
$R^{13}$ and $R^{14}$ are each independently selected from H, halogen, CN, OH, $NH_2$, O($C_1$-$C_3$ alkyl), or $C_1$-$C_6$ alkyl;
each $R^{4a}$ is independently selected from halogen, CN, =O, OH, $NH_2$, $C_1$-$C_6$ alkyl, $C_3$-$C_6$ cycloalkyl, or 4-7 membered heterocyclyl, wherein the OH, $NH_2$, $C_1$-$C_6$ alkyl, $C_3$-$C_6$ cycloalkyl, or 4-7 membered heterocyclyl is optionally substituted with $R^d$;
each $R^d$ is independently selected from halogen, CN, =O, $C_1$-$C_3$ alkyl, OH, O($C_1$-$C_3$ alkyl), $NH_2$, NH($C_1$-$C_3$ alkyl), or N($C_1$-$C_3$ alkyl)$_2$.

**10.** The compound of formula (Ia) or the pharmaceutically acceptable salt thereof according to claim 9, wherein

$R^3$ is selected from H, OH, or $NH_2$, and $R^4$ and $R^5$, together with the atom linked thereto, form 5-6 membered heterocyclyl or 5-6 membered heteroaryl, wherein the 5-6 membered heterocyclyl or 5-6 membered heteroaryl is optionally substituted with $R^{4a}$; or
$R^3$ is selected from H, and $R^4$ and $R^5$, together with the atom linked thereto, form 5-6 membered heterocyclyl or 5-6 membered heteroaryl, wherein the 5-6 membered heterocyclyl or 5-6 membered heteroaryl is optionally substituted with $R^{4a}$; or
$R^3$ is selected from H, OH, or $NH_2$, and $R^4$ and $R^5$, together with the atom linked thereto, form

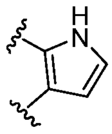, 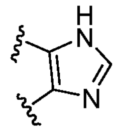, 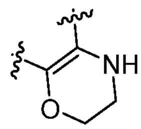, 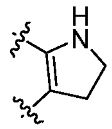, or 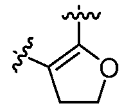,

wherein the

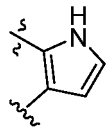, 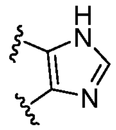,

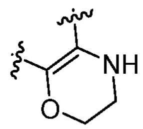, 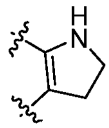, or 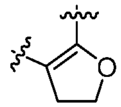

is optionally substituted with $R^{4a}$; or
$R^3$ is selected from H, and $R^4$ and $R^5$, together with the atom linked thereto, form

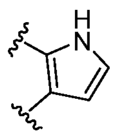, 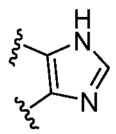,

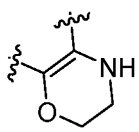, 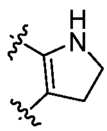, or 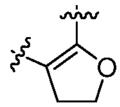,

wherein the

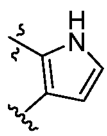, 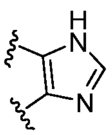, 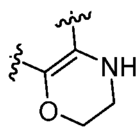, 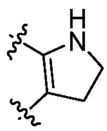,

or

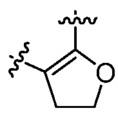

is optionally substituted with $R^{4a}$.

**11.** The compound of formula (Ia) or the pharmaceutically acceptable salt thereof according to claim 9 or 10, wherein

$R^5$ is selected from H, OH, or $NH_2$, and $R^3$ and $R^4$, together with the atom linked thereto, form $C_5$-$C_6$ cycloalkenyl, 5-6 membered heterocyclyl, or 5-6 membered heteroaryl, wherein the $C_5$-$C_6$ cycloalkenyl, 5-6 membered heterocyclyl, or 5-6 membered heteroaryl is optionally substituted with $R^{4a}$; or
$R^5$ is selected from H or $NH_2$, and $R^3$ and $R^4$, together with the atom linked thereto, form $C_5$-$C_6$ cycloalkenyl,

5-6 membered heterocyclyl, or 5-6 membered heteroaryl, wherein the $C_5$-$C_6$ cycloalkenyl, 5-6 membered heterocyclyl, or 5-6 membered heteroaryl is optionally substituted with $R^{4a}$; or
$R^5$ is selected from H, OH, or $NH_2$, and $R^3$ and $R^4$, together with the atom linked thereto, form

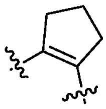, 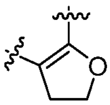, 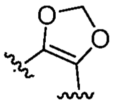, 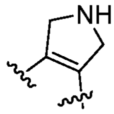, 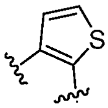, 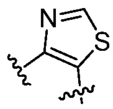, 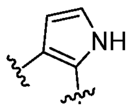, 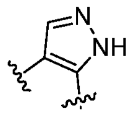, 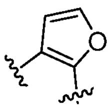,

or

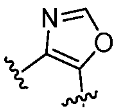,

wherein the

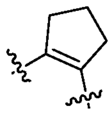, 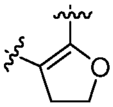, 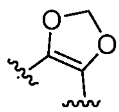, 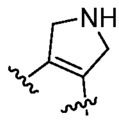, 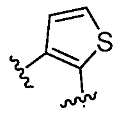, 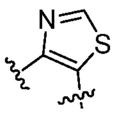,

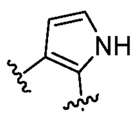, 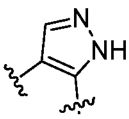, 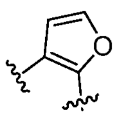, or 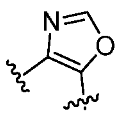

is optionally substituted with $R^{4a}$; or $R^5$ is selected from H or $NH_2$, and $R^3$ and $R^4$, together with the atom linked thereto, form

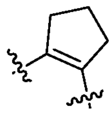,

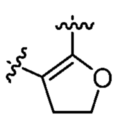, 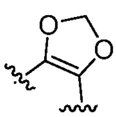, 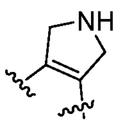, 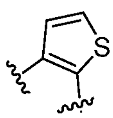, 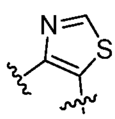, 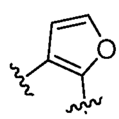, or 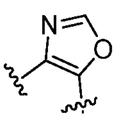,

wherein the

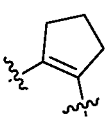, 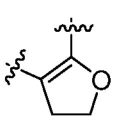, 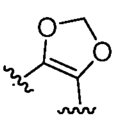, 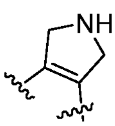, 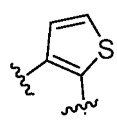, 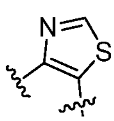, 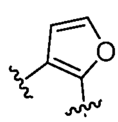, or 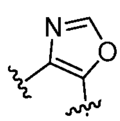

is optionally substituted with $R^{4a}$.

**12.** The compound of formula (Ia) or the pharmaceutically acceptable salt thereof according to any one of claims 9 to 11, wherein X is selected from O, S, $C_1$-$C_3$ alkylene-O, $C_1$-$C_3$ alkylene-S, or $C(R^7)(R^8)$, wherein $R^7$ and $R^8$ are each independently selected from H, halogen, CN, OH, $NH_2$, or $C_1$-$C_3$ alkyl, or $R^7$ and $R^8$, together with the atom linked

thereto, form $C_3$-$C_6$ cycloalkyl, wherein the OH, $NH_2$, $C_1$-$C_3$ alkyl, or $C_3$-$C_6$ cycloalkyl is optionally substituted with $R^b$; or

$R^7$ and $R^8$ are each independently selected from H, halogen, or $C_1$-$C_3$ alkyl, or $R^7$ and $R^8$, together with the atom linked thereto, form $C_3$-$C_6$ cycloalkyl.

**13.** The compound of formula (Ia) or the pharmaceutically acceptable salt thereof according to any one of claims 9 to 12, wherein

$R^{4a}$ is selected from halogen, CN, =O, OH, $NH_2$, $C_1$-$C_6$ alkyl, or $C_3$-$C_6$ cycloalkyl; or
$R^{4a}$ is selected from =O or $NH_2$.

**14.** The compound of formula (Ia) or the pharmaceutically acceptable salt thereof according to any one of claims 9 to 13, wherein $R^{13}$ and $R^{14}$ are each independently selected from H or $NH_2$.

**15.** The compound of formula (Ia) or the pharmaceutically acceptable salt thereof according to any one of claims 9 to 14, wherein structural unit

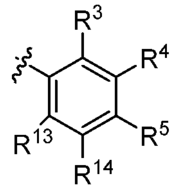

is selected from

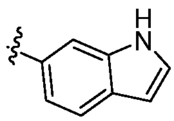 ,

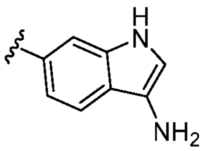 , 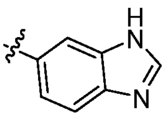 , 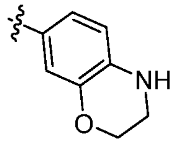 , 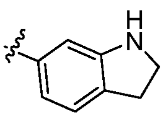 , 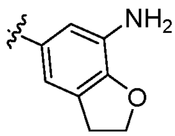 , 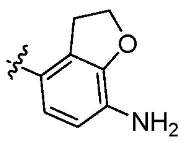 ,

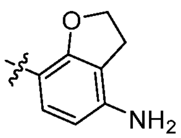 , 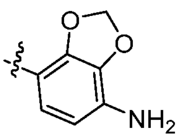 , 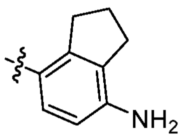 , 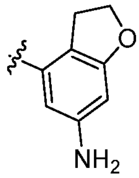 , 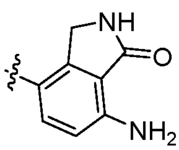 , 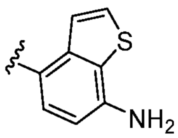 ,

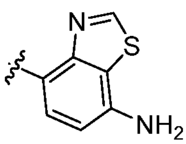 , 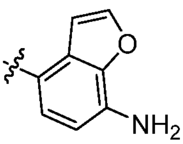 , 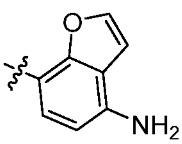 , 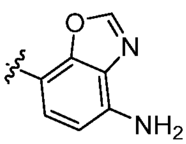 , 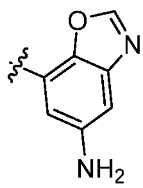 , or 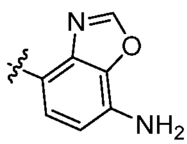 .

**16.** A compound or a pharmaceutically acceptable salt thereof, wherein the compound is selected from one of the following structures:

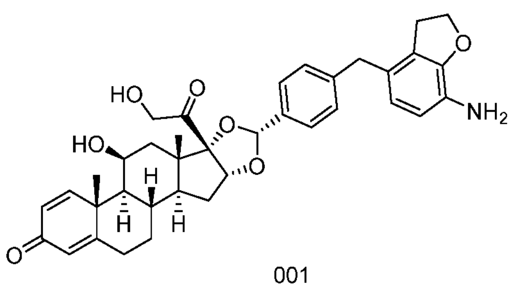

001

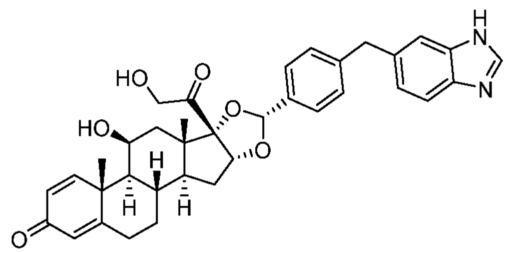

002

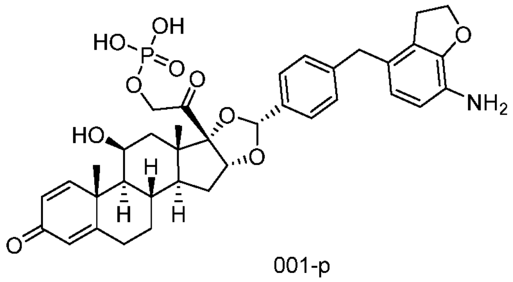

001-p

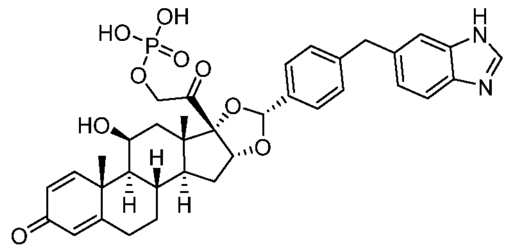

002-p

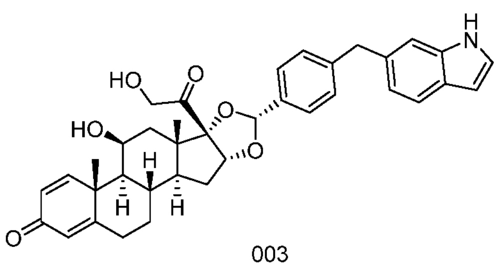

003

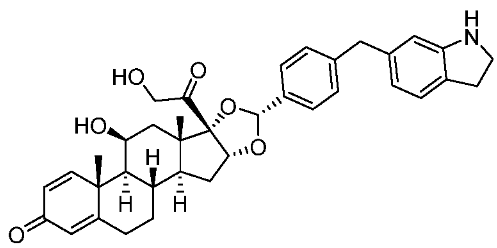

004

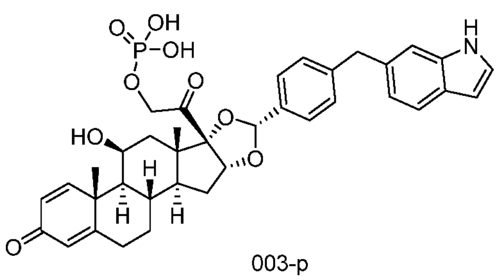

003-p

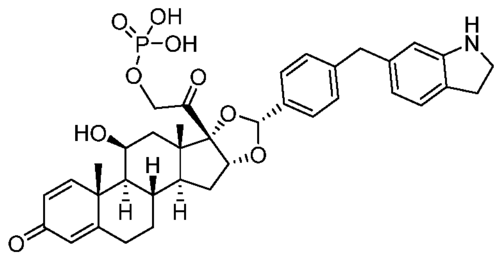

004-p

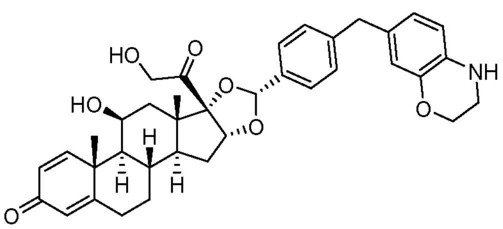

005

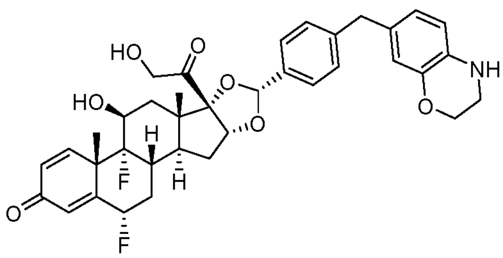

006

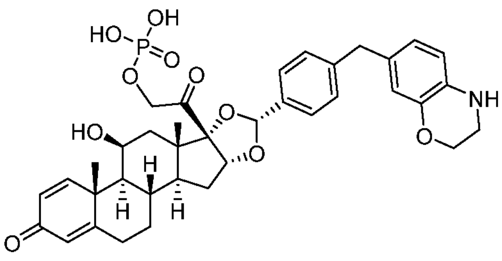

005-p

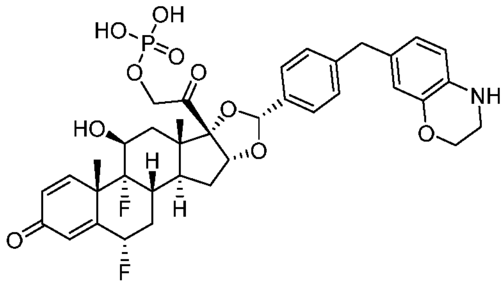

006-p

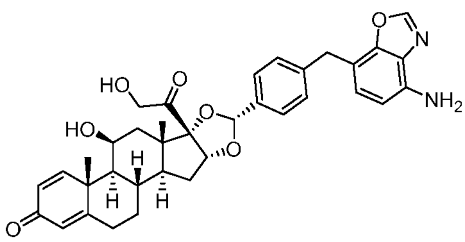

007

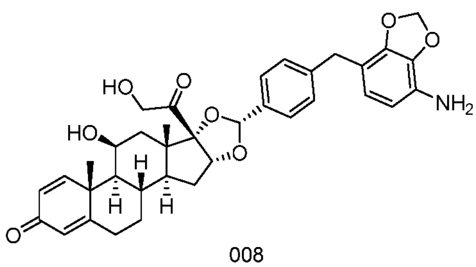

008

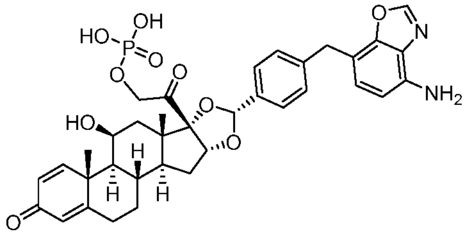

007-p

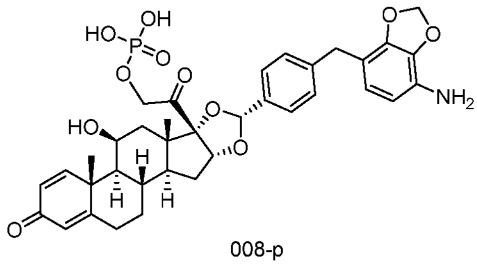

008-p

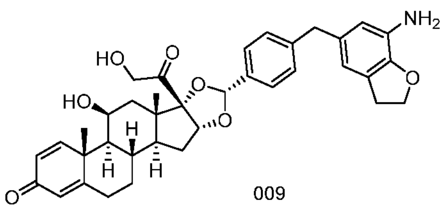

009

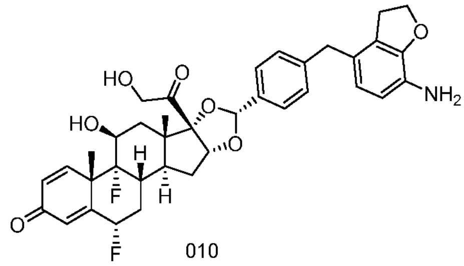

010

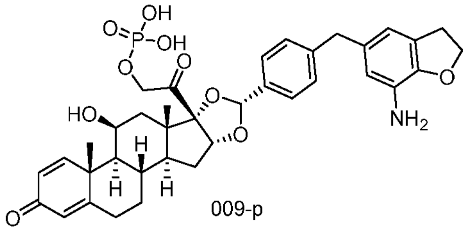

009-p

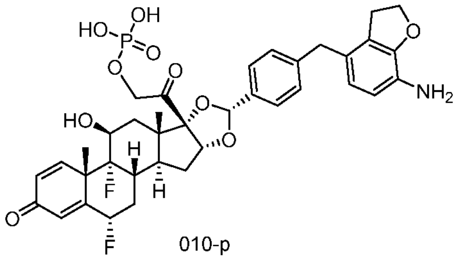

010-p

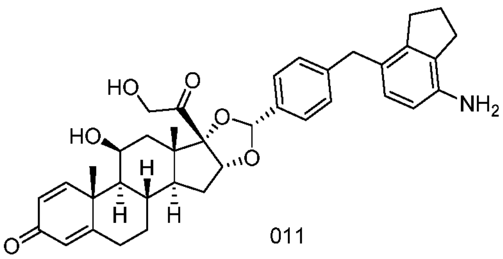

011

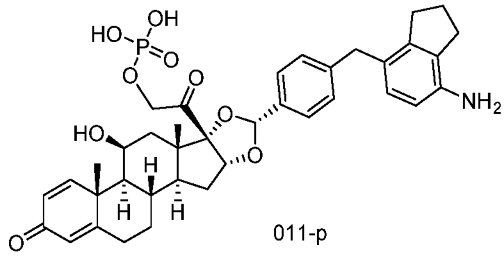

011-p

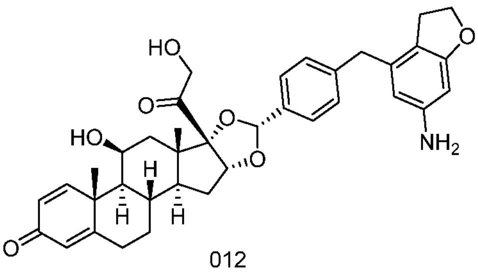

012

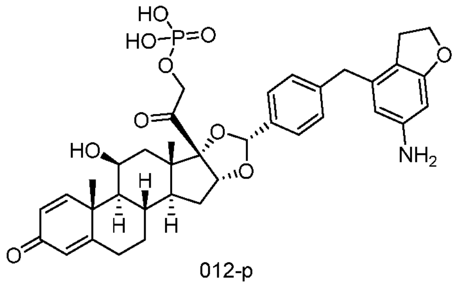

012-p

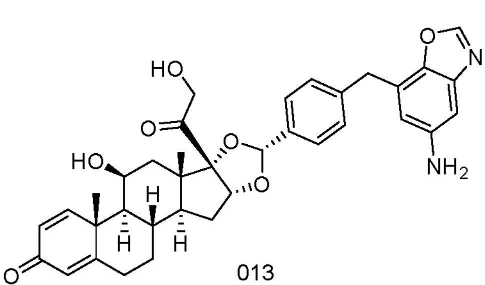

013

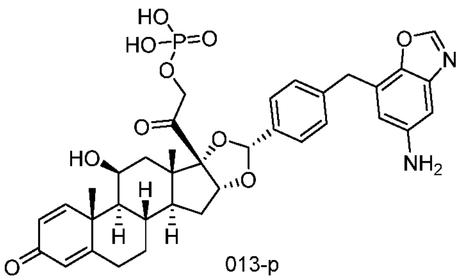

013-p

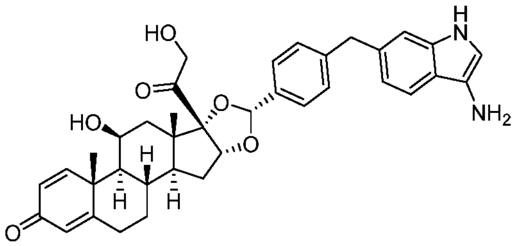

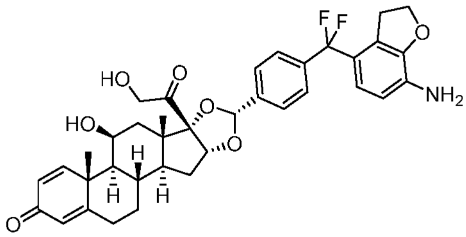

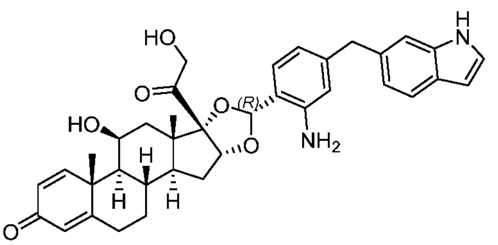

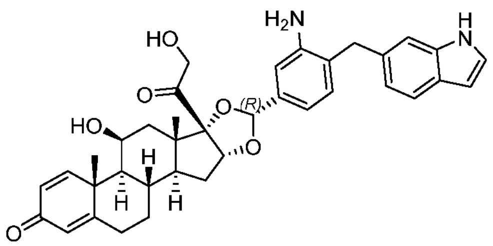

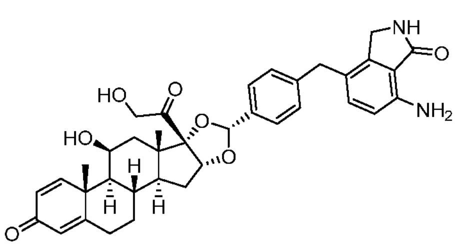

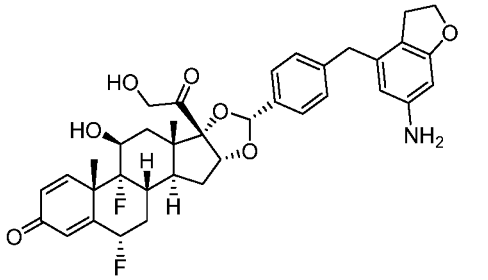

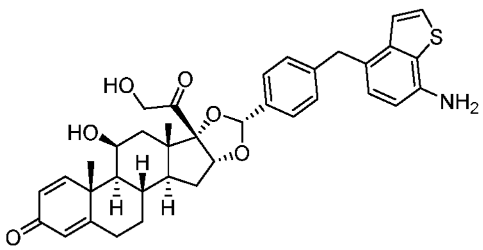

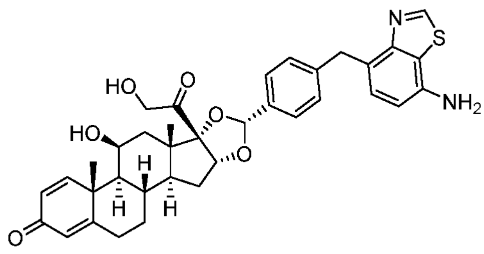

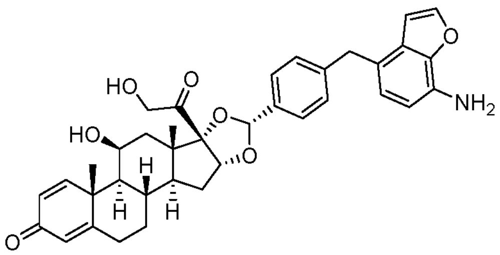

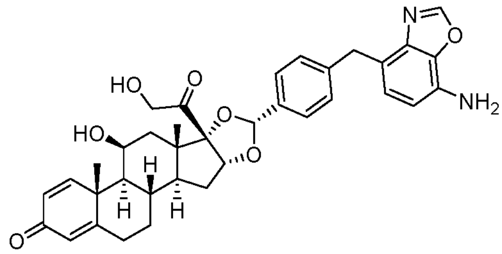

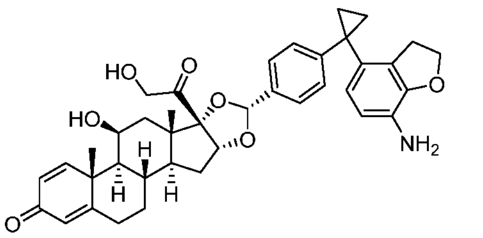

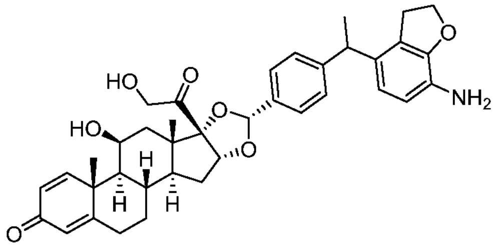

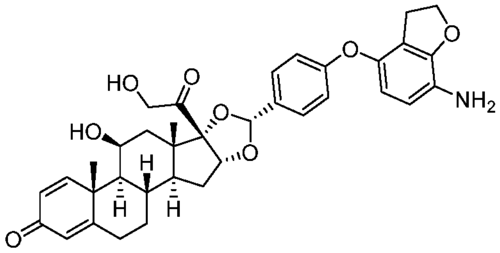

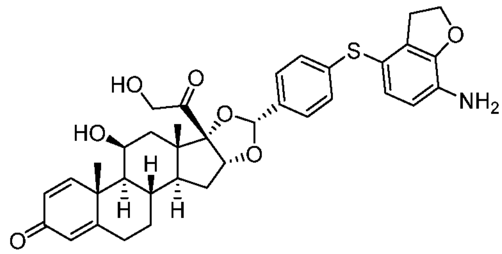

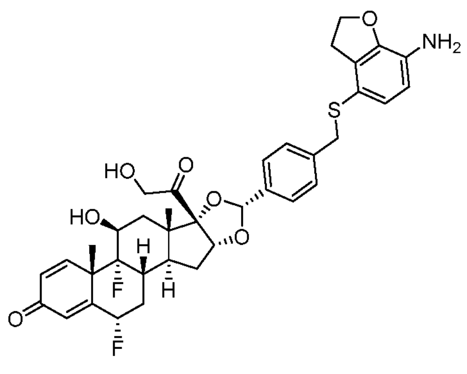

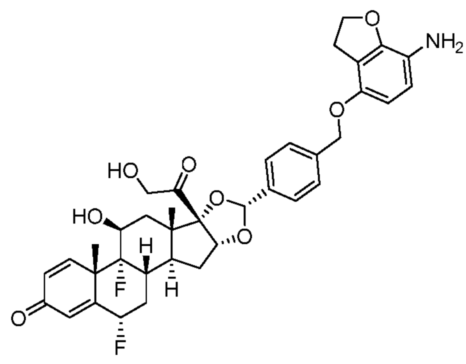

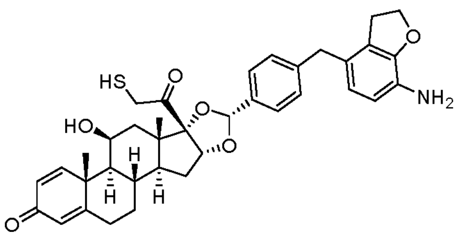

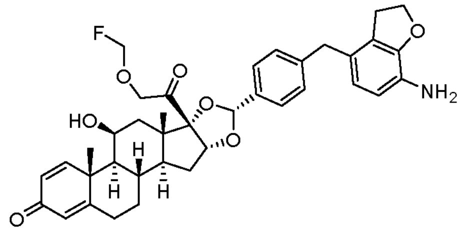

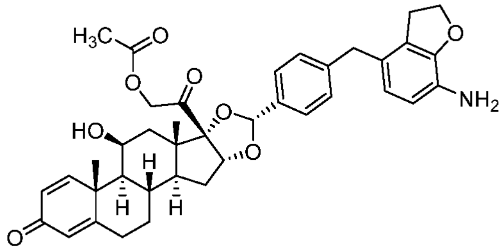

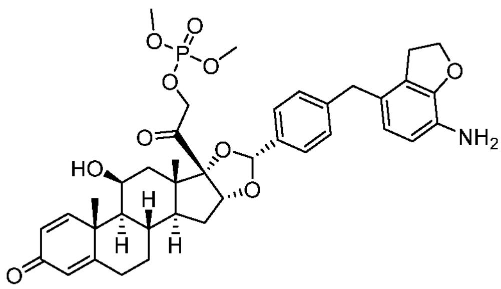

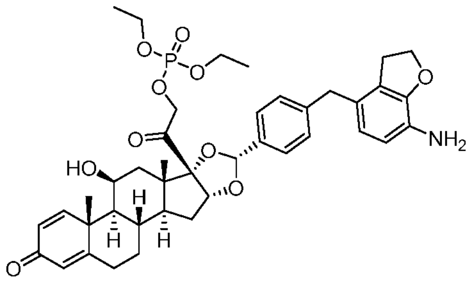

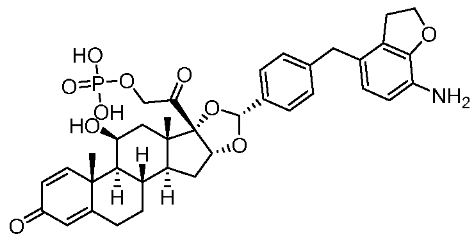

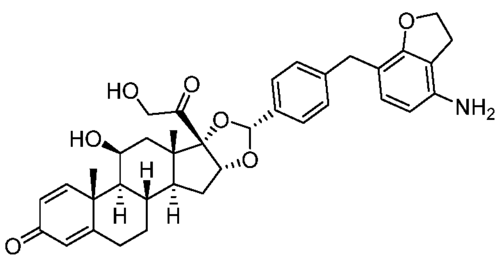

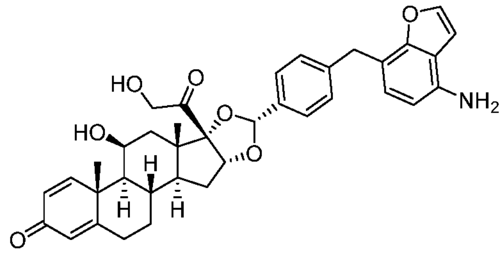

or

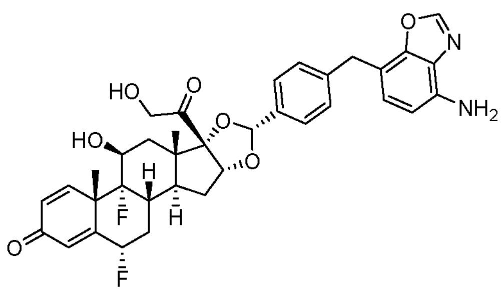

.

**17.** A pharmaceutical composition, comprising the compound or the pharmaceutically acceptable salt thereof according to any one of claims 1 to 16 and a pharmaceutically acceptable excipient.

**18.** Use of the compound or the pharmaceutically acceptable salt thereof according to any one of claims 1 to 16, or the pharmaceutical composition according to claim 17 in preparing a medicament for use in preventing or treating a

disease mediated by a glucocorticoid receptor.

COSY diagram of intermediate 1-5

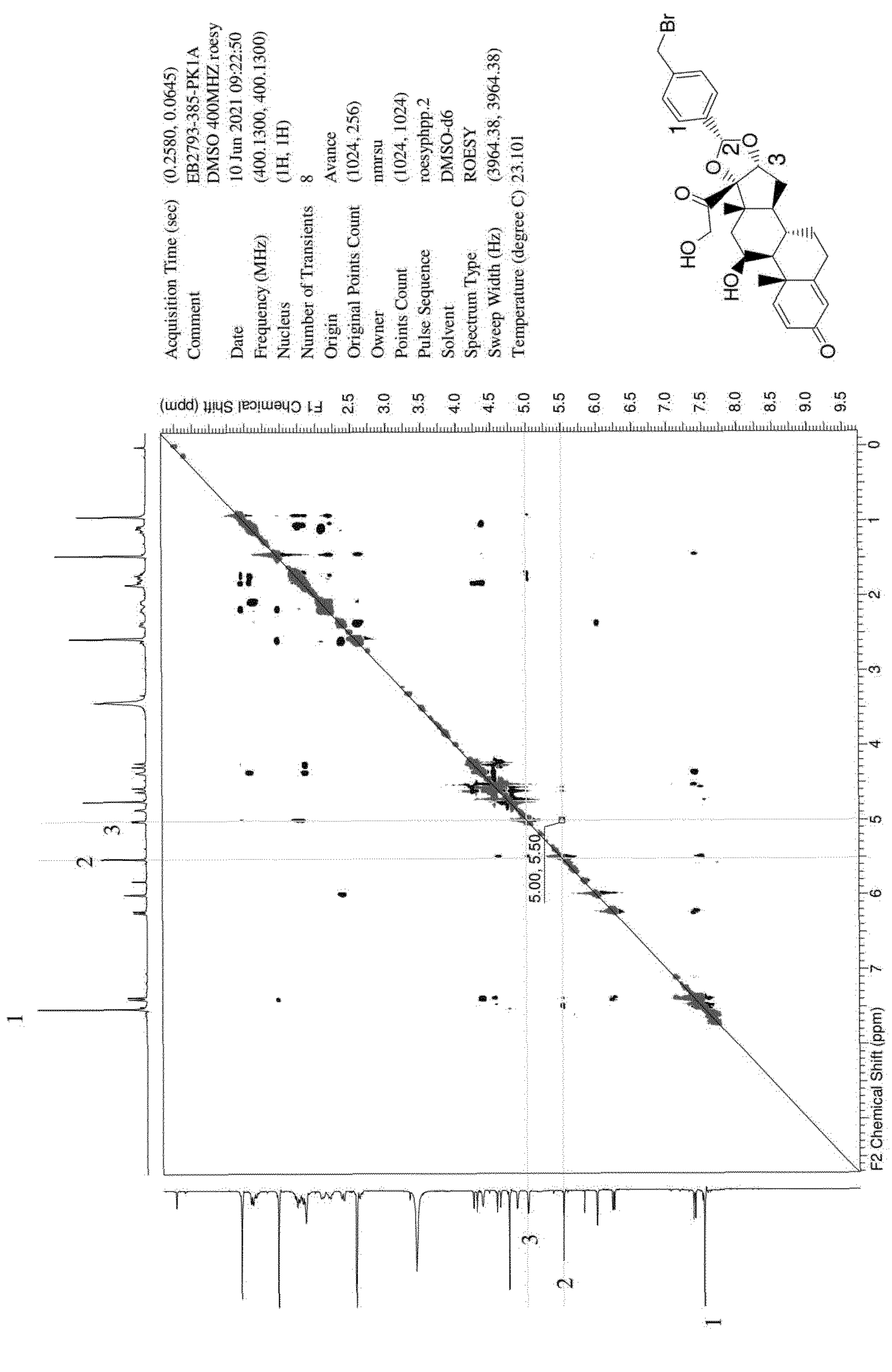


FIG. 1

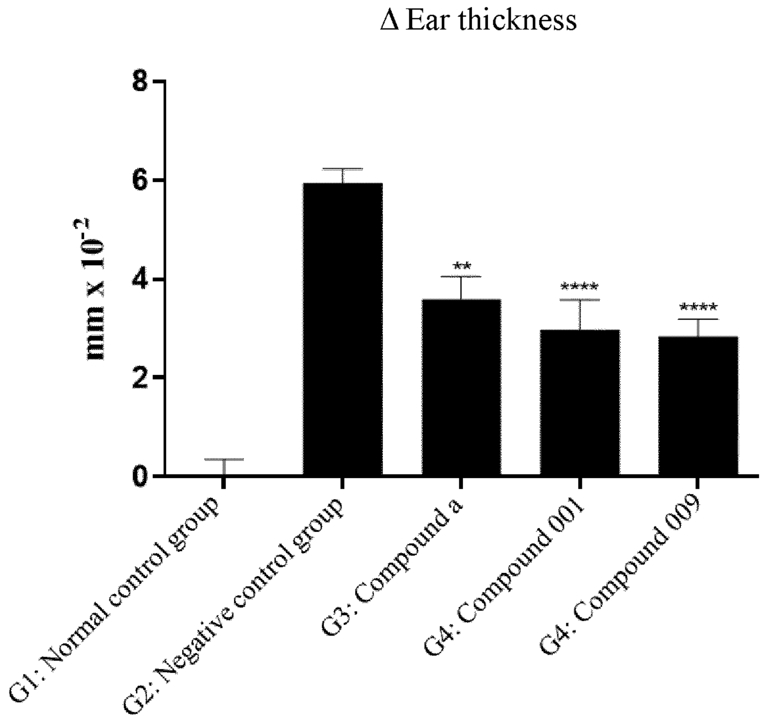
Δ Ear thickness
mm x 10-2
8
6
4
2
0
**
****
****
G1: Normal control group
G2: Negative control group
G3: Compound a
G4: Compound 001
G4: Compound 009

FIG. 2

**INTERNATIONAL SEARCH REPORT**

International application No.
**PCT/CN2022/100881**

**A. CLASSIFICATION OF SUBJECT MATTER**

C07J 71/00(2006.01)i； A61K 31/58(2006.01)i； A61P 5/46(2006.01)i

According to International Patent Classification (IPC) or to both national classification and IPC

**B. FIELDS SEARCHED**

Minimum documentation searched (classification system followed by classification symbols)

C07J; A61K; A61P

Documentation searched other than minimum documentation to the extent that such documents are included in the fields searched

Electronic data base consulted during the international search (name of data base and, where practicable, search terms used)

CNABS, CNTXT, SIPOABS, DWPI, WOTXT, EPTXT, USTXT, CNKI, 万方, WANFANG, 读秀, DUXIU, PUBMED, ISI_Web of Science, Science Direct, STNext: 江苏先声, 刘力峰, 李桢, 结构检索, 糖皮质激素, structure search, glucocorticoid

**C. DOCUMENTS CONSIDERED TO BE RELEVANT**

| Category* | Citation of document, with indication, where appropriate, of the relevant passages | Relevant to claim No. |
|---|---|---|
| PX | WO 2021216913 A1 (IMMUNEXT, INC.) 28 October 2021 (2021-10-28) embodiments 1-3 | 1-8, 17-18 |
| X | CN 101878221 A (PFIZER INC.) 03 November 2010 (2010-11-03) claims 1-16, and embodiments 1-3 and 5 | 1-8, 17-18 |
| X | CN 111417410 A (ABBVIE INC.) 14 July 2020 (2020-07-14) description, paragraphs 0148-0157, and claims 1-11 | 1-8, 17-18 |
| X | CN 111465399 A (ABBVIE INC.) 28 July 2020 (2020-07-28) description, paragraphs 0228, 0357 and 0240, and claims, paragraphs 1-37 | 1-8, 17-18 |
| X | CN 109476699 A (ABBVIE INC.) 15 March 2019 (2019-03-15) description, paragraphs 0853, 0862 and 0878-0880 | 1-8, 17-18 |
| X | MILLAN, D. S. et al. "Design and synthesis of long acting inhaled corticosteroids for the treatment of asthma," *Bioorganic & Medicinal Chemistry Letters,* Vol. 21, 11 August 2011 (2011-08-11), pp. 5826-5830 | 1-8, 17-18 |

☑ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **31 August 2022** | **28 September 2022** |
| Name and mailing address of the ISA/CN<br>**China National Intellectual Property Administration (ISA/CN)**<br>**No. 6, Xitucheng Road, Jimenqiao, Haidian District, Beijing 100088, China** | Authorized officer |
| Facsimile No. **(86-10)62019451** | Telephone No. |

Form PCT/ISA/210 (second sheet) (January 2015)

**INTERNATIONAL SEARCH REPORT**

International application No.
**PCT/CN2022/100881**

**C. DOCUMENTS CONSIDERED TO BE RELEVANT**

| Category* | Citation of document, with indication, where appropriate, of the relevant passages | Relevant to claim No. |
|---|---|---|
| X | KNIGHT, C. J. et al. "Kilogram-Scale Synthesis of an Inhaled Corticosteroid," *Org. Process Res. Dev.,* Vol. 16, 14 March 2012 (2012-03-14), pp. 697-703 | 1-8, 17-18 |
| A | CN 101227912 A (GILEAD SCIENCES INC. (US)) 23 July 2008 (2008-07-23) entire document | 1-18 |
| A | CN 101878221 A (PFIZER INC.) 03 November 2010 (2010-11-03) claims 1-16, and embodiments 1-3 and 5 | 9-16 |
| A | CN 111417410 A (ABBVIE INC.) 14 July 2020 (2020-07-14) description, paragraphs 0148-0157, and claims 1-11 | 9-16 |
| A | CN 111465399 A (ABBVIE INC.) 28 July 2020 (2020-07-28) description, paragraphs 0228, 0357 and 0240, and claims 1-37 | 9-16 |
| A | CN 109476699 A (ABBVIE INC.) 15 March 2019 (2019-03-15) description, paragraphs 0853, 0862 and 0878-0880 | 9-16 |

Form PCT/ISA/210 (second sheet) (January 2015)

**INTERNATIONAL SEARCH REPORT**
**Information on patent family members**

International application No.
**PCT/CN2022/100881**

| Patent document cited in search report | | | Publication date (day/month/year) | Patent family member(s) | | | Publication date (day/month/year) |
|---|---|---|---|---|---|---|---|
| WO | 2021216913 | A1 | 28 October 2021 | None | | | |
| CN | 101878221 | A | 03 November 2010 | ZA | 201003439 | B | 23 February 2011 |
| | | | | NZ | 585434 | A | 29 June 2012 |
| | | | | DO | P2010000161 | A | 15 July 2010 |
| | | | | AP | 2010005267 | A0 | 30 June 2010 |
| | | | | SV | 2010003580 | A | 23 March 2011 |
| | | | | HK | 1144821 | A1 | 11 March 2011 |
| | | | | GT | 201000153 | A | 27 April 2012 |
| | | | | US | 2014336160 | A1 | 13 November 2014 |
| | | | | UY | 31496 | A1 | 17 July 2009 |
| | | | | TW | 200930378 | A | 16 July 2009 |
| | | | | TN | 2010000230 | A1 | 11 November 2011 |
| | | | | WO | 2009069032 | A2 | 04 June 2009 |
| | | | | AR | 069490 | A1 | 27 January 2010 |
| | | | | AU | 2008331187 | A1 | 04 June 2009 |
| | | | | EA | 201070535 | A1 | 30 December 2010 |
| | | | | CA | 2706180 | A1 | 04 June 2009 |
| | | | | ES | 2399945 | T3 | 04 April 2013 |
| | | | | EP | 2225256 | A2 | 08 September 2010 |
| | | | | PE | 20091072 | A1 | 23 July 2009 |
| | | | | NI | 201000090 | A | 04 October 2010 |
| | | | | KR | 20100074306 | A | 01 July 2010 |
| | | | | PA | 8805801 | A1 | 23 July 2009 |
| | | | | BR | PI0820447 | A2 | 26 May 2015 |
| | | | | JP | 2011505350 | A | 24 February 2011 |
| | | | | DK | 2225256 | T3 | 11 March 2013 |
| | | | | CO | 6280401 | A2 | 20 May 2011 |
| | | | | EC | SP10010209 | A | 29 June 2010 |
| | | | | MX | 2010005861 | A | 11 June 2010 |
| | | | | CL | 2008003549 | A1 | 20 November 2009 |
| | | | | MA | 31867 | B1 | 01 November 2010 |
| | | | | IL | 205356 | D0 | 30 December 2010 |
| | | | | US | 2009227548 | A1 | 10 September 2009 |
| | | | | CR | 11393 | A | 19 May 2010 |
| CN | 111417410 | A | 14 July 2020 | LT | 3658192 | T | 12 July 2021 |
| | | | | CA | 3082356 | A1 | 06 June 2019 |
| | | | | CL | 2020001452 | A1 | 11 September 2020 |
| | | | | PT | 3658192 | T | 25 June 2021 |
| | | | | ZA | 202003325 | B | 28 July 2021 |
| | | | | UY | 37991 | A | 28 June 2019 |
| | | | | SI | 3658192 | T1 | 31 August 2021 |
| | | | | CL | 2021001075 | A1 | 15 October 2021 |
| | | | | RS | 61994 | B1 | 30 July 2021 |
| | | | | JP | 2021054845 | A | 08 April 2021 |
| | | | | MA | 49796 | A | 03 June 2020 |
| | | | | US | 2021015938 | A1 | 21 January 2021 |
| | | | | JP | 6813712 | B2 | 13 January 2021 |
| | | | | KR | 20200095477 | A | 10 August 2020 |
| | | | | AU | 2018374634 | A1 | 28 May 2020 |
| | | | | HR | P20210917 | T1 | 03 September 2021 |

Form PCT/ISA/210 (patent family annex) (January 2015)

**INTERNATIONAL SEARCH REPORT**
**Information on patent family members**

International application No.
**PCT/CN2022/100881**

| Patent document cited in search report | Publication date (day/month/year) | Patent family member(s) | Publication date (day/month/year) |
|---|---|---|---|
| | | PE 20201286 A1 | 24 November 2020 |
| | | DK 3658192 T3 | 21 June 2021 |
| | | PL 3658192 T3 | 18 October 2021 |
| | | IL 274651 A | 30 June 2020 |
| | | EC SP20029001 A | 30 June 2020 |
| | | DO P2020000116 A | 31 August 2020 |
| | | CO 2020006446 A2 | 09 June 2020 |
| | | EP 3658192 A1 | 03 June 2020 |
| | | ES 2877659 T3 | 17 November 2021 |
| | | WO 2019106609 A1 | 06 June 2019 |
| | | TW 201924723 A | 01 July 2019 |
| | | BR 112020010694 A2 | 10 November 2020 |
| | | CR 20200239 A | 21 September 2020 |
| | | US 2019167804 A1 | 06 June 2019 |
| | | EP 3884962 A1 | 29 September 2021 |
| | | RU 2020117698 A3 | 04 January 2022 |
| | | HU E054428 T2 | 28 September 2021 |
| | | SG 11202004867 W A | 29 June 2020 |
| CN 111465399 A | 28 July 2020 | PH 12020550551 A1 | 22 March 2021 |
| | | CL 2020001442 A1 | 11 September 2020 |
| | | BR 112020010691 A2 | 10 November 2020 |
| | | EP 3716982 A1 | 07 October 2020 |
| | | SG 11202004865 S A | 29 June 2020 |
| | | CA 3081559 A1 | 06 June 2019 |
| | | JP 2021504430 A | 15 February 2021 |
| | | KR 20200095493 A | 10 August 2020 |
| | | EC SP20034868 A | 31 August 2020 |
| | | CR 20200285 A | 04 September 2020 |
| | | PE 20201464 A1 | 17 December 2020 |
| | | WO 2019106608 A1 | 06 June 2019 |
| | | IL 274650 A | 30 June 2020 |
| | | AU 2018374633 A1 | 21 May 2020 |
| | | DO P2020000119 A | 31 August 2020 |
| | | RU 2020117156 A | 04 January 2022 |
| CN 109476699 A | 15 March 2019 | JP 2021088582 A | 10 June 2021 |
| | | RU 2018145728 A | 10 July 2020 |
| | | DK 3464318 T3 | 28 June 2021 |
| | | KR 20190014542 A | 12 February 2019 |
| | | PE 20190622 A1 | 26 April 2019 |
| | | CR 20180594 A | 29 July 2019 |
| | | US 2018126000 A1 | 10 May 2018 |
| | | PH 12018502539 A1 | 30 September 2019 |
| | | MX 2018014927 A | 09 April 2019 |
| | | PL 3464318 T3 | 08 November 2021 |
| | | AU 2021269433 A1 | 16 December 2021 |
| | | TW 201801749 A | 16 January 2018 |
| | | SG 10202001787 Q A | 29 April 2020 |
| | | EP 3901162 A1 | 27 October 2021 |
| | | WO 2017210471 A1 | 07 December 2017 |
| | | EC SP18094857 A | 31 January 2019 |

Form PCT/ISA/210 (patent family annex) (January 2015)

**INTERNATIONAL SEARCH REPORT**
**Information on patent family members**

International application No.
**PCT/CN2022/100881**

| Patent document cited in search report | | | Publication date (day/month/year) | Patent family member(s) | | | Publication date (day/month/year) |
|---|---|---|---|---|---|---|---|
| | | | | DO | P2018000261 | A | 31 December 2018 |
| | | | | BR | 112018074922 | A2 | 12 March 2019 |
| | | | | IL | 263214 | A | 31 December 2018 |
| | | | | CL | 2018003406 | A1 | 15 February 2019 |
| | | | | ZA | 201808623 | B | 28 August 2019 |
| | | | | UY | 37269 | A | 02 January 2018 |
| | | | | SG | 11201810678 W | A | 28 December 2018 |
| | | | | LT | 3464318 | T | 25 June 2021 |
| | | | | JP | 2019524645 | A | 05 September 2019 |
| | | | | PT | 3464318 | T | 02 July 2021 |
| | | | | HU | E054804 | T2 | 28 September 2021 |
| | | | | CO | 2018012996 | A2 | 18 January 2019 |
| | | | | SI | 3464318 | T1 | 30 July 2021 |
| | | | | MA | 51586 | A | 10 April 2019 |
| | | | | ES | 2877548 | T3 | 17 November 2021 |
| | | | | HR | P20210924 | T1 | 03 September 2021 |
| | | | | AU | 2017274442 | A1 | 13 December 2018 |
| | | | | EP | 3464318 | A1 | 10 April 2019 |
| | | | | US | 2020338208 | A1 | 29 October 2020 |
| | | | | RS | 62040 | B1 | 30 July 2021 |
| | | | | CA | 3025377 | A1 | 07 December 2017 |
| | | | | US | 2019262465 | A1 | 29 August 2019 |
| CN | 101227912 | A | 23 July 2008 | EP | 1893220 | A1 | 05 March 2008 |
| | | | | WO | 2006138212 | A1 | 28 December 2006 |
| | | | | US | 2010209508 | A1 | 19 August 2010 |
| | | | | IL | 187816 | D0 | 07 August 2008 |
| | | | | CA | 2612364 | A1 | 28 December 2006 |
| | | | | RU | 2008100237 | A | 20 July 2009 |
| | | | | AR | 054475 | A1 | 27 June 2007 |
| | | | | TW | 200718707 | A | 16 May 2007 |
| | | | | BR | PI0611567 | A2 | 16 November 2016 |
| | | | | IN | 4823KOLNP2007 | A | 04 July 2008 |
| | | | | JP | 2008546694 | A | 25 December 2008 |
| | | | | AU | 2006259604 | A1 | 28 December 2006 |
| | | | | NO | 20080182 | L | 21 February 2008 |
| | | | | KR | 20060130515 | A | 19 December 2006 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202110706921X
- CN 202210101639
- CN 202210340109
- WO 2016169504 A1
- CN 110903258
- WO 2017210471 A


### Non-patent literature cited in the description


- **MAEHR.** *JChem.Ed.,* 1985, vol. 62, 114-120
- *CHEMICAL ABSTRACTS,* 1677706-25-0
- *CHEMICAL ABSTRACTS,* 1267216-18-1